# EUROPEAN PATENT APPLICATION

(11) **EP 4 424 315 A2**
(43) Date of publication of application: **04.09.2024**
(21) Application number: 24159857.2
(22) Date of filing: 01.03.2019
(51) Int. Cl.: A61K 31/64

(54) **NOVEL COMPOUNDS**

(30) Priority: 02.03.2018 GB 201803394
(62) Divisional of application: 19708310.8
(71) Applicant: Inflazome Limited, Dublin 2 (IE)
(72) Inventor: COOPER, Matthew, Cambridge, CB22 5LD (GB); MILLER, David, Cambridge, CB22 5LD (GB); MACLEOD, Angus, Cambridge, CB22 5LD (GB)
(74) Representative: Bernard, Guillaume

(57) **Abstract**

The present invention relates to phenylsulfonylureas and phenylsulfonylthioureas, wherein the phenyl ring is substituted with a monovalent group comprising either (i) an aryl or a heteroaryl group, or (ii) a nitrogen-containing heterocyclic group, and wherein the group attached to the terminal nitrogen atom of the urea group is a 6-membered cyclic group substituted at the 2- and 4-positions. The present invention further relates to salts, solvates and prodrugs of such compounds, to pharmaceutical compositions comprising such compounds, and to the use of such compounds in the treatment and prevention of medical disorders and diseases, most especially by NLRP3 inhibition.

## Description

### Field of the Invention

The present invention relates to phenylsulfonylureas and phenylsulfonylthioureas, wherein the phenyl ring is substituted with a monovalent group comprising either (i) an aryl or a heteroaryl group, or (ii) a nitrogen-containing heterocyclic group, and wherein the group attached to the terminal nitrogen atom of the urea group is a 6-membered cyclic group substituted at the 2- and 4-positions. The present invention further relates to salts, solvates and prodrugs of such compounds, to pharmaceutical compositions comprising such compounds, and to the use of such compounds in the treatment and prevention of medical disorders and diseases, most especially by NLRP3 inhibition.

### Background

The NOD-like receptor (NLR) family, pyrin domain-containing protein 3 (NLRP3) inflammasome is a component of the inflammatory process, and its aberrant activity is pathogenic in inherited disorders such as cryopyrin-associated periodic syndromes (CAPS) and complex diseases such as multiple sclerosis, type 2 diabetes, Alzheimer's disease and atherosclerosis.

NLRP3 is an intracellular signalling molecule that senses many pathogen-derived, environmental and host-derived factors. Upon activation, NLRP3 binds to apoptosis-associated speck-like protein containing a caspase activation and recruitment domain (ASC). ASC then polymerises to form a large aggregate known as an ASC speck. Polymerised ASC in turn interacts with the cysteine protease caspase-1 to form a complex termed the inflammasome. This results in the activation of caspase-1, which cleaves the precursor forms of the proinflammatory cytokines IL-1β and IL-18 (termed pro-IL-1β and pro-IL-18 respectively) to thereby activate these cytokines. Caspase-1 also mediates a type of inflammatory cell death known as pyroptosis. The ASC speck can also recruit and activate caspase-8, which can process pro-IL-1β and pro-IL-18 and trigger apoptotic cell death.

Caspase-1 cleaves pro-IL-1β and pro-IL-18 to their active forms, which are secreted from the cell. Active caspase-1 also cleaves gasdermin-D to trigger pyroptosis. Through its control of the pyroptotic cell death pathway, caspase-1 also mediates the release of alarmin molecules such as IL-33 and high mobility group box 1 protein (HMGB1).

Caspase-1 also cleaves intracellular IL-1R2 resulting in its degradation and allowing the release of IL-1α. In human cells caspase-1 may also control the processing and secretion of IL-37. A number of other caspase-1 substrates such as components of the cytoskeleton and glycolysis pathway may contribute to caspase-1-dependent inflammation.

NLRP3-dependent ASC specks are released into the extracellular environment where they can activate caspase-1, induce processing of caspase-1 substrates and propagate inflammation.

Active cytokines derived from NLRP3 inflammasome activation are important drivers of inflammation and interact with other cytokine pathways to shape the immune response to infection and injury. For example, IL-1β signalling induces the secretion of the pro-inflammatory cytokines IL-6 and TNF. IL-1β and IL-18 synergise with IL-23 to induce IL-17 production by memory CD4 Th17 cells and by γδ T cells in the absence of T cell receptor engagement. IL-18 and IL-12 also synergise to induce IFN-γ production from memory T cells and NK cells driving a Th1 response.

The inherited CAPS diseases Muckle-Wells syndrome (MWS), familial cold autoinflammatory syndrome and neonatal-onset multisystem inflammatory disease are caused by gain-of-function mutations in NLRP3, thus defining NLRP3 as a critical component of the inflammatory process. NLRP3 has also been implicated in the pathogenesis of a number of complex diseases, notably including metabolic disorders such as type 2 diabetes, atherosclerosis, obesity and gout.

A role for NLRP3 in diseases of the central nervous system is emerging, and lung diseases have also been shown to be influenced by NLRP3. Furthermore, NLRP3 has a role in the development of liver disease, kidney disease and aging. Many of these associations were defined using *Nlrp3*^{*-*/*-*} mice, but there have also been insights into the specific activation of NLRP3 in these diseases. In type 2 diabetes mellitus (T2D), the deposition of islet amyloid polypeptide in the pancreas activates NLRP3 and IL-1β signaling, resulting in cell death and inflammation.

Several small molecules have been shown to inhibit the NLRP3 inflammasome. Glyburide inhibits IL-1β production at micromolar concentrations in response to the activation of NLRP3 but not NLRC4 or NLRP1. Other previously characterised NLRP3 inhibitors include parthenolide, 3,4-methylenedioxy-β-nitrostyrene and dimethyl sulfoxide (DMSO), although these agents have limited potency and are nonspecific.

Current treatments for NLRP3-related diseases include biologic agents that target IL-1. These are the recombinant IL-1 receptor antagonist anakinra, the neutralizing IL-1β antibody canakinumab and the soluble decoy IL-1 receptor rilonacept. These approaches have proven successful in the treatment of CAPS, and these biologic agents have been used in clinical trials for other IL-1β-associated diseases.

Some diarylsulfonylurea-containing compounds have been identified as cytokine release inhibitory drugs (CRIDs) (Perregaux et al.; J. Pharmacol. Exp. Ther. 299, 187-197, 2001). CRIDs are a class of diarylsulfonylurea-containing compounds that inhibit the post-translational processing of IL-1β. Post-translational processing of IL-1β is accompanied by activation of caspase-1 and cell death. CRIDs arrest activated monocytes so that caspase-1 remains inactive and plasma membrane latency is preserved.

Certain sulfonylurea-containing compounds are also disclosed as inhibitors of NLRP3 (see for example, Baldwin et al., J. Med. Chem., 59(5), 1691-1710, 2016; and WO 2016/131098 A1, WO 2017/129897 A1, WO 2017/140778 A1, WO 2017/184604 A1, WO 2017/184623 A1, WO 2017/184624 A1 and WO 2018/015445 A1).

There is a need to provide compounds with improved pharmacological and/or physiological and/or physicochemical properties and/or those that provide a useful alternative to known compounds.

### Summary of the Invention

A first aspect of the invention provides a compound of formula (I): 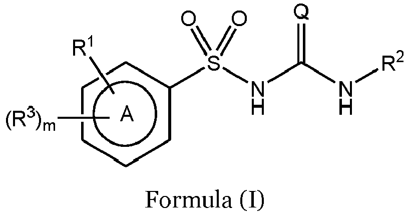 wherein:
Q is selected from O or S;
R¹ is selected from (i) a monovalent group comprising an aryl or a heteroaryl group, or (ii) a monovalent group comprising a heterocyclic group, wherein the heterocyclic group contains at least one nitrogen atom in its ring structure;
R² is a 6-membered cyclic group substituted at the 2- and 4-positions, wherein the 6-membered cyclic group may optionally be further substituted;
m is 0, 1, 2, 3 or 4;
each R³ is independently a halo, -OH, -NO₂, -NH₂, -N₃, -SH, -SO₂H, -SO₂NH, or a saturated or unsaturated hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton; and
wherein optionally any R³, and any two adjacent carbon atoms of ring A, may together form a 4- to 12-membered saturated or unsaturated cyclic group fused to ring A, wherein the cyclic group fused to ring A may optionally be substituted.

In the context of the present specification, a "hydrocarbyl" substituent group or a hydrocarbyl moiety in a substituent group only includes carbon and hydrogen atoms but, unless stated otherwise, does not include any heteroatoms, such as N, O or S, in its carbon skeleton. A hydrocarbyl group/moiety may be saturated or unsaturated (including aromatic), and may be straight-chained or branched, or be or include cyclic groups wherein, unless stated otherwise, the cyclic group does not include any heteroatoms, such as N, O or S, in its carbon skeleton. Examples of hydrocarbyl groups include alkyl, alkenyl, alkynyl, cycloalkyl, cycloalkenyl and aryl groups/moieties and combinations of all of these groups/moieties. Typically a hydrocarbyl group is a C₁-C₂₀ hydrocarbyl group. More typically a hydrocarbyl group is a C₁-C₁₅ hydrocarbyl group. More typically a hydrocarbyl group is a C₁-C₁₀ hydrocarbyl group. A "hydrocarbylene" group is similarly defined as a divalent hydrocarbyl group.

An "alkyl" substituent group or an alkyl moiety in a substituent group may be linear or branched. Examples of alkyl groups/moieties include methyl, ethyl, n-propyl, i-propyl, n-butyl, i-butyl, t-butyl and n-pentyl groups/moieties. Unless stated otherwise, the term "alkyl" does not include "cycloalkyl". Typically an alkyl group is a C₁-C₁₂ alkyl group. More typically an alkyl group is a C₁-C₆ alkyl group. An "alkylene" group is similarly defined as a divalent alkyl group.

An "alkenyl" substituent group or an alkenyl moiety in a substituent group refers to an unsaturated alkyl group or moiety having one or more carbon-carbon double bonds. Examples of alkenyl groups/moieties include ethenyl, propenyl, 1-butenyl, 2-butenyl, 1-pentenyl, 1-hexenyl, 1,3-butadienyl, 1,3-pentadienyl, 1,4-pentadienyl and 1,4-hexadienyl groups/moieties. Unless stated otherwise, the term "alkenyl" does not include "cycloalkenyl". Typically an alkenyl group is a C₂-C₁₂ alkenyl group. More typically an alkenyl group is a C₂-C₆ alkenyl group. An "alkenylene" group is similarly defined as a divalent alkenyl group.

An "alkynyl" substituent group or an alkynyl moiety in a substituent group refers to an unsaturated alkyl group or moiety having one or more carbon-carbon triple bonds. Examples of alkynyl groups/moieties include ethynyl, propargyl, but-1-ynyl and but-2-ynyl groups/moieties. Typically an alkynyl group is a C₂-C₁₂ alkynyl group. More typically an alkynyl group is a C₂-C₆ alkynyl group. An "alkynylene" group is similarly defined as a divalent alkynyl group.

A "cyclic" substituent group or a cyclic moiety in a substituent group refers to any hydrocarbyl ring, wherein the hydrocarbyl ring may be saturated or unsaturated (including aromatic) and may include one or more heteroatoms, e.g. N, O or S, in its carbon skeleton. Examples of cyclic groups include cycloalkyl, cycloalkenyl, heterocyclic, aryl and heteroaryl groups as discussed below. A cyclic group may be monocyclic, bicyclic (e.g. bridged, fused or spiro), or polycyclic. Typically, a cyclic group is a 3- to 12-membered cyclic group, which means it contains from 3 to 12 ring atoms. More typically, a cyclic group is a 3- to 7-membered monocyclic group, which means it contains from 3 to 7 ring atoms.

As used herein, where it is stated that a cyclic group is monocyclic, it is to be understood that the cyclic group is not substituted with a divalent bridging substituent (e.g. -O-, -S-, -NH-, -N(Rβ)- or -R^{α}-) so as to form a bridged, fused or spiro substituent. However, unless stated otherwise, a substituted monocyclic group may be substituted with one or more monovalent cyclic groups. Similarly, where it is stated that a group is bicyclic, it is to be understood that the cyclic group including any bridged, fused or spiro divalent bridging substituents attached to the cyclic group, but excluding any monovalent cyclic substituents, is bicyclic.

A "heterocyclic" substituent group or a heterocyclic moiety in a substituent group refers to a cyclic group or moiety including one or more carbon atoms and one or more heteroatoms, e.g. N, O or S, in the ring structure. Examples of heterocyclic groups include heteroaryl groups as discussed below and non-aromatic heterocyclic groups such as azetinyl, azetidinyl, oxetanyl, thietanyl, pyrrolidinyl, tetrahydrofuranyl, tetrahydrothiophenyl, pyrazolidinyl, imidazolidinyl, dioxolanyl, oxathiolanyl, piperidinyl, tetrahydropyranyl, thianyl, piperazinyl, dioxanyl, morpholinyl and thiomorpholinyl groups.

A "cycloalkyl" substituent group or a cycloalkyl moiety in a substituent group refers to a saturated hydrocarbyl ring containing, for example, from 3 to 7 carbon atoms, examples of which include cyclopropyl, cyclobutyl, cyclopentyl and cyclohexyl. Unless stated otherwise, a cycloalkyl substituent group or moiety may include monocyclic, bicyclic or polycyclic hydrocarbyl rings.

A "cycloalkenyl" substituent group or a cycloalkenyl moiety in a substituent group refers to a non-aromatic unsaturated hydrocarbyl ring having one or more carbon-carbon double bonds and containing, for example, from 3 to 7 carbon atoms, examples of which include cyclopent-1-en-1-yl, cyclohex-1-en-1-yl and cyclohex-1,3-dien-1-yl. Unless stated otherwise, a cycloalkenyl substituent group or moiety may include monocyclic, bicyclic or polycyclic hydrocarbyl rings.

An "aryl" substituent group or an aryl moiety in a substituent group refers to an aromatic hydrocarbyl ring. The term "aryl" includes monocyclic aromatic hydrocarbons and polycyclic fused ring aromatic hydrocarbons wherein all of the fused ring systems (excluding any ring systems which are part of or formed by optional substituents) are aromatic. Examples of aryl groups/moieties include phenyl, naphthyl, anthracenyl and phenanthrenyl. Unless stated otherwise, the term "aryl" does not include "heteroaryl".

A "heteroaryl" substituent group or a heteroaryl moiety in a substituent group refers to an aromatic heterocyclic group or moiety. The term "heteroaryl" includes monocyclic aromatic heterocycles and polycyclic fused ring aromatic heterocycles wherein all of the fused ring systems (excluding any ring systems which are part of or formed by optional substituents) are aromatic. Examples of heteroaryl groups/moieties include the following: 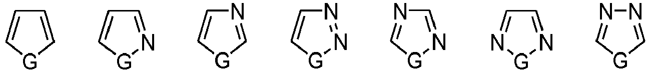 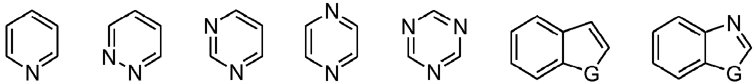 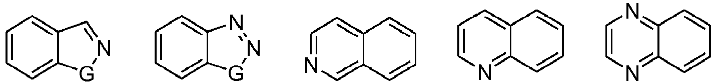 wherein G = O, S or NH.

Unless stated otherwise, where a cyclic group or moiety is stated to be non-aromatic, such as a cycloalkyl, cycloalkenyl or non-aromatic heterocyclic group, it is to be understood that the group or moiety, excluding any ring systems which are part of or formed by optional substituents, is non-aromatic. Similarly, where a cyclic group or moiety is stated to be aromatic, such as an aryl or a heteroaryl group, it is to be understood that the group or moiety, excluding any ring systems which are part of or formed by optional substituents, is aromatic. A cyclic group or moiety is considered non-aromatic, when it does not have any tautomers that are aromatic. When a cyclic group or moiety has a tautomer that is aromatic, it is considered aromatic, even if it has tautomers that are not aromatic.

By way of example, the following are considered aromatic heterocyclic groups, because they have an aromatic tautomer:

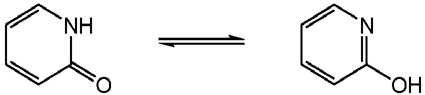

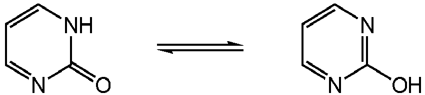

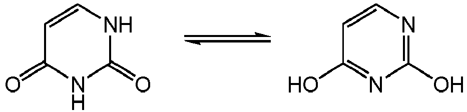

For the avoidance of doubt, the term "non-aromatic heterocyclic group" does not exclude heterocyclic groups or moieties which may possess aromatic character only by virtue of mesomeric charge separation.

For example, the following is considered a non-aromatic heterocyclic group, because it does not have an aromatic tautomer: 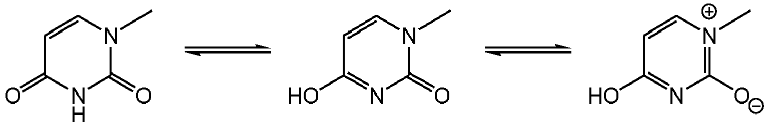 because the last shown structure is not taken into consideration because of mesomeric charge separation.

For the purposes of the present specification, where a combination of moieties is referred to as one group, for example, arylalkyl, arylalkenyl, arylalkynyl, alkylaryl, alkenylaryl or alkynylaryl, the last mentioned moiety contains the atom by which the group is attached to the rest of the molecule. An example of an arylalkyl group is benzyl.

For the purposes of the present specification, in an optionally substituted group or moiety:
(i) each hydrogen atom may optionally be replaced by a group independently selected from halo; -CN; -NO₂; -N₃; -R^{β}; -OH; -OR^{β}; -R^{α}-halo; -R^{α}-CN; -R^{α}-NO₂; -R^{α}-N₃; -R^{α}-R^{β}; -R^{α}-OH; -R^{α}-OR^{β}; -SH; -SR^{β}; -SOR^{β}; -SO₂H; -SO₂R^{β}; -SO₂NH₂; -SO₂NHR^{β}; -SO₂N(R^{β})₂; -R^{α}-SH; -R^{α}-SR^{β}; -R^{α}-SOR^{β}; -R^{α}-SO₂H; -R^{α}-SO₂R^{β}; -R^{α}-SO₂NH₂; -R^{α}-SO₂NHR^{β}; -R^{α}-SO₂N(R^{β})₂; -NH₂; -NHR^{β}; -N(R^{β})₂; -R^{α}-NH₂; -R^{α}-NHR^{β}; -R^{α}-N(R^{β})₂; -CHO; -COR^{β}; -COOH; -COOR^{β}; -OCOR^{β}; -R^{α}-CHO; -R^{α}-COR^{β}; -R^{α}-COOH; -R^{α}-COOR^{β}; -R^{α}-OCOR^{β}; -NH-CHO; -NR^{β}-CHO; -NH-COR^{β}; -NR^{β}-COR^{β}; -CONH₂; -CONHR^{β}; -CON(R^{β})₂; -R^{α}-NH-CHO; -R^{α}-NR^{β}-CHO; -R^{α}-NH-COR^{β}; -R^{α}-NR^{β}-COR^{β}; -R^{α}-CONH₂; -R^{α}-CONHR^{β}; -R^{α}-CON(R^{β})₂; -O-R^{α}-OH; -O-R^{α}-OR^{β}; -O-R^{α}-NH₂; -O-R^{α}-NHR^{β}; -O-R^{α}-N(R^{β})₂; -NH-R^{α}-OH; -NH-R^{α}-OR^{β}; -NH-R^{α}-NH₂; -NH-R^{α}-NHR^{β}; -NH-R^{α}-N(R^{β})₂; -NR^{β}-R^{α}-OH; -NR^{β}-R^{α}-OR^{β}; -NR^{β}-R^{α}-NH₂; -NR^{β}-R^{α}-NHR^{β}; or -NR^{β}-R^{α}-N(R^{β})₂; and/or
(ii) any two hydrogen atoms attached to the same carbon atom may optionally be replaced by a π-bonded substituent independently selected from oxo (=O), =S, =NH or =NR^{β}; and/or
(iii) any two hydrogen atoms attached to the same or different atoms, within the same optionally substituted group or moiety, may optionally be replaced by a bridging substituent independently selected from -O-, -S-, -NH-, -N(R^{β})- or -R^{α}-;

wherein each -R^{α}- is independently selected from an alkylene, alkenylene or alkynylene group, wherein the alkylene, alkenylene or alkynylene group contains from 1 to 6 atoms in its backbone, wherein one or more carbon atoms in the backbone of the alkylene, alkenylene or alkynylene group may optionally be replaced by one or more heteroatoms N, O or S, and wherein the alkylene, alkenylene or alkynylene group may optionally be substituted with one or more halo and/or -R^{β} groups; and
wherein each -R^{β} is independently selected from a C₁-C₆ alkyl, C₂-C₆ alkenyl, C₂-C₆ alkynyl or C₂-C₆ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -C=CH, oxo (=O), or 4- to 6-membered heterocyclic group.

Typically, in an optionally substituted group or moiety:
(i) each hydrogen atom may optionally be replaced by a group independently selected from halo; -CN; -NO₂; -N₃; -R^{β}; -OH; -OR^{β}; -R^{α}-halo; -R^{α}-CN; -R^{α}-NO₂; -R^{α}-N₃; -R^{α}-R^{β}; -R^{α}-OH; -R^{α}-OR^{β}; -SH; -SR^{β}; -SOR^{β}; -SO₂H; -SO₂R^{β}; -SO₂NH₂; -SO₂NHR^{β}; -SO₂N(R^{β})₂; -R^{α}-SH; -R^{α}-SR^{β}; -R^{α}-SOR^{β}; -R^{α}-SO₂H; -R^{α}-SO₂R^{β}; -R^{α}-SO₂NH₂; -R^{α}-SO₂NHR^{β}; -R^{α}-SO₂N(R^{β})₂; -NH₂; -NHR^{β}; -N(R^{β})₂; -R^{α}-NH₂; -R^{α}-NHR^{β}; -R^{α}-N(R^{β})₂; -CHO; -COR^{β}; -COOH; -COOR^{β}; -OCOR^{β}; -R^{α}-CHO; -R^{α}-COR^{β}; -R^{α}-COOH; -R^{α}-COOR^{β}; or -R^{α}-OCOR^{β}; and/or
(ii) any two hydrogen atoms attached to the same carbon atom may optionally be replaced by a π-bonded substituent independently selected from oxo (=O), =S, =NH or =NR^{β}; and/or
(iii) any two hydrogen atoms attached to the same or different atoms, within the same optionally substituted group or moiety, may optionally be replaced by a bridging substituent independently selected from -O-, -S-, -NH-, -N(R^{β})- or -R^{α}-;

wherein each -R^{α}- is independently selected from an alkylene, alkenylene or alkynylene group, wherein the alkylene, alkenylene or alkynylene group contains from 1 to 6 atoms in its backbone, wherein one or more carbon atoms in the backbone of the alkylene, alkenylene or alkynylene group may optionally be replaced by one or more heteroatoms N, O or S, and wherein the alkylene, alkenylene or alkynylene group may optionally be substituted with one or more halo and/or -R^{β} groups; and
wherein each -R^{β} is independently selected from a C₁-C₆ alkyl, C₂-C₆ alkenyl, C₂-C₆ alkynyl or C₂-C₆ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -C=CH, oxo (=O), or 4- to 6-membered heterocyclic group.

Typically, in an optionally substituted group or moiety:
(i) each hydrogen atom may optionally be replaced by a group independently selected from halo; -CN; -NO₂; -N₃; -R^{β}; -OH; -OR^{β}; -R^{α}-halo; -R^{α}-CN; -R^{α}-NO₂; -R^{α}-N₃; -R^{α}-R^{β}; -R^{α}-OH; -R^{α}-OR^{β}; -SH; -SR^{β}; -SOR^{β}; -SO₂H; -SO₂R^{β}; -SO₂NH₂; -SO₂NHR^{β}; -SO₂N(R^{β})₂; -R^{α}-SH; -R^{α}-SR^{β}; -R^{α}-SOR^{β}; -R^{α}-SO₂H; -R^{α}-SO₂R^{β}; -R^{α}-SO₂NH₂; -R^{α}-SO₂NHR^{β}; -R^{α}-SO₂N(R^{β})₂; -NH₂; -NHR^{β}; -N(R^{β})₂; -R^{α}-NH₂; -R^{α}-NHR^{β}; -R^{α}-N(R^{β})₂; -CHO; -COR^{β}; -COOH; -COOR^{β}; -OCOR^{β}; -R^{α}-CHO; -R^{α}-COR^{β}; -R^{α}-COOH; -R^{α}-COOR^{β}; or -R^{α}-OCOR^{β}; and/or
(ii) any two hydrogen atoms attached to the same carbon atom may optionally be replaced by a π-bonded substituent independently selected from oxo (=O), =S, =NH or =NR^{β}; and/or
(iii) any two hydrogen atoms attached to the same or different atoms, within the same optionally substituted group or moiety, may optionally be replaced by a bridging substituent independently selected from -O-, -S-, -NH-, -N(R^{β})- or -R^{α}-;

wherein each -R^{α}- is independently selected from an alkylene, alkenylene or alkynylene group, wherein the alkylene, alkenylene or alkynylene group contains from 1 to 6 atoms in its backbone, wherein one or more carbon atoms in the backbone of the alkylene, alkenylene or alkynylene group may optionally be replaced by one or more heteroatoms N, O or S, and wherein the alkylene, alkenylene or alkynylene group may optionally be substituted with one or more halo and/or -R^{β} groups; and
wherein each -R^{β} is independently selected from a C₁-C₆ alkyl, C₂-C₆ alkenyl, C₂-C₆ alkynyl or C₂-C₆ cyclic group, and wherein any -R^{β} may optionally be substituted with one or more C₁-C₄ alkyl, halo, -OH, or -O(C₁-C₄ alkyl) groups.

Typically a substituted group comprises 1, 2, 3 or 4 substituents, more typically 1, 2 or 3 substituents, more typically 1 or 2 substituents, and more typically 1 substituent.

Unless stated otherwise, any divalent bridging substituent (e.g. -O-, -S-, -NH-, -N(R^{β})- or -R^{α}-) of an optionally substituted group or moiety (e.g. R¹) must only be attached to the specified group or moiety and may not be attached to a second group or moiety (e.g. R²), even if the second group or moiety can itself be optionally substituted.

The term "halo" includes fluoro, chloro, bromo and iodo.

Unless stated otherwise, any reference to an element is to be considered a reference to all isotopes of that element. Thus, for example, unless stated otherwise any reference to hydrogen is considered to encompass all isotopes of hydrogen including deuterium and tritium.

Where reference is made to a hydrocarbyl or other group including one or more heteroatoms N, O or S in its carbon skeleton, or where reference is made to a carbon atom of a hydrocarbyl or other group being replaced by an N, O or S atom, what is intended is that: 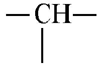 is replaced by 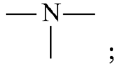
-CH₂- is replaced by -NH-, -O- or -S-;
-CH₃ is replaced by -NH₂, -OH or -SH;
-CH= is replaced by -N=;
CH₂= is replaced by NH=, O= or S=; or
CH= is replaced by N=;
provided that the resultant group comprises at least one carbon atom. For example, methoxy, dimethylamino and aminoethyl groups are considered to be hydrocarbyl groups including one or more heteroatoms N, O or S in their carbon skeleton.

In the context of the present specification, unless otherwise stated, a Cₓ-C_{y} group is defined as a group containing from x to y carbon atoms. For example, a C₁-C₄ alkyl group is defined as an alkyl group containing from 1 to 4 carbon atoms. Optional substituents and moieties are not taken into account when calculating the total number of carbon atoms in the parent group substituted with the optional substituents and/or containing the optional moieties. For the avoidance of doubt, replacement heteroatoms, e.g. N, O or S, are not to be counted as carbon atoms when calculating the number of carbon atoms in a Cₓ-C_{y} group. For example, a morpholinyl group is to be considered a C₄ heterocyclic group, not a C₆ heterocyclic group.

As will be understood, ring A is a substituted phenyl group. Ring A is therefore aromatic.

In one embodiment ring A is monocyclic. In such an embodiment, the groups R¹ and, if present, R³ are monovalent, but may be or include cyclic groups.

As will be understood, R¹ is monovalent and may be directly attached to any carbon atom of ring A, other than to the carbon atom that is directly attached to the sulfur atom of the sulfonyl group. For example, R¹ may be attached at an ortho-, meta- or para- position of ring A.

For the purposes of the present specification, where it is stated that a first atom or group is "directly attached" to a second atom or group it is to be understood that the first atom or group is covalently bonded to the second atom or group with no intervening atom(s) or groups being present. So, for example, for the group -(C=O)N(CH₃)₂, the carbon atom of each methyl group is directly attached to the nitrogen atom and the carbon atom of the carbonyl group is directly attached to the nitrogen atom, but the carbon atom of the carbonyl group is not directly attached to the carbon atom of either methyl group.

Typically, R¹ is attached at a meta- or para-position of ring A. More typically, R¹ is attached at a meta-position of ring A, i.e. the compound is a compound of formula (Ia): 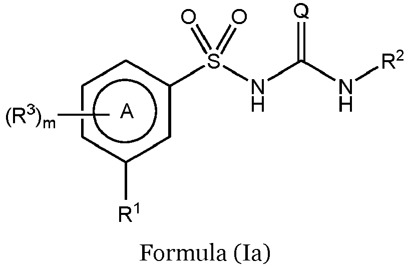 wherein R¹, R², R³, m and Q are as defined herein.

In one embodiment, R¹ is a monovalent group comprising an aryl or a heteroaryl group.

In one embodiment, R¹ is R¹¹-L-, wherein:
L is a bond or an alkylene, alkenylene or alkynylene group, wherein one or more carbon atoms in the backbone of the alkylene, alkenylene or alkynylene group may optionally be replaced by one or more heteroatoms N, O or S, and wherein the alkylene, alkenylene or alkynylene group may optionally be substituted; and
R¹¹ is an aryl or a heteroaryl group, wherein the aryl or the heteroaryl group may optionally be substituted.

For the avoidance of doubt, it is noted that it is a ring atom of the aryl or the heteroaryl group of R¹¹ that is directly attached to L, or (where L is a bond) to a ring atom of ring A, not any optional substituent.

As will be appreciated, when R¹ is R¹¹-L- and L is a bond, R¹ is R¹¹. In such an embodiment, a ring atom of the aryl group or a ring atom of the heteroaryl group is directly attached to a ring atom of ring A.

The aryl or the heteroaryl group of R¹, e.g. R¹¹, may be monocyclic, bicyclic, tricyclic or polycyclic, wherein the aryl or the heteroaryl group may optionally be substituted. Typically, the aryl or the heteroaryl group of R¹, e.g. R¹¹, is a monocyclic or a bicyclic group. More typically, the aryl or the heteroaryl group of R¹, e.g. R¹¹, is monocyclic. Where the aryl or the heteroaryl group of R¹ is monocyclic, it may optionally be substituted with any monovalent substituent, such as those defined herein, but may not be substituted with a divalent bridging substituent (e.g. -R^{α}-) so as to form a bridged or fused substituent.

Where the aryl or the heteroaryl group of R¹ e.g. R¹¹, is monocyclic, typically the heteroaryl group contains from one to three heteroatoms independently selected from oxygen, nitrogen and sulfur in its ring structure. More typically, the monocyclic heteroaryl group contains a single heteroatom selected from oxygen, nitrogen and sulfur in its ring structure, or the monocyclic heteroaryl group contains one nitrogen atom and one further heteroatom selected from oxygen, nitrogen and sulfur in its ring structure. As will be understood, in such an embodiment the remainder of the atoms in the monocyclic hetroaryl ring structure are carbons atoms.

Where the aryl or the heteroaryl group of R¹, e.g. R¹¹, is bicyclic, tricyclic or polycyclic, each ring in the bicyclic, tricyclic or polycyclic system is aromatic and is fused to at least one other ring in the system. For example, where the aryl or the heteroaryl group of R¹, e.g. R¹¹, is a bicyclic group, the aryl or the heteroaryl group may be a naphthalenyl, indolizinyl, indolyl, isoindolyl, benzofuranyl, benzothiophenyl, indazolyl, benzimidazolyl, benzothiazolyl, purinyl, quinolinyl, isoquinolinyl, cinnolinyl, phthalazinyl, quinazolinyl, quinoxalinyl, naphthyridinyl, or pteridinyl group. Where the aryl or the heteroaryl group of R¹ is bicyclic or tricyclic, the bicyclic or tricyclic aryl or heteroaryl group may optionally be substituted with any monovalent substituent, such as those defined herein, but may not be substituted with a divalent bridging substituent (e.g. -R^{α}-) so as to form a bridged or fused substituent.

In one embodiment, R¹ is a monovalent group comprising a monocyclic phenyl group, a 5- or 6-membered monocyclic heteroaryl group, a bicyclic naphthalenyl group, or an 8-to 10-membered bicyclic heteroaryl group. Typically, R¹ is a monovalent group comprising a monocyclic phenyl group or a 5- or 6-membered monocyclic heteroaryl group.

In one embodiment, R¹¹ is selected from a phenyl group, a 5- or 6-membered heteroaryl group, a naphthalenyl group, or an 8- to 10-membered bicyclic heteroaryl group, wherein the phenyl group, the 5- or 6-membered heteroaryl group, the naphthalenyl group, or the 8- to 10-membered bicyclic heteroaryl group may optionally be substituted with one or more monovalent substituents. Typically, R¹¹ is selected from a phenyl group or a 5- or 6-membered heteroaryl group, wherein the phenyl group or the 5- or 6-membered heteroaryl group may optionally be substituted with one or more monovalent substituents. Most typically, R¹¹ is selected from a phenyl, pyrrolyl, imidazolyl, pyrazolyl, triazolyl, furanyl, oxazolyl, isoxazolyl, thiophenyl, thiazolyl, isothiazolyl, pyridinyl, pyrazinyl, pyrimidinyl, pyridazinyl or triazinyl group, any of which may optionally be substituted with one or more monovalent substituents.

The aryl or the heteroaryl group of R¹, e.g. R¹¹, may be unsubstituted or substituted with one or more substituents, such as any of those defined herein. In one embodiment, the aryl or the heteroaryl group of R¹, e.g. R¹¹, is unsubstituted. In one embodiment, R¹ is selected from an unsubstituted phenyl or an unsubstituted pyridinyl group, such as an unsubstituted pyridin-4-yl group.

In another embodiment, the aryl or the heteroaryl group of R¹ may be part of a partially aromatic bicyclic, tricyclic or polycyclic group, wherein at least one ring structure in the bicyclic, tricyclic or polycyclic group is non-aromatic and at least one ring structure is aromatic.

For example. R¹ may be R¹²-L-, wherein:
L is a bond or an alkylene, alkenylene or alkynylene group, wherein one or more carbon atoms in the backbone of the alkylene, alkenylene or alkynylene group may optionally be replaced by one or more heteroatoms N, O or S, and wherein the alkylene, alkenylene or alkynylene group may optionally be substituted; and
R¹² is a bicyclic, tricyclic or polycyclic group, wherein at least one ring structure in the bicyclic, tricyclic or polycyclic group is non-aromatic and at least one ring structure is aromatic, and wherein the bicyclic, tricyclic or polycyclic group may optionally be substituted.

For the avoidance of doubt, it is noted that it is a ring atom of the bicyclic, tricyclic or polycyclic group of R¹² that is directly attached to L, or (where L is a bond) to a ring atom of ring A, not any optional substituent. Typically, the ring of the bicyclic, tricyclic or polycyclic group of R¹² that is directly attached to L or (where L is a bond) to a ring atom of ring A is aromatic, such that the bicyclic, tricyclic or polycyclic group may be seen as an aryl or heteroaryl group substituted with a saturated or partially unsaturated divalent bridging substituent so as to form a fused substituent.

As will be appreciated, when R¹ is R¹²-L- and L is a bond, R¹ is R¹². In such an embodiment, a ring atom of the bicyclic, tricyclic or polycyclic group of R¹² is directly attached to a ring atom of ring A.

Where R¹ comprises a partially aromatic bicyclic, tricyclic or polycyclic group, e.g. R¹², any non-aromatic ring structure within such a group may be a non-aromatic hydrocarbyl ring structure or a non-aromatic heterocyclic ring structure. Similarly, any aromatic ring structure may be an aromatic hydrocarbyl ring structure or an aromatic heterocyclic ring structure.

Typically, where R¹ comprises a partially aromatic bicyclic, tricyclic or polycyclic group, e.g. R¹², the bicyclic, tricyclic or polycyclic group is a fused bicyclic, a fused tricyclic or a fused polycyclic group, wherein at least one fused ring structure is aromatic and at least one fused ring structure is non-aromatic. In such a system it is to be understood that each ring in the fused bicyclic, fused tricyclic or fused polycyclic group, excluding any optional substituents, is fused to at least one other ring in the group.

More typically, where R¹ comprises a partially aromatic bicyclic, tricyclic or polycyclic group, e.g. R¹², the bicyclic, tricyclic or polycyclic group is a fused bicyclic or a fused tricyclic group. Most typically, where R¹ comprises a partially aromatic bicyclic, tricyclic or polycyclic group, e.g. R¹², the bicyclic, tricyclic or polycyclic group is a fused bicyclic group. Typically, the partially aromatic fused bicyclic group, e.g. R¹², is an optionally substituted 7- to 10-membered fused bicyclic group wherein one ring structure in the fused bicyclic group is aromatic and one is non-aromatic. Examples of such partially aromatic fused bicyclic groups include 3H-indolyl, indolinyl, 4H-quinolizinyl, indanyl and tetralinyl groups.

Where R¹ comprises a partially aromatic bicyclic or tricyclic group, e.g. R¹², the partially aromatic bicyclic or tricyclic group may optionally be substituted with any monovalent substituent or any divalent π-bonded substituent, such as those defined herein, but may not be substituted with a divalent bridging substituent (e.g. -O-, -S-, -NH-, -N(R^{β})- or -R^{α}-) so as to form a bridged, fused or spiro substituent.

In another embodiment, R¹ is a monovalent group comprising a heterocyclic group, wherein the heterocyclic group contains at least one nitrogen atom in its ring structure.

In one embodiment, R¹ is R¹³-L-, wherein:
L is a bond or an alkylene, alkenylene or alkynylene group, wherein one or more carbon atoms in the backbone of the alkylene, alkenylene or alkynylene group may optionally be replaced by one or more heteroatoms N, O or S, and wherein the alkylene, alkenylene or alkynylene group may optionally be substituted; and
R¹³ is a heterocyclic group, wherein the heterocyclic group contains at least one nitrogen atom in its ring structure, and wherein the heterocyclic group may optionally be substituted.

For the avoidance of doubt, it is noted that it is a ring atom of the heterocyclic group of R¹³ that is directly attached to L, or (where L is a bond) to a ring atom of ring A, not any optional substituent.

As will be appreciated, when R¹ is R¹³-L- and L is a bond, R¹ is R¹³. In such an embodiment, a ring atom of the heterocyclic group is directly attached to a ring atom of ring A.

The heterocyclic group of R¹, e.g. R¹³, may be monocyclic, bicyclic, tricyclic or polycyclic, wherein the heterocyclic group may optionally be substituted. Typically, where the heterocyclic group of R¹, e.g. R¹³, is bicyclic, tricyclic or polycyclic, the heterocyclic group of R¹, e.g. R¹³, is a fused bicyclic, a fused tricyclic or a fused polycyclic system. In such a system it is to be understood that each ring in the fused bicyclic, fused tricyclic or fused polycyclic group, excluding any optional substituents, is fused to at least one other ring in the group.

Typically, when R¹³ is a heterocyclic bicyclic, tricyclic or polycyclic group, the ring of R¹³ that is directly attached to L or (where L is a bond) to a ring atom of ring A contains at least one nitrogen atom in its ring structure.

Typically, the heterocyclic group of R¹, e.g. R¹³, is a monocyclic or a fused bicyclic group. More typically, the heterocyclic group of R¹, e.g. R¹³, is monocyclic. Where the heterocyclic group of R¹, e.g. R¹³, is monocyclic, it may optionally be substituted with any monovalent substituent or any divalent π-bonded substituent, such as those defined herein, but may not be substituted with a divalent bridging substituent (e.g. -O-, -S-, -NH-, -N(R^{β})- or -R^{α}-) so as to form a bridged, fused or spiro substituent.

Where the heterocyclic group of R¹ e.g. R¹³, is monocyclic, typically the heterocyclic group contains one nitrogen atom and zero, one or two further heteroatoms independently selected from oxygen, nitrogen and sulfur in its ring structure. More typically, the monocyclic heterocyclic group contains a single nitrogen atom in its ring structure, or the monocyclic heterocyclic group contains one nitrogen atom and one further heteroatom selected from oxygen, nitrogen and sulfur in its ring structure. As will be understood, in such an embodiment the remainder of the atoms in the monocyclic heterocyclic ring structure are carbons atoms.

In one embodiment, the heterocyclic group of R¹, e.g. R¹³, is a heteroaryl group containing at least one nitrogen atom in its ring structure.

The heteroaryl group of R¹, e.g. R¹³, may be monocyclic, bicyclic, tricyclic or polycyclic, wherein the heteroaryl group may optionally be substituted. Typically, the heteroaryl group of R¹, e.g. R¹³, is a monocyclic or a bicyclic group. More typically, heteroaryl group of R¹, e.g. R¹³, is monocyclic. Where the heteroaryl group of R¹ is monocyclic, it may optionally be substituted with any monovalent substituent, such as those defined herein, but may not be substituted with a divalent bridging substituent (e.g. -R^{α}-) so as to form a bridged or fused substituent.

Where the heteroaryl group of R¹, e.g. R¹³, is bicyclic, tricyclic or polycyclic, each ring in the bicyclic, tricyclic or polycyclic system is aromatic and is fused to at least one other ring in the system. For example, where the heteroaryl group of R¹, e.g. R¹³, is a fused bicyclic group containing at least one nitrogen atom in its ring structure, the heteroaryl group may be an indolizinyl, indolyl, isoindolyl, indazolyl, benzimidazolyl, benzothiazolyl, purinyl, quinolinyl, isoquinolinyl, cinnolinyl, phthalazinyl, quinazolinyl, quinoxalinyl, naphthyridinyl, or pteridinyl group. Where the heteroaryl group of R¹ is bicyclic or tricyclic, the bicyclic or tricyclic heteroaryl group may optionally be substituted with any monovalent substituent, such as those defined herein, but may not be substituted with a divalent bridging substituent (e.g. -R^{α}-) so as to form a bridged or fused substituent.

In one embodiment, R¹ is a monovalent group comprising a 5- or 6-membered monocyclic heteroaryl group, or an 8- to 10-membered bicyclic heteroaryl group, wherein the 5- or 6-membered monocyclic heteroaryl group or the 8- to 10-membered bicyclic heteroaryl group contains at least one nitrogen atom in its ring structure. Typically, R¹ is a monovalent group comprising a 5- or 6-membered monocyclic heteroaryl group, wherein the 5- or 6-membered monocyclic heteroaryl group contains at least one nitrogen atom in its ring structure.

In one embodiment, R¹³ is selected from a 5- or 6-membered heteroaryl group, or an 8-to 10-membered bicyclic heteroaryl group, wherein the 5- or 6-membered heteroaryl group or the 8- to 10-membered bicyclic heteroaryl group contains at least one nitrogen atom in its ring structure, and wherein the 5- or 6-membered heteroaryl group or the 8-to 10-membered bicyclic heteroaryl group may optionally be substituted with one or more monovalent substituents. Typically, R¹³ is a 5- or 6-membered heteroaryl group, wherein the 5- or 6-membered heteroaryl group contains at least one nitrogen atom in its ring structure, and wherein the 5- or 6-membered heteroaryl group may optionally be substituted with one or more monovalent substituents. More typically, R¹³ is selected from a pyrrolyl, imidazolyl, pyrazolyl, triazolyl, oxazolyl, isoxazolyl, thiazoly, isothiazolyl, pyridinyl, pyrazinyl, pyrimidinyl, pyridazinyl or triazinyl group, any of which may optionally be substituted with one or more monovalent substituents.

Where the heterocyclic group of R¹, e.g. R¹³, is a heteroaryl group containing at least one nitrogen atom in its ring structure, the heteroaryl group may be unsubstituted or substituted with one or more substituents, such as any of those defined herein. In one embodiment, the heteroaryl group of R¹, e.g. R¹³, is an unsubstituted heteroaryl group, wherein the heteroaryl group contains at least one nitrogen atom in its ring structure. In one embodiment, R¹ is an unsubstituted pyridinyl group, such as an unsubstituted pyridin-4-yl group.

In another embodiment, the heterocyclic group of R¹, e.g. R¹³, is a partially aromatic bicyclic, tricyclic or polycyclic group, wherein at least one ring structure in the bicyclic, tricyclic or polycyclic group is non-aromatic, at least one ring structure is aromatic, and at least one ring structure contains a nitrogen atom.

Where the heterocyclic group of R¹, e.g. R¹³, is a partially aromatic bicyclic, tricyclic or polycyclic group, any non-aromatic ring structure within such a group may be a non-aromatic hydrocarbyl ring structure or a non-aromatic heterocyclic ring structure. Similarly, any aromatic ring structure may be an aromatic hydrocarbyl ring structure or an aromatic heterocyclic ring structure. However, at least one ring structure must be a non-aromatic nitrogen containing heterocyclic ring structure or an aromatic nitrogen containing heterocyclic ring structure.

Typically, where the heterocyclic group of R¹, e.g. R¹³, is a partially aromatic bicyclic, tricyclic or polycyclic group, the heterocyclic group is a fused bicyclic, a fused tricyclic or a fused polycyclic group, wherein at least one fused ring structure is aromatic, at least one fused ring structure is non-aromatic, and at least one fused ring structure contains a nitrogen atom. In such a system it is to be understood that each ring in the fused bicyclic, fused tricyclic or fused polycyclic group, excluding any optional substituents, is fused to at least one other ring in the group.

In one embodiment, when R¹³ is a partially aromatic bicyclic, tricyclic or polycyclic group, the ring of R¹³ that is directly attached to L or (where L is a bond) to a ring atom of ring A is aromatic and contains at least one nitrogen atom in its ring structure, such that the partially aromatic bicyclic, tricyclic or polycyclic group may be seen as a heteroaryl group substituted with a saturated or partially unsaturated divalent bridging substituent so as to form a fused non-aromatic substituent.

In another embodiment, when R¹³ is a partially aromatic bicyclic, tricyclic or polycyclic group, the ring of R¹³ that is directly attached to L or (where L is a bond) to a ring atom of ring A is non-aromatic and contains at least one nitrogen atom in its ring structure, such that the partially aromatic bicyclic, tricyclic or polycyclic group may be seen as a non-aromatic heterocyclic group substituted with an unsaturated divalent bridging substituent so as to form a fused aromatic substituent.

Typically, where the heterocyclic group of R¹, e.g. R¹³, is a partially aromatic bicyclic, tricyclic or polycyclic group, the bicyclic, tricyclic or polycyclic group is a fused bicyclic or a fused tricyclic group. Most typically, where the heterocyclic group of R¹, e.g. R¹³, is a partially aromatic bicyclic, tricyclic or polycyclic group, the bicyclic, tricyclic or polycyclic group is a fused bicyclic group. Typically, the partially aromatic fused bicyclic group, e.g. R¹³, is an optionally substituted 7- to 10-membered fused bicyclic group wherein one ring structure in the fused bicyclic group is aromatic, one ring structure is non-aromatic, and at least one ring structure contains a nitrogen atom. Examples of such partially aromatic fused bicyclic groups include 3H-indolyl, indolinyl and 4H-quinolizinyl groups.

In another embodiment, the heterocyclic group of R¹, e.g. R¹³, is a non-aromatic heterocyclic group containing at least one nitrogen atom in its ring structure.

Where the non-aromatic heterocyclic group of R¹, e.g. R¹³, is bicyclic, tricyclic or polycyclic, each ring in bicyclic, tricyclic or polycyclic system, excluding any optional substituents, is non-aromatic.

Typically, the non-aromatic heterocyclic group of R¹, e.g. R¹³, is monocyclic.

In one embodiment, R¹ is a monovalent group comprising a 3- to 7-membered monocyclic non-aromatic heterocyclic group or a 7- to 10-membered bicyclic non-aromatic heterocyclic group, wherein the 3- to 7-membered monocyclic non-aromatic heterocyclic group or the 7- to 10-membered bicyclic non-aromatic heterocyclic group contains at least one nitrogen atom in its ring structure. Typically, R¹ is a monovalent group comprising a 4-, 5- or 6-membered monocyclic non-aromatic heterocyclic group, wherein the 4-, 5- or 6-membered monocyclic non-aromatic heterocyclic group contains at least one nitrogen atom in its ring structure.

In one embodiment, R¹³ is selected from a 3- to 7-membered monocyclic non-aromatic heterocyclic group, or a 7- to 10-membered bicyclic non-aromatic heterocyclic group, wherein the 3- to 7-membered monocyclic non-aromatic heterocyclic group or the 7- to 10-membered bicyclic non-aromatic heterocyclic group contains at least one nitrogen atom in its ring structure, and wherein the 3- to 7-membered monocyclic non-aromatic heterocyclic group or the 7- to 10-membered bicyclic non-aromatic heterocyclic group may optionally be substituted with one or more monovalent and/or divalent π-bonded substituents. Typically, R¹³ is selected from a 4-, 5- or 6-membered monocyclic non-aromatic heterocyclic group, wherein the 4-, 5- or 6-membered monocyclic non-aromatic heterocyclic group contains at least one nitrogen atom in its ring structure, and wherein the 4-, 5- or 6-membered monocyclic non-aromatic heterocyclic group may optionally be substituted with one or more monovalent and/or divalent π-bonded substituents.

Examples of 4-, 5- or 6-membered monocyclic non-aromatic heterocyclic groups, which contain at least one nitrogen atom in their ring structure, and which may be optionally substituted, include:

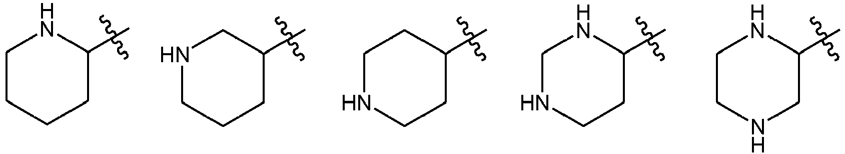

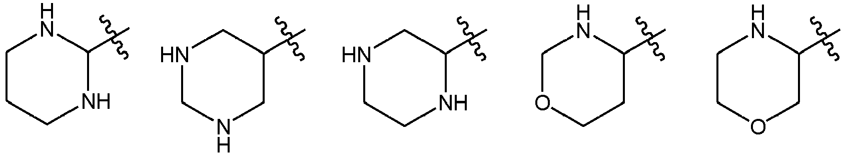

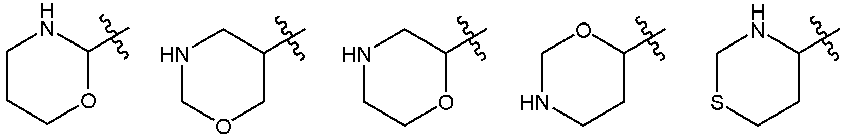

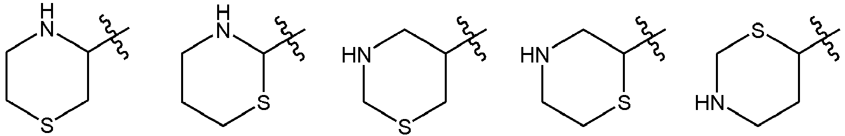

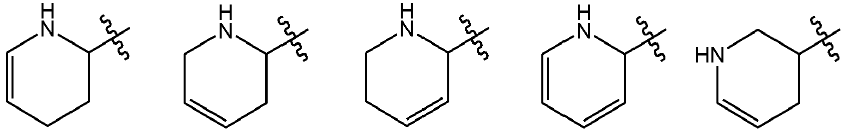

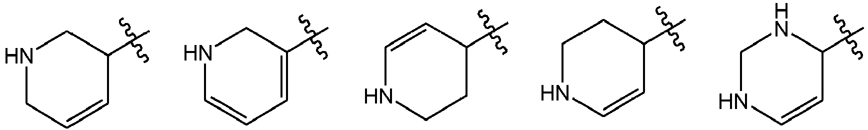

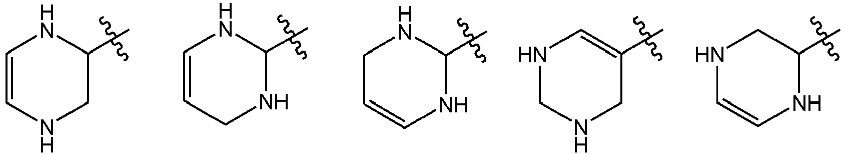

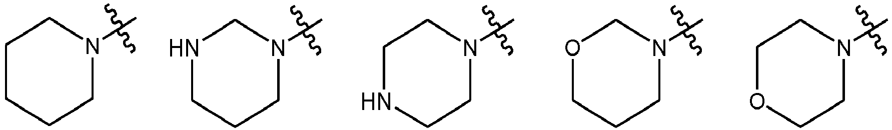

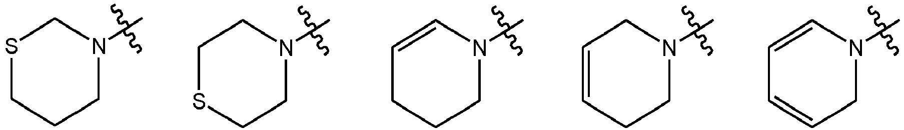

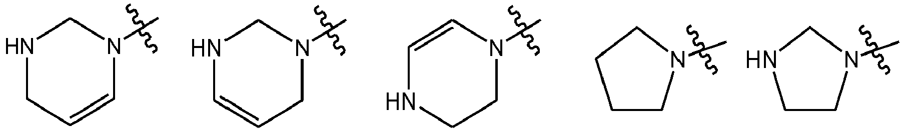

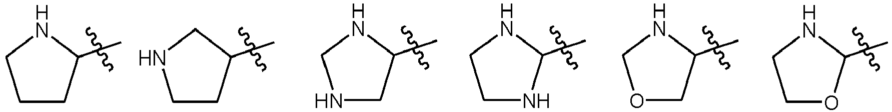

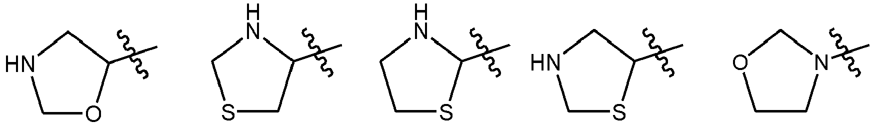

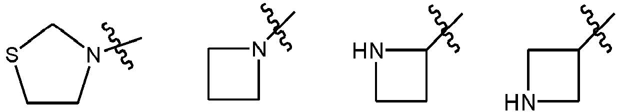

The non-aromatic heterocyclic group of R¹, e.g. R¹³, may be fully saturated or partially unsaturated. Accordingly, the non-aromatic heterocyclic group of R¹ may comprise one or more double bonds in the cyclic ring, provided the cyclic ring is non-aromatic. The non-aromatic heterocyclic group of R¹ does not have any tautomers that are aromatic.

In one embodiment, the non-aromatic heterocyclic group of R¹, e.g. R¹³, is fully saturated. As will be understood, in such an embodiment all of the ring atoms of the non-aromatic hetrocyclic group, when considered after any optional substitution, are *sp³* hybridised. Thus, for example, in such an embodiment the non-aromatic heterocyclic group may not be substituted with a π-bonded substituent such as an oxo (=O) group.

In one embodiment, R¹ is a monovalent group comprising a 3- to 7-membered fully saturated monocyclic heterocyclic group, or a 7- to 10-membered fully saturated bicyclic heterocyclic group, wherein the 3- to 7-membered fully saturated monocyclic heterocyclic group or the 7- to 10-membered fully saturated bicyclic heterocyclic group contains at least one nitrogen atom in its ring structure. Typically, R¹ is a monovalent group comprising a 4-, 5- or 6-membered fully saturated monocyclic heterocyclic group, wherein the 4-, 5- or 6-membered fully saturated monocyclic heterocyclic group contains at least one nitrogen atom in its ring structure.

In one embodiment, R¹³ is selected from a 3- to 7-membered fully saturated monocyclic heterocyclic group, or a 7- to 10-membered fully saturated bicyclic heterocyclic group, wherein the 3- to 7-membered fully saturated monocyclic heterocyclic group or the 7-to 10-membered fully saturated bicyclic heterocyclic group contains at least one nitrogen atom in its ring structure, and wherein the 3- to 7-membered fully saturated monocyclic heterocyclic group or the 7- to 10-membered fully saturated bicyclic heterocyclic group may optionally be substituted with one or more monovalent substituents. Typically, R¹³ is selected from a 4-, 5- or 6-membered fully saturated monocyclic heterocyclic group, wherein the 4-, 5- or 6-membered fully saturated monocyclic heterocyclic group contains at least one nitrogen atom in its ring structure, and wherein the 4-, 5- or 6-membered fully saturated monocyclic heterocyclic group may optionally be substituted with one or more monovalent substituents. Most typically in such an embodiment, R¹ is selected from a pyrrolidinyl, pyrazolidinyl, imidazolidinyl, oxazolidinyl, isoxazolidinyl, piperidinyl, piperazinyl or morpholinyl group, any of which may optionally be substituted.

Where the heterocyclic group of R¹, e.g. R¹³, is a non-aromatic heterocyclic group containing at least one nitrogen atom in its ring structure, the non-aromatic heterocyclic group may be unsubstituted or substituted with one or more substituents, such as any of those defined herein. In one embodiment, the non-aromatic heterocyclic group of R¹, e.g. R¹³, is an unsubstituted non-aromatic heterocyclic group, such as an unsubstituted fully saturated heterocyclic group, wherein the heterocyclic group contains at least one nitrogen atom in its ring structure. For example, R¹ may be an unsubstituted morpholinyl group, such as an unsubstituted morpholin-4-yl group.

Any aryl, heteroaryl, heterocyclic or partially aromatic group of R¹, e.g. R¹¹, R¹² or R¹³, may be optionally substituted with one or more substituents, such as those defined herein.

Where an aryl or a heteroaryl group of R¹, or an aromatic ring structure of a partially aromatic bicyclic, tricyclic or polycyclic group of R¹ is substituted, typically it is substituted with one or more monovalent substituents. Typically, the one or more monovalent substituents are independently selected from a halo, -OH, -NO₂, -NH₂, -N₃, -SH, -SO₂H, -SO₂NH₂ or a saturated or unsaturated hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton. More typically, where an aryl or a heteroaryl group of R¹, or an aromatic ring structure of a partially aromatic bicyclic, tricyclic or polycyclic group of R¹ is substituted, it is substituted with one or more groups independently selected from halo, -OH, -NO₂, -NH₂, -CN, -R¹⁴, -OR¹⁴, -NHR¹⁴ or -N(R¹⁴)₂, wherein each R¹⁴ is independently selected from a C₁-C₆ alkyl, C₁-C₆ haloalkyl, C₃-C₆ cycloalkyl or C₃-C₆ halocycloalkyl group, or any two R¹⁴ directly attached to the same nitrogen atom may together form a C₂-C₅ alkylene or C₂-C₅ haloalkylene group. More typically still, where an aryl or a heteroaryl group of R¹, or an aromatic ring structure of a partially aromatic bicyclic, tricyclic or polycyclic group of R¹ is substituted, it is substituted with one or more groups independently selected from halo, -OH, -NO₂, -CN, -R¹⁴ or -OR¹⁴, wherein each R¹⁴ is independently selected from a C₁-C₄ alkyl, C₁-C₄ haloalkyl, C₃-C₄ cycloalkyl or C₃-C₄ halocycloalkyl group. Most typically, where an aryl or a heteroaryl group of R¹, or an aromatic ring structure of a partially aromatic bicyclic, tricyclic or polycyclic group of R¹ is substituted, it is substituted with one or more chloro, fluoro, -CN, -Me and/or -OMe groups, wherein any methyl (Me) group may optionally be substituted with one or more fluoro and/or chloro groups.

Typically, where an aryl or a heteroaryl group of R¹, or an aromatic ring structure of a partially aromatic bicyclic, tricyclic or polycyclic group of R¹ is substituted, it is substituted with one, two or three substituents such as any of those described herein. More typically, where an aryl or a heteroaryl group of R¹, or an aromatic ring structure of a partially aromatic bicyclic, tricyclic or polycyclic group of R¹ is substituted, it is substituted with one or two substituents. Most typically, where an aryl or a heteroaryl group of R¹, or an aromatic ring structure of a partially aromatic bicyclic, tricyclic or polycyclic group of R¹ is substituted, it is substituted with one substituent.

Where a non-aromatic heterocyclic group of R¹, or a non-aromatic ring structure of a partially aromatic bicyclic, tricyclic or polycyclic group of R¹ is substituted, typically it is substituted with one or more monovalent substituents and/or one or more π-bonded substituents. Typically, the one or more monovalent substituents are independently selected from a halo, -OH, -NO₂, -NH₂, -N₃, -SH, -SO₂H, -SO₂NH₂ or a saturated or unsaturated hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton. Typically, the one or more π-bonded substituents are independently selected from =O, =S, =NH or =NR¹⁵, wherein R¹⁵ is a saturated or unsaturated hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton.

More typically, where a non-aromatic heterocyclic group of R¹, or a non-aromatic ring structure of a partially aromatic bicyclic, tricyclic or polycyclic group of R¹ is substituted, it is substituted with one or more substituents independently selected from halo, -CN, -OH, -NH₂, oxo (=O), =NH, -R¹⁵, -OR¹⁵, -NHR¹⁵, -N(R¹⁵)₂ or =NR¹⁵, wherein each R¹⁵ is independently selected from a C₁-C₆ alkyl, C₁-C₆ haloalkyl, C₃-C₆ cycloalkyl or C₃-C₆ halocycloalkyl group, or any two R¹⁵ directly attached to the same nitrogen atom may together form a C₂-C₅ alkylene or C₂-C₅ haloalkylene group. More typically still, where a non-aromatic heterocyclic group of R¹, or a non-aromatic ring structure of a partially aromatic bicyclic, tricyclic or polycyclic group of R¹ is substituted, it is substituted with one or more substituents independently selected from halo, -CN, -OH, -NH₂, -R¹⁵, -OR¹⁵, -NHR¹⁵ and -N(R¹⁵)₂, wherein each R¹⁵ is independently selected from a C₁-C₄ alkyl, C₁-C₄ haloalkyl, C₃-C₄ cycloalkyl or C₃-C₄ halocycloalkyl group, or any two R¹⁵ directly attached to the same nitrogen atom may together form a C₂-C₅ alkylene or C₂-C₅ haloalkylene group. Yet more typically, where a non-aromatic heterocyclic group of R¹, or a non-aromatic ring structure of a partially aromatic bicyclic, tricyclic or polycyclic group of R¹ is substituted, it is substituted with one or more substituents independently selected from fluoro, chloro, -OH, -NH₂, -Me, -Et, -OMe, -OEt, -NHMe, -NHEt, -N(Me)₂, -N(Me)Et and -N(Et)₂ groups, wherein any methyl (Me) or ethyl (Et) group may optionally be substituted with one or more fluoro and/or chloro groups. Most typically, where a non-aromatic heterocyclic group of R¹, or a non-aromatic ring structure of a partially aromatic bicyclic, tricyclic or polycyclic group of R¹ is substituted, it is substituted with one or more substituents independently selected from fluoro, -OH, -Me, -Et, -OMe, -OEt, -N(Me)₂, -N(Me)Et and -N(Et)₂ groups, wherein any methyl (Me) or ethyl (Et) group may optionally be substituted with one or more fluoro groups.

Typically, where a non-aromatic heterocyclic group of R¹, or a non-aromatic ring structure of a partially aromatic bicyclic, tricyclic or polycyclic group of R¹ is substituted, it is substituted with one, two or three substituents such as any of those described herein. More typically, where a non-aromatic heterocyclic group of R¹, or a non-aromatic ring structure of a partially aromatic bicyclic, tricyclic or polycyclic group of R¹ is substituted, it is substituted with one or two substituents. Most typically, where a non-aromatic heterocyclic group of R¹, or a non-aromatic ring structure of a partially aromatic bicyclic, tricyclic or polycyclic group of R¹ is substituted, it is substituted with one substituent.

Typically where R¹ is R¹¹-L-, R¹²-L- or R¹³-L-, L is a bond or an alkylene or an alkenylene group, wherein the alkylene or alkenylene group may optionally include one or more heteroatoms N or O in its carbon skeleton, and wherein the alkylene or alkenylene group may be optionally substituted. More typically, L is a bond or an alkylene group, wherein the alkylene group may optionally include one or two heteroatoms N or O in its carbon skeleton, wherein the alkylene group may be optionally substituted.

Where L is substituted, typically it is substituted with one or more substituents independently selected from halo, -CN, -OH, -NH₂, oxo (=O) and =NH. More typically, where L is substituted, it is substituted with one or more substituents independently selected from halo, -CN, -OH, -NH₂ and oxo (=O). Most typically, where L is substituted, it is substituted with one or more substituents independently selected from fluoro, chloro and oxo (=O).

In one embodiment, L contains only atoms selected from the group consisting of carbon, hydrogen, nitrogen, oxygen and halogen atoms.

In another embodiment, L does not contain an amide group. In a further embodiment, L does not contain a carbonyl group.

Typically, L is a bond or contains from 1 to 10 atoms other than hydrogen or halogen. More typically, L is a bond or contains from 1 to 6 atoms other than hydrogen or halogen. More typically still, L is a bond or contains from 1 to 4 atoms other than hydrogen or halogen.

More typically, L is a bond or a -CH₂- group. Most typically, L is a bond.

In one embodiment, R¹ contains only atoms selected from the group consisting of carbon, hydrogen, nitrogen, oxygen and halogen atoms.

In one embodiment, R¹ contains from 3 to 20 atoms other than hydrogen or halogen. Typically, R¹ contains from 4 to 15 atoms other than hydrogen or halogen. More typically, R¹ contains from 4 to 8 atoms other than hydrogen or halogen.

As stated above, m is 0, 1, 2 or 3. More typically, m is 0, 1 or 2. More typically still, m is 0 or 1. Most typically, m is 1.

As will be understood, each R³ where present may be directly attached to any carbon atom of ring A, other than to the carbon atom that is directly attached to the sulfur atom of the sulfonyl group, or than to the carbon atom that is directly attached to R¹. For example, each R³ may be attached at an ortho-, meta- or para- position of ring A.

In one embodiment, each R³ is monovalent.

Typically, where m is 1 and R³ is monovalent, the compound is a compound of formula (Ib): 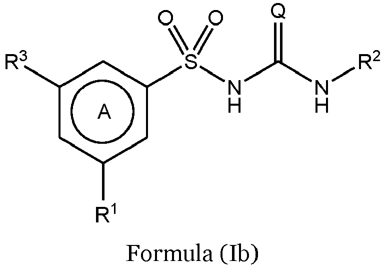 wherein R¹, R², R³ and Q are as defined herein.

In any of the above embodiments, each R³ may be independently selected from halo; -CN; -NO₂; -N₃; -R^{β}; -OH; -OR^{β}; -R^{α}-halo; -R^{α}-CN; -R^{α}-NO₂; -R^{α}-N₃; -R^{α}-R^{β}; -R^{α}-OH; -R^{α}-OR^{β}; -SH; -SR^{β}; -SOR^{β}; -SO₂H; -SO₂R^{β}; -SO₂NH₂; -SO₂NHR^{β}; -SO₂N(R^{β})₂; -R^{α}-SH; -R^{α}-SR^{β}; -R^{α}-SOR^{β}; -R^{α}-SO₂H; -R^{α}-SO₂R^{β}; -R^{α}-SO₂NH₂; -R^{α}-SO₂NHR^{β}; -R^{α}-SO₂N(R^{β})₂; -NH₂; -NHR^{β}; -N(R^{β})₂; -R^{α}-NH₂; -R^{α}-NHR^{β}; -R^{α}-N(R^{β})₂; -CHO; -COR^{β}; -COOH; -COOR^{β}; -OCOR^{β}; -R^{α}-CHO; -R^{α}-COR^{β}; -R^{α}-COOH; -R^{α}-COOR^{β}; -R^{α}-OCOR^{β}; -NH-CHO; -NR^{β}-CHO; -NH-COR^{β}; -NR^{β}-COR^{β}; -CONH₂; -CONHR^{β}; -CON(R^{β})₂; -R^{α}-NH-CHO; -R^{α}-NR^{β}-CHO; -R^{α}-NH-COR^{β}; -R^{α}-NR^{β}-COR^{β}; -R^{α}-CONH₂; -R^{α}-CONHR^{β}; -R^{α}-CON(R^{β})₂; -O-R^{α}-OH; -O-R^{α}-OR^{β}; -O-R^{α}-NH₂; -O-R^{α}-NHR^{β}; -O-R^{α}-N(R^{β})₂; -NH-R^{α}-OH; -NH-R^{α}-OR^{β}; -NH-R^{α}-NH₂; -NH-R^{α}-NHR^{β}; -NH-R^{α}-N(R^{β})₂; -NR^{β}-R^{α}-OH; -NR^{β}-R^{α}-OR^{β}; -NR^{β}-R^{α}-NH₂; -NR^{β}-R^{α}-NHR^{β}; or -NR^{β}-R^{α}-N(R^{β})₂;
wherein each -R^{α}- is independently selected from an alkylene, alkenylene or alkynylene group, wherein the alkylene, alkenylene or alkynylene group contains from 1 to 6 atoms in its backbone, wherein one or more carbon atoms in the backbone of the alkylene, alkenylene or alkynylene group may optionally be replaced by one or more heteroatoms N, O or S, and wherein the alkylene, alkenylene or alkynylene group may optionally be substituted with one or more halo and/or -R^{β} groups; and
wherein each -R^{β} is independently selected from a C₁-C₆ alkyl, C₂-C₆ alkenyl, C₂-C₆ alkynyl or C₂-C₆ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -C=CH, oxo (=O), or 4- to 6-membered heterocyclic group.

In one embodiment, each R³ is independently selected from halo; -CN; -NO₂; -N₃; -R^{β}; -OH; -OR^{β}; -R^{α}-halo; -R^{α}-CN; -R^{α}-NO₂; -R^{α}-N₃; -R^{α}-R^{β}; -R^{α}-OH; -R^{α}-OR^{β}; -SH; -SR^{β}; -SOR^{β}; -SO₂H; -SO₂R^{β}; -SO₂NH₂; -SO₂NHR^{β}; -SO₂N(R^{β})₂; -R^{α}-SH; -R^{α}-SR^{β}; -R^{α}-SOR^{β}; -R^{α}-SO₂H; -R^{α}-SO₂R^{β}; -R^{α}-SO₂NH₂; -R^{α}-SO₂NHR^{β}; -R^{α}-SO₂N(R^{β})₂; -NH₂; -NHR^{β}; -N(R^{β})₂; -R^{α}-NH₂; -R^{α}-NHR^{β}; -R^{α}-N(R^{β})₂; -CHO; -COR^{β}; -COOH; -COOR^{β}; -OCOR^{β}; -R^{α}-CHO; -R^{α}-COR^{β}; -R^{α}-COOH; -R^{α}-COOR^{β}; or -R^{α}-OCOR^{β};
wherein each -R^{α}- is independently selected from an alkylene, alkenylene or alkynylene group, wherein the alkylene, alkenylene or alkynylene group contains from 1 to 6 atoms in its backbone, wherein one or more carbon atoms in the backbone of the alkylene, alkenylene or alkynylene group may optionally be replaced by one or more heteroatoms N, O or S, and wherein the alkylene, alkenylene or alkynylene group may optionally be substituted with one or more halo and/or -R^{β} groups; and
wherein each -R^{β} is independently selected from a C₁-C₆ alkyl, C₂-C₆ alkenyl, C₂-C₆ alkynyl or C₂-C₆ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -C=CH, oxo (=O), or 4- to 6-membered heterocyclic group.

Alternatively, each R³ may be independently selected from halo; -CN; -NO₂; -N₃; -R^{β}; -OH; -OR^{β}; -R^{α}-halo; -R^{α}-CN; -R^{α}-NO₂; -R^{α}-N₃; -R^{α}-R^{β}; -R^{α}-OH; -R^{α}-OR^{β}; -SH; -SR^{β}; -SOR^{β}; -SO₂H; -SO₂R^{β}; -SO₂NH₂; -SO₂NHR^{β}; -SO₂N(R^{β})₂; -R^{α}-SH; -R^{α}-SR^{β}; -R^{α}-SOR^{β}; -R^{α}-SO₂H; -R^{α}-SO₂R^{β}; -R^{α}-SO₂NH₂; -R^{α}-SO₂NHR^{β}; -R^{α}-SO₂N(R^{β})₂; -NH₂; -NHR^{β}; -N(R^{β})₂; -R^{α}-NH₂; -R^{α}-NHR^{β}; -R^{α}-N(R^{β})₂; -CHO; -COR^{β}; -COOH; -COOR^{β}; -OCOR^{β}; -R^{α}-CHO; -R^{α}-COR^{β}; -R^{α}-COOH; -R^{α}-COOR^{β}; or -R^{α}-OCOR^{β};
wherein each -R^{α}- is independently selected from an alkylene, alkenylene or alkynylene group, wherein the alkylene, alkenylene or alkynylene group contains from 1 to 6 atoms in its backbone, wherein one or more carbon atoms in the backbone of the alkylene, alkenylene or alkynylene group may optionally be replaced by one or more heteroatoms N, O or S, and wherein the alkylene, alkenylene or alkynylene group may optionally be substituted with one or more halo and/or -R^{β} groups; and
wherein each -R^{β} is independently selected from a C₁-C₆ alkyl, C₂-C₆ alkenyl, C₂-C₆ alkynyl or C₂-C₆ cyclic group, and wherein any -R^{β} may optionally be substituted with one or more C₁-C₄ alkyl, halo, -OH, or -O(C₁-C₄ alkyl) groups.

Typically, each R³ is independently a halo, -OH, -NO₂, -NH₂, -N₃, -SH, -SO₂H, -SO₂NH, or a saturated or unsaturated hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton. Where the hydrocarbyl group of R³ is optionally substituted, typically it is substituted with one or more groups independently selected from halo, -CN, -OH, -NH₂, oxo (=O) and =NH.

More typically, each R³ is independently a halo, -NO₂, or a saturated or unsaturated hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N or O in its carbon skeleton.

Where m is 2, typically at least one R³ is selected from a halo group. Where m is 3, typically at least two R³ are selected from halo groups. Where m is 4, typically at least three R³ are selected from halo groups. Where R³ is selected from a halo group, typically R³ is selected from a fluoro, chloro or bromo group. More typically, where R³ is selected from a halo group, R³ is selected from a fluoro or chloro group. Most typically, where R³ is selected from a halo group, R³ is a fluoro group.

In one embodiment, at least one R³ is a saturated or unsaturated hydrocarbyl group, wherein the hydrocarbyl group may be straight-chained or branched, or be or include cyclic groups, wherein the hydrocarbyl group may optionally be substituted, wherein the hydrocarbyl group includes at least one oxygen atom in its carbon skeleton, and wherein the hydrocarbyl group may optionally include one or more additional heteroatoms N or O in its carbon skeleton. Typically, a hydrocarbyl group that includes at least one oxygen atom in its carbon skeleton is attached at a meta- or para-position of ring A. More typically, a hydrocarbyl group that includes at least one oxygen atom in its carbon skeleton is attached at a meta-position of ring A. Typically in such an embodiment, m is 1. For example, in one aspect of such an embodiment, the compound may be a compound of formula (Ib). Alternatively, m may be 2, 3 or 4, wherein one R³ is a hydrocarbyl group that includes at least one oxygen atom in its carbon skeleton as described according to the present embodiment, and the other R³ are independently selected from halo groups.

Where a hydrocarbyl group of R³ is optionally substituted, typically it is substituted with one or more groups independently selected from halo, -CN, -OH, -NH₂ and oxo (=O). More typically, where a hydrocarbyl group of R³ is optionally substituted, it is substituted with one or more groups independently selected from halo, -CN and -OH.

Typically, where R³ is a hydrocarbyl group, the hydrocarbyl group contains from 1 to 8 carbon atoms. More typically, the hydrocarbyl group contains from 1 to 6 carbon atoms. Most typically, the hydrocarbyl group contains from 1 to 4 carbon atoms.

In one embodiment, at least one R³ is selected from a -CHO, -COR³¹, -COOH, -COOR³¹, -CONH₂, -CONHR³¹, -CON(R³¹)₂, -CH₂OR³², -CH(R³¹)OR³², -C(R³¹)₂OR³², -CH(OR³³)₂ or -CR³¹(OR³³)₂ group, wherein
each R³¹ is independently selected from a C₁-C₆ alkyl or a C₃-C₆ cycloalkyl group, wherein the C₁-C₆ alkyl or C₃-C₆ cycloalkyl group may optionally be substituted with one or more halo groups, or wherein any two R³¹ attached to the same carbon or nitrogen atom may, together with the carbon or nitrogen atom to which they are attached, form a 3- to 6-membered cyclic group, wherein the 3- to 6-membered cyclic group may optionally be substituted with one or more halo groups;
each R³² is independently selected from hydrogen or a C₁-C₆ alkyl or a C₃-C₆ cycloalkyl group, wherein the C₁-C₆ alkyl or C₃-C₆ cycloalkyl group may optionally be substituted with one or more halo groups; and
each R³³ is independently selected from a C₁-C₆ alkyl or a C₃-C₆ cycloalkyl group, wherein the C₁-C₆ alkyl or C₃-C₆ cycloalkyl group may optionally be substituted with one or more halo groups, or wherein any two R³³ attached to the same group -CH(OR³³)₂ or -CR³¹(OR³³)₂ may, together with the two oxygen atoms to which the two R³³ are attached and the carbon atom to which the two oxygen atoms are attached, form a 4- to 6-membered heterocyclic group, wherein the 4- to 6-membered heterocyclic group may optionally be substituted with one or more halo groups.

Typically, the -CHO, -COR³¹, -COOH, -COOR³¹, -CONH₂, -CONHR³¹, -CON(R³¹)₂, -CH₂OR³², -CH(R³¹)OR³², -C(R³¹)₂OR³², -CH(OR³³)₂ or -CR³¹(OR³³)₂ group is attached at a meta- or para-position of ring A. More typically, the -CHO, -COR³¹, -COOH, -COOR³¹, -CONH₂, -CONHR³¹, -CON(R³¹)₂, -CH₂OR³², -CH(R³¹)OR³², -C(R³¹)₂OR³², -CH(OR³³)₂ or -CR³¹(OR³³)₂ group is attached at a meta-position of ring A. Typically in such an embodiment, m is 1. For example, in one aspect of such an embodiment, the compound may be a compound of formula (Ib). Alternatively, m may be 2, 3 or 4, wherein one R³ is a -CHO, -COR³¹, -COOH, -COOR³¹, -CONH₂, -CONHR³¹, -CON(R³¹)₂, -CH₂OR³², -CH(R³¹)OR³², -C(R³¹)₂OR³², -CH(OR³³)₂ or -CR³¹(OR³³)₂ group, and the other R³ are independently selected from halo groups.

In another embodiment, at least one R³ is selected from a -COR³⁴, -COOR³⁴, -CH₂OH, -CH(R³⁴)OH or -C(R³⁴)₂OH group, wherein each R³⁴ is independently selected from a C₁-C₄ alkyl or a C₃-C₄ cycloalkyl group, wherein the C₁-C₄ alkyl or C₃-C₄ cycloalkyl group may optionally be substituted with one or more fluoro and/or chloro groups, or wherein any two R³⁴ attached to the same carbon atom may, together with the carbon atom to which they are attached, form a C₃-C₄ cycloalkyl group, wherein the C₃-C₄ cycloalkyl group may optionally be substituted with one or more fluoro and/or chloro groups. Typically, the -COR³⁴, -COOR³⁴, -CH₂OH, -CH(R³⁴)OH or -C(R³⁴)₂OH group is attached at a meta- or para-position of ring A. More typically, the -COR³⁴, -COOR³⁴, -CH₂OH, -CH(R³⁴)OH or -C(R³⁴)₂OH group is attached at a meta-position of ring A. Typically in such an embodiment, m is 1. For example, in one aspect of such an embodiment, the compound may be a compound of formula (Ib). Alternatively, m may be 2, 3 or 4, wherein one R³ is a -COR³⁴, -COOR³⁴, -CH₂OH, -CH(R³⁴)OH or -C(R³⁴)₂OH group, and the other R³ are independently selected from halo groups.

In a further embodiment at least one R³ is a saturated hydrocarbyl group, wherein the saturated hydrocarbyl group may be straight-chained or branched, or be or include cyclic groups, wherein the saturated hydrocarbyl group may optionally be substituted with one or more groups independently selected from halo, -CN and -OH. Typically, the saturated hydrocarbyl group is attached at a meta- or para-position of ring A. More typically, the saturated hydrocarbyl group is attached at a meta-position of ring A. Typically in such an embodiment, m is 1. For example, in one aspect of such an embodiment, the compound may be a compound of formula (Ib). Alternatively, m may be 2, 3 or 4, wherein one R³ is a saturated hydrocarbyl group according to the present embodiment, and the other R³ are independently selected from halo groups.

In one embodiment, at least one R³ is a C₁-C₆ alkyl or a C₃-C₆ cycloalkyl group, wherein the C₁-C₆ alkyl or C₃-C₆ cycloalkyl group may optionally be substituted with one or more substituents independently selected from halo, -CN and -OH. More typically in such an embodiment, the at least one R³ is selected from a C₁-C₄ alkyl or a C₃-C₆ cycloalkyl group, wherein the C₁-C₄ alkyl or C₃-C₆ cycloalkyl group is substituted with one or two -OH groups. For example, R³ may be a -C(Me)₂OH group. Typically, the C₁-C₆ alkyl or C₃-C₆ cycloalkyl group is attached at a meta- or para-position of ring A. More typically, the C₁-C₆ alkyl or C₃-C₆ cycloalkyl group is attached at a meta-position of ring A. Typically in such an embodiment, m is 1. For example, in one aspect of such an embodiment, the compound may be a compound of formula (Ib). Alternatively, m may be 2, 3 or 4, wherein one R³ is a C₁-C₆ alkyl or C₃-C₆ cycloalkyl group according to the present embodiment, and the other R³ are independently selected from halo groups.

In one aspect of any of the above embodiments, the group: 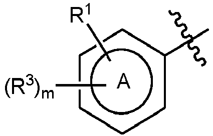 contains from 9 to 30 atoms other than hydrogen or halogen. More typically, the group contains from 10 to 20 atoms other than hydrogen or halogen. More typically still, the group contains from 12 to 18 atoms other than hydrogen or halogen.

R² is a 6-membered cyclic group substituted at the 2- and 4-positions, wherein the 6-membered cyclic group may optionally be further substituted. Typically, R² is a 6-membered cyclic group substituted at the 2-, 4- and 6-positions, wherein the 6-membered cyclic group may optionally be further substituted. For the avoidance of doubt, it is noted that it is a ring atom of the 6-membered cyclic group of R² that is directly attached to the nitrogen atom of the urea or thiourea group, not any substituent.

As used herein, reference to the n-position, e.g. 2-, 4- or 6-position, of the 6-membered cyclic group of R² refers to the position of the atoms of the 6-membered cyclic group relative to the point of attachment of the 6-membered cyclic group to the remainder of the molecule. For example, where -R² is a 8-fluoro-1,2,3,5,6,7-hexahydro-s-indacen-4-yl moiety, the 1-6 positions as referred to in the definition of R² are numbered as follows:

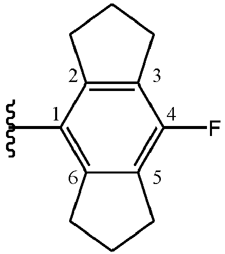

As will be noted, any fused rings are ignored when allocating the 1-6 positons to the 6-membered cyclic group; it is only the ring-atoms of the 6-membered cyclic group itself that are numbered. Moreover, as used herein, no preference is given to the nature or position of the substituents on the cyclic group or to the nature or position of any heteroatoms when allocating the 1-6 positons to the 6-membered cyclic group. For example, for the following group either position marked with a star (*) could be seen as either the 2-position or the 6-position. Substitution at either position marked with a star would fulfil the requirement that the 6-membered cyclic group is substituted at the 2-position:

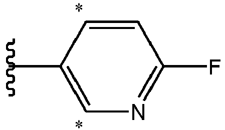

In one embodiment of the first aspect of the invention, R² is a phenyl or a 6-membered heteroaryl group substituted at the 2- and 4-positions, wherein the phenyl or the 6-membered heteroaryl group may optionally be further substituted. Typically, R² is a phenyl or a 6-membered heteroaryl group substituted at the 2-, 4- and 6-positions, wherein the phenyl or the 6-membered heteroaryl group may optionally be further substituted.

In an alternative embodiment, R² is a non-aromatic 6-membered cyclic group substituted at the 2- and 4-positions, wherein the non-aromatic 6-membered cyclic group may optionally be further substituted. Typically, R² is a non-aromatic 6-membered cyclic group substituted at the 2-, 4- and 6-positions positions, wherein the non-aromatic 6-membered cyclic group may optionally be further substituted. For example, R² may be a cyclohexyl, cyclohexenyl or non-aromatic 6-membered heterocyclic group substituted at least at the 2-, 4- and 6-positions.

In one aspect of any of the above embodiments, the substituent at the 4-position of the 6-membered cyclic group of R² is a group -R²⁰, wherein R²⁰ is a halo, -OH, -NO₂, -NH₂, -N₃, -SH, -SO₂H, -SO₂NH, or a saturated or unsaturated hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton.

Typically, R²⁰ contains from 1 to 8 atoms other than hydrogen. More typically, R²⁰ contains from 1 to 6 atoms other than hydrogen. Most typically, R²⁰ contains from 1 to 4 atoms other than hydrogen.

In one embodiment, R²⁰ is a halo, -NO₂, -CN, or a saturated hydrocarbyl group, wherein the saturated hydrocarbyl group may be straight-chained or branched, wherein the saturated hydrocarbyl group may optionally be substituted with one or more groups independently selected from halo, -CN, -OH, -NH₂ and oxo (=O), and wherein the saturated hydrocarbyl group may optionally include one or two heteroatoms N or O in its carbon skeleton.

In a further embodiment, R²⁰ is a halo, -NO₂, -CN, -CHO, -COR²¹, -COOH, -COOR²¹, -CONH₂, -CONHR²¹ or -CON(R²¹)₂ group, wherein each -R²¹ is independently selected from a C₁-C₆ alkyl, C₃-C₆ cycloalkyl, phenyl, benzyl, -R²² or -CH₂R²² group, wherein R²² is a 5- or 6-membered heteroaryl group, and wherein any -R²¹ may optionally be substituted with one or more halo groups, or wherein any two -R²¹ together with the nitrogen atom to which they are attached may form a 3- to 6-membered heterocyclic group, wherein the 3- to 6-membered heterocyclic group may optionally be substituted with one or more halo groups.

More typically, R²⁰ is a halo, -NO₂, -CN, -COOR²¹, -CONH₂, -CONHR²¹ or -CON(R²¹)₂ group, wherein each -R²¹ is independently selected from a C₁-C₄ alkyl group, and wherein any -R²¹ may optionally be substituted with one or more halo groups.

Most typically, R²⁰ is a fluoro, chloro, bromo, -CN, -CO₂Me or -CONH₂ group.

In any of the above embodiments, typical substituents at the 2- and/or 6- positions of the parent 6-membered cyclic group of R² comprise a carbon atom. For example, typical substituents at the 2- and/or 6- positions may be independently selected from -R⁴, -OR⁴ and -COR⁴ groups, wherein each R⁴ is independently selected from a C₁-C₆ alkyl, C₂-C₆ alkenyl, C₂-C₆ alkynyl or C₂-C₆ cyclic group and wherein each R⁴ is optionally further substituted with one or more halo groups. More typically, the substituents at the 2- and 6- positions are independently selected from alkyl and cycloalkyl groups, such as C₃-C₆ branched alkyl and C₃-C₆ cycloalkyl groups, e.g. isopropyl, cyclopropyl, cyclohexyl or t-butyl groups, wherein the alkyl and cycloalkyl groups are optionally further substituted with one or more fluoro and/or chloro groups.

In one aspect of any of the above embodiments, each substituent at the 2- and/or 6-positions comprises a carbon atom.

Other typical substituents at the 2- and/or 6- positions of the parent 6-membered cyclic group of R² may include cycloalkyl, cycloalkenyl, non-aromatic heterocyclic, aryl or heteroaryl rings which are fused to the parent cyclic group across the 2,3- and/or 5,6-positions respectively. Such fused cyclic groups are described in greater detail below.

In one embodiment, R² is a fused phenyl or a fused 6-membered heteroaryl group, wherein a cycloalkyl, cycloalkenyl, non-aromatic heterocyclic, aryl or heteroaryl ring is fused to the phenyl or the 6-membered heteroaryl group across the 2,3- positions, wherein the phenyl or the 6-membered heteroaryl group is further substituted at the 4-position, and wherein R² may optionally be further substituted. Typically in such an embodiment, the phenyl or the 6-membered heteroaryl group is also substituted at the 6-position. More typically, R² is a fused phenyl group, wherein a first cycloalkyl, cycloalkenyl, non-aromatic heterocyclic, aryl or heteroaryl ring is fused to the phenyl group across the 2,3-positions and a second cycloalkyl, cycloalkenyl, non-aromatic heterocyclic, aryl or heteroaryl ring is fused to the phenyl group across the 5,6-positions, wherein the phenyl group is further substituted at the 4-position, and wherein R² may optionally be further substituted. Typically in such an embodiment, R² is tricyclic.

In one embodiment, -R² has a formula selected from: 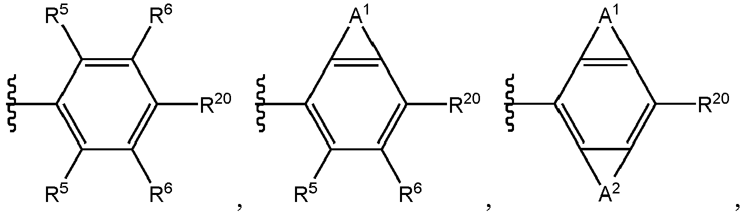 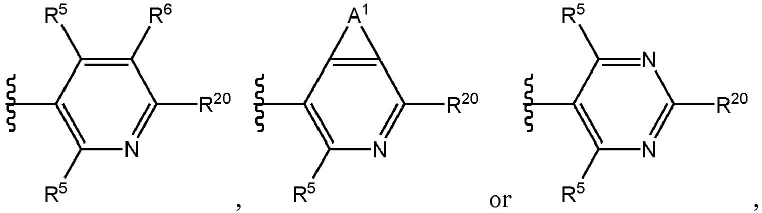 wherein:
A¹ and A² are each independently selected from an optionally substituted alkylene or alkenylene group, wherein one or more carbon atoms in the backbone of the alkylene or alkenylene group may optionally be replaced by one or more heteroatoms N, O or S;
each R⁵ is independently selected from a -R⁵¹, -OR⁵¹ or -COR⁵¹ group;
each R⁶ is independently selected from hydrogen or a halo, -R⁵¹, -OR⁵¹ or -COR⁵¹ group;
each R⁵¹ is independently selected from a C₁-C₆ alkyl, C₂-C₆ alkenyl, C₂-C₆ alkynyl or a 3- to 7-membered cyclic group, wherein each R⁵¹ is optionally further substituted; and
each R²⁰ is as defined herein.

Typically, any ring containing A¹ or A² is a 5- or 6-membered ring. Typically, A¹ and A² are each independently selected from an optionally substituted straight chain alkylene group, wherein one or two carbon atoms in the backbone of the alkylene group may optionally be replaced by one or two heteroatoms independently selected from nitrogen and oxygen. Typically, A¹ and A² are unsubstituted or substituted with one or more halo, -OH, -CN, -NO₂, -O(C₁-C₄ alkyl) or -O(C₁-C₄ haloalkyl) groups. Where a ring contains both A¹ and A² groups, A¹ and A² may be the same or different. Typically, A¹ and A² are the same.

Where R⁵¹ is a substituted C₁-C₆ alkyl, C₂-C₆ alkenyl or C₂-C₆ alkynyl group, typically the C₁-C₆ alkyl, C₂-C₆ alkenyl or C₂-C₆ alkynyl group is substituted with one or more halo, -OH, -CN, -NO₂, -O(C₁-C₄ alkyl) or -O(C₁-C₄ haloalkyl) groups. Where R⁵¹ is a substituted 3- to 7-membered cyclic group, typically the 3- to 7-membered cyclic group is substituted with one or more halo, -OH, -NH₂, -CN, -NO₂, -R⁵², -OR⁵², -NHR⁵² or -N(R⁵²)₂ groups, wherein R⁵² is a C₁-C₄ alkyl, C₁-C₄ alkenyl or C₁-C₄ alkynyl group which may optionally be halo-substituted.

Typically, each R⁵ is an -R⁵¹ group. More typically, each R⁵ is independently selected from a C₁-C₆ alkyl (in particular C₃-C₆ branched alkyl) or C₂-C₆ cycloalkyl group, wherein each R⁵ is optionally further substituted with one or more halo groups. Most typically, each R⁵ is independently selected from a C₁-C₄ alkyl group. Where a group R⁵ is present at both the 2- and 6-positions, each R⁵ may be the same or different. Typically, each R⁵ is the same.

Typically, each R⁶ is independently selected from hydrogen or a halo group. More typically, each R⁶ is hydrogen.

In one embodiment, -R² has a formula selected from: 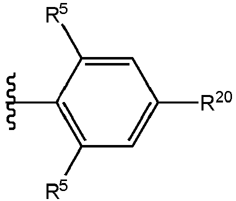 wherein each R⁵ is independently selected from a C₁-C₄ alkyl group, and R²⁰ is a halo, -NO₂, -CN, -COOR²¹, -CONH₂, -CONHR²¹ or -CON(R²¹)₂ group, wherein each -R²¹ is independently selected from a C₁-C₄ alkyl group, and wherein any -R²¹ may optionally be substituted with one or more halo groups.

Typically, -R² has a formula selected from:

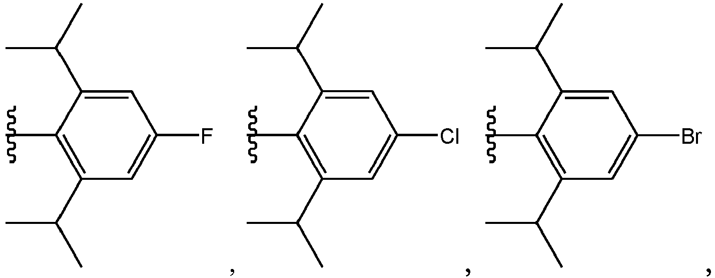

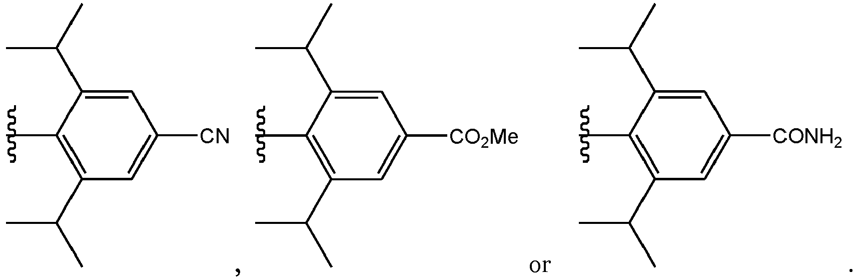

In a further embodiment, -R² has a formula selected from: 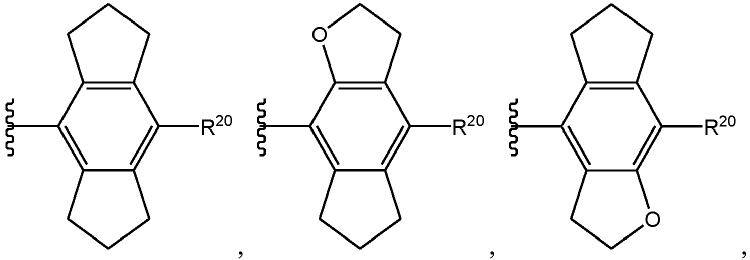 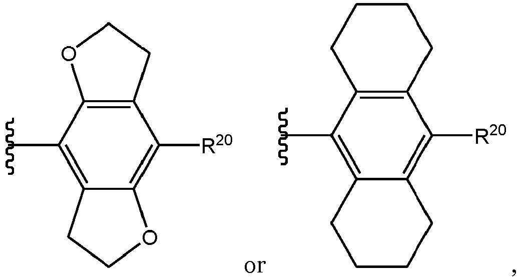
wherein R²⁰ is a halo, -NO₂, -CN, -COOR²¹, -CONH₂, -CONHR²¹ or -CON(R²¹)₂ group,
wherein each -R²¹ is independently selected from a C₁-C₄ alkyl group, and wherein any -R²¹ may optionally be substituted with one or more halo groups.

Typically, -R² has a formula selected from:

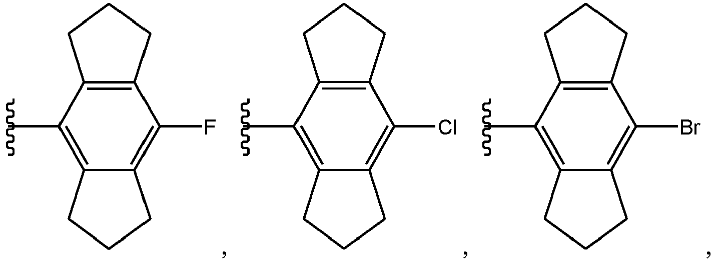

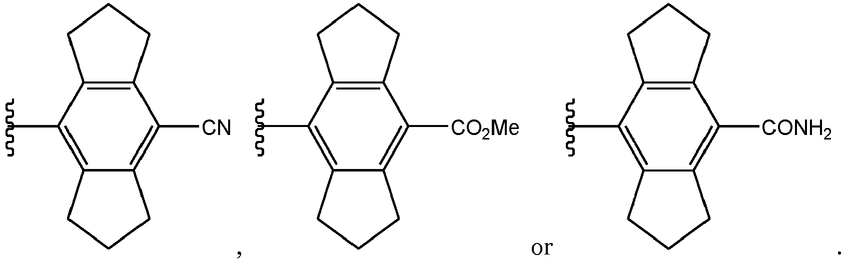

Yet other typical substituents at the 2- and/or 6- positions of the parent 6-membered cyclic group of R² may include monovalent heterocyclic groups and monovalent aromatic groups, wherein a ring atom of the heterocyclic or aromatic group is directly attached via a single bond to the ring atom at the 2- or the 6-position of the parent 6-membered cyclic group, wherein the heterocyclic or aromatic group may optionally be substituted. Such R² groups are described in greater detail below.

In one embodiment, R² is a parent 6-membered cyclic group substituted at the 2-position with a monovalent heterocyclic group or a monovalent aromatic group, wherein the heterocyclic or aromatic group may optionally be substituted, wherein the parent 6-membered cyclic group is further substituted at the 4-position and wherein the parent 6-membered cyclic group may optionally be further substituted. Typically, R² is a parent phenyl or 6-membered heteroaryl group substituted at the 2-position with a monovalent heterocyclic group or a monovalent aromatic group, wherein the heterocyclic or aromatic group may optionally be substituted, wherein the parent phenyl or 6-membered heteroaryl group is further substituted at the 4-position and wherein the parent phenyl or 6-membered heteroaryl group may optionally be further substituted. In such an embodiment, typical substituents at the 4-position include R²⁰ as defined herein.

In one embodiment, the monovalent heterocyclic or aromatic group at the 2-position is phenyl or a 5- or 6-membered heterocyclic group, all of which may optionally be substituted. In one embodiment, the monovalent heterocyclic or aromatic group at the 2-position is a phenyl, pyridinyl, pyridazinyl, pyrimidinyl, pyrazinyl, pyrrolyl, furanyl, thiophenyl, pyrazolyl, imidazolyl, oxazolyl, isoxazolyl, thiazolyl, isothiazolyl, triazolyl, oxadiazolyl, azetinyl, azetidinyl, oxetanyl, thietanyl, pyrrolidinyl, tetrahydrofuranyl, tetrahydrothiophenyl, pyrazolidinyl, imidazolidinyl, 1,3-dioxolanyl, 1,2-oxathiolanyl, 1,3-oxathiolanyl, piperidinyl, tetrahydropyranyl, thianyl, piperazinyl, 1,4-dioxanyl, morpholinyl or thiomorpholinyl group, all of which may optionally be substituted. In one embodiment, the monovalent heterocyclic or aromatic group at the 2-position is a phenyl, pyridinyl, pyridazinyl, pyrimidinyl, pyrazinyl, pyrrolyl, furanyl, thiophenyl, pyrazolyl, imidazolyl, oxazolyl, isoxazolyl, thiazolyl, isothiazolyl, piperidinyl or tetrahydropyranyl group, all of which may optionally be substituted. In one embodiment, the monovalent heterocyclic or aromatic group at the 2-position is a phenyl, pyridinyl, pyrimidinyl, pyrazolyl, imidazolyl, isoxazolyl, thiazolyl or tetrahydropyranyl group, all of which may optionally be substituted. In one embodiment, the monovalent heterocyclic or aromatic group at the 2-position is a phenyl, pyridinyl, pyrimidinyl or pyrazolyl group, all of which may optionally be substituted with one or two substituents independently selected from halo, -OH, -NH₂, -CN, -NO₂, -R⁸¹, -OR⁸¹, -NHR⁸¹ or -N(R⁸¹)₂, wherein R⁸¹ is a C₁-C₄ alkyl, C₁-C₄ alkenyl or C₁-C₄ alkynyl group which may optionally be halo-substituted. In one embodiment, the monovalent heterocyclic or aromatic group at the 2-position is an unsubstituted phenyl, pyridinyl, pyrimidinyl or pyrazolyl group. In one embodiment, the monovalent heterocyclic group at the 2-position is a pyridin-2-yl, pyridin-3-yl or pyridin-4-yl group, all of which may optionally be substituted with one or two substituents independently selected from halo, -OH, -NH₂, -CN, -NO₂, -R⁸¹, -OR⁸¹, -NHR⁸¹ or -N(R⁸¹)₂, wherein R⁸¹ is as defined herein. In one embodiment, the monovalent heterocyclic group at the 2-position is an unsubstituted pyridin-3-yl group or a pyridin-4-yl group optionally substituted with one or two substituents independently selected from halo, -OH, -NH₂, -CN, -NO₂, -R⁸¹, -OR⁸¹, -NHR⁸¹ or -N(R⁸¹)₂, wherein R⁸¹ is as defined herein.

In one embodiment, R² is a parent 6-membered cyclic group substituted at the 2-position with a monovalent heterocyclic group or a monovalent aromatic group, wherein the heterocyclic or aromatic group may optionally be substituted, wherein the parent 6-membered cyclic group is further substituted at the 4- and 6-positions and wherein the parent 6-membered cyclic group may optionally be further substituted.

Typically, R² is a parent phenyl or 6-membered heteroaryl group substituted at the 2-position with a monovalent heterocyclic group or a monovalent aromatic group, wherein the heterocyclic or aromatic group may optionally be substituted, wherein the parent phenyl or 6-membered heteroaryl group is further substituted at the 4- and 6-positions and wherein the parent phenyl or 6-membered heteroaryl group may optionally be further substituted. In such an embodiment, typical substituents at the 4-position include R²⁰ as defined herein. In such an embodiment, typical substituents at the 6-position may be independently selected from halo, -R⁷¹, -OR⁷¹ or -COR⁷¹ groups, wherein each R⁷¹ is independently selected from a C₁-C₆ alkyl, C₂-C₆ alkenyl, C₂-C₆ alkynyl or C₂-C₆ cyclic group and wherein each R⁷¹ is optionally further substituted with one or more halo groups. Typically, such further substituents at the 6-position of the parent cyclic group of R² are independently selected from halo, C₁-C₆ alkyl (in particular C₃-C₆ branched alkyl) or C₃-C₆ cycloalkyl groups, e.g. fluoro, chloro, isopropyl, cyclopropyl, cyclohexyl or t-butyl groups, wherein the alkyl and cycloalkyl groups are optionally further substituted with one or more fluoro and/or chloro groups.

In one embodiment, -R² has a formula selected from: 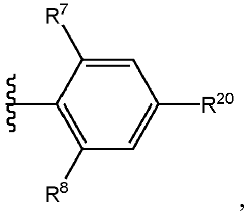 wherein R⁷ is a C₁-C₄ alkyl group, R⁸ is a 5- or 6-membered, optionally substituted heterocyclic or aromatic group, and R²⁰ is a halo, -NO₂, -CN, -COOR²¹, -CONH₂, -CONHR²¹ or -CON(R²¹)₂ group, wherein each -R²¹ is independently selected from a C₁-C₄ alkyl group, and wherein any -R²¹ may optionally be substituted with one or more halo groups. In one embodiment, the optional substituents on the heterocyclic or aromatic group are independently selected from halo, -OH, -NH₂, -CN, -NO₂, -R⁸², -OR⁸², -NHR⁸² or -N(R⁸²)₂, wherein R⁸² is a C₁-C₄ alkyl, C₁-C₄ alkenyl or C₁-C₄ alkynyl group which may optionally be halo-substituted.

Typically, -R² has a formula selected from: 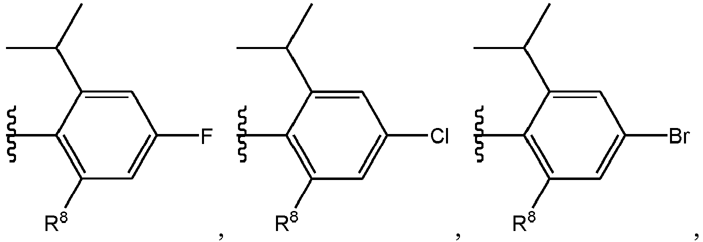 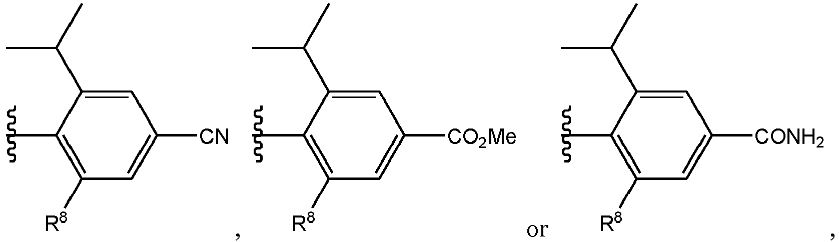 wherein R⁸ is a 5- or 6-membered, optionally substituted heterocyclic or aromatic group. In one embodiment, the optional substituents on the heterocyclic or aromatic group are independently selected from halo, -OH, -NH₂, -CN, -NO₂, -R⁸², -OR⁸², -NHR⁸² or -N(R⁸²)₂, wherein R⁸² is a C₁-C₄ alkyl, C₁-C₄ alkenyl or C₁-C₄ alkynyl group which may optionally be halo-substituted.

In one embodiment, R² is a parent 6-membered cyclic group substituted at the 2-position with a monovalent heterocyclic group or a monovalent aromatic group, wherein the heterocyclic or aromatic group may optionally be substituted, wherein a cycloalkyl, cycloalkenyl, non-aromatic heterocyclic, aryl or heteroaryl ring is fused to the parent 6-membered cyclic group across the 5,6-positions, wherein the parent 6-membered cyclic group is further substituted at the 4-position, and wherein the parent 6-membered cyclic group may optionally be further substituted. Typically, R² is a parent phenyl or 6-membered heteroaryl group substituted at the 2-position with a monovalent heterocyclic group or a monovalent aromatic group, wherein the heterocyclic or aromatic group may optionally be substituted, wherein a cycloalkyl, cycloalkenyl, non-aromatic heterocyclic, aryl or heteroaryl ring is fused to the parent phenyl or 6-membered heteroaryl group across the 5,6-positions, wherein the parent phenyl or 6-membered heteroaryl group is further substituted at the 4-position, and wherein the parent phenyl or 6-membered heteroaryl group may optionally be further substituted. In such an embodiment, typical substituents at the 4-position include R²⁰ as defined herein.

In one embodiment, -R² has a formula selected from: 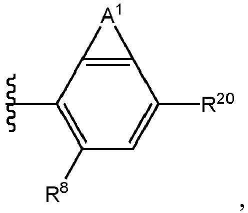 wherein A¹ is a straight chain alkylene group, wherein one or two carbon atoms in the backbone of the alkylene group may optionally be replaced by one or two heteroatoms independently selected from nitrogen and oxygen, wherein the alkylene group may optionally be substituted with one or more halo, -OH, -CN, -O(C₁-C₄ alkyl) or -O(C₁-C₄ haloalkyl) groups, and wherein the ring containing A¹ is a 5- or 6-membered ring, R⁸ is a 5- or 6-membered, optionally substituted heterocyclic or aromatic group, and R²⁰ is a halo, -NO₂, -CN, -COOR²¹, -CONH₂, -CONHR²¹ or -CON(R²¹)₂ group, wherein each -R²¹ is independently selected from a C₁-C₄ alkyl group, and wherein any -R²¹ may optionally be substituted with one or more halo groups. Typically, the optional substituents on the heterocyclic or aromatic group are selected from halo, -OH, -NH₂, -CN, -NO₂, -R⁸², -OR⁸², -NHR⁸² or -N(R⁸²)₂, wherein R⁸² is a C₁-C₄ alkyl, C₁-C₄ alkenyl or C₁-C₄ alkynyl group which may optionally be halo-substituted.

In one embodiment, -R² has a formula selected from: 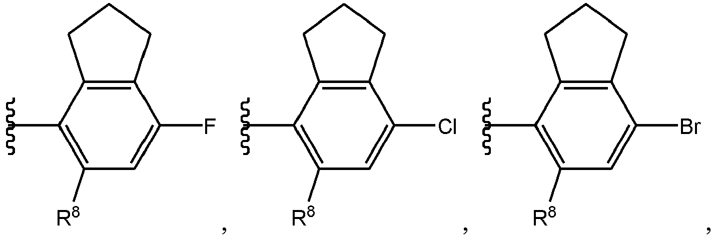 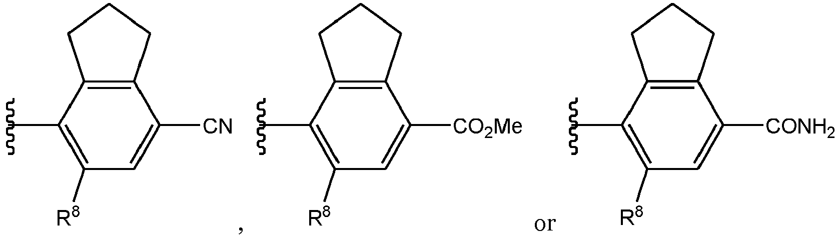 wherein R⁸ is a 5- or 6-membered, optionally substituted heterocyclic or aromatic group. In one embodiment, the optional substituents on the heterocyclic or aromatic group are independently selected from halo, -OH, -NH₂, -CN, -NO₂, -R⁸², -OR⁸², -NHR⁸² or -N(R⁸²)₂, wherein R⁸² is a C₁-C₄ alkyl, C₁-C₄ alkenyl or C₁-C₄ alkynyl group which may optionally be halo-substituted.

In one aspect of any of the above embodiments, R² contains from 6 to 30 atoms other than hydrogen or halogen. More typically, R² contains from 8 to 25 atoms other than hydrogen or halogen. More typically, R² contains from 9 to 20 atoms other than hydrogen or halogen.

Q is selected from O or S. In one embodiment of the first aspect of the invention, Q is O.

In one aspect of any of the above embodiments, the compound of formula (I) has a molecular weight of from 350 to 2000 Da. Typically, the compound of formula (I) has a molecular weight of from 400 to 900 Da. More typically, the compound of formula (I) has a molecular weight of from 500 to 600 Da.

A second aspect of the invention provides a compound selected from the group consisting of: 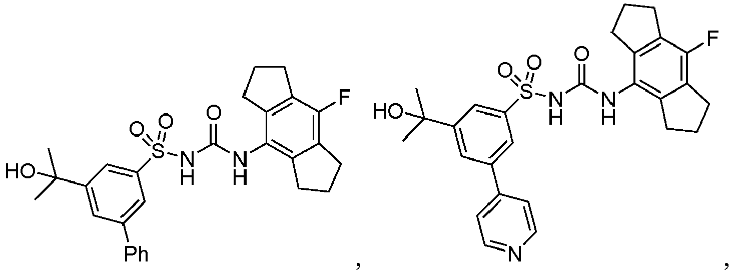 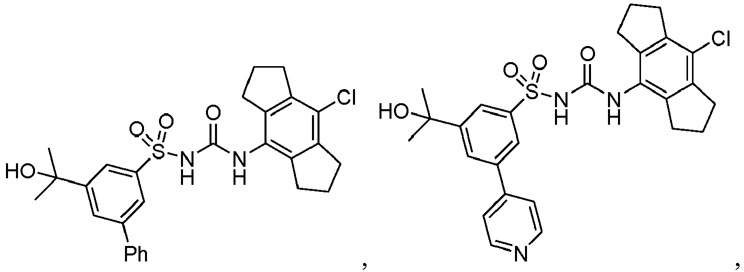 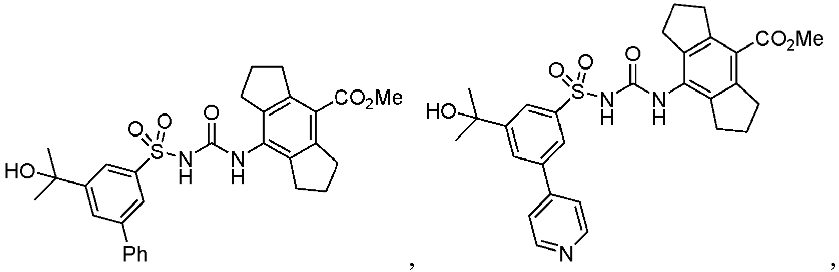 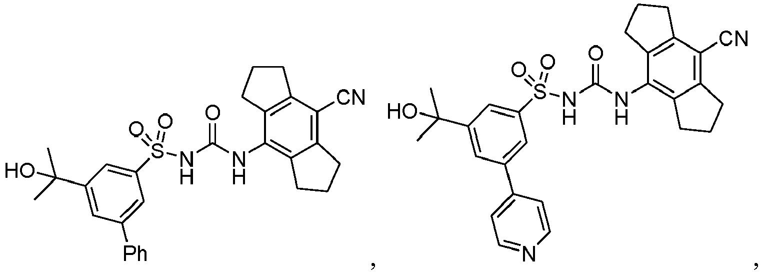 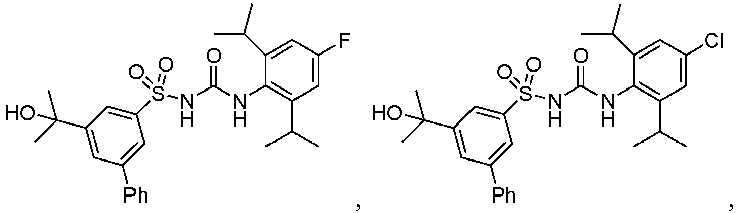 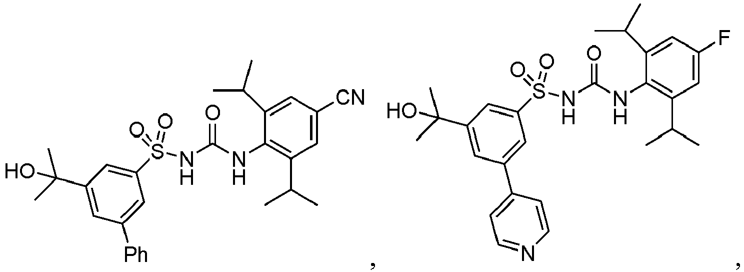 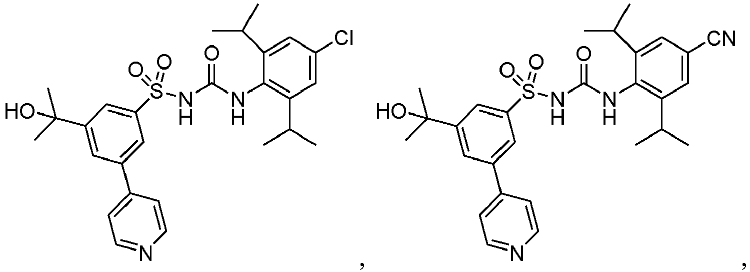 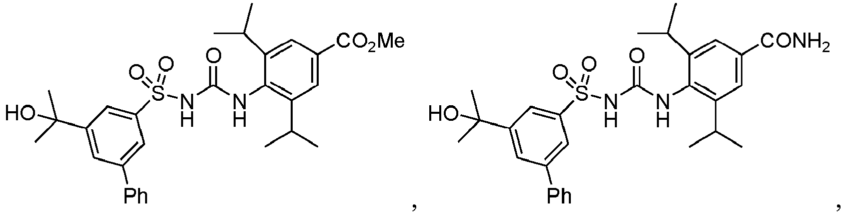 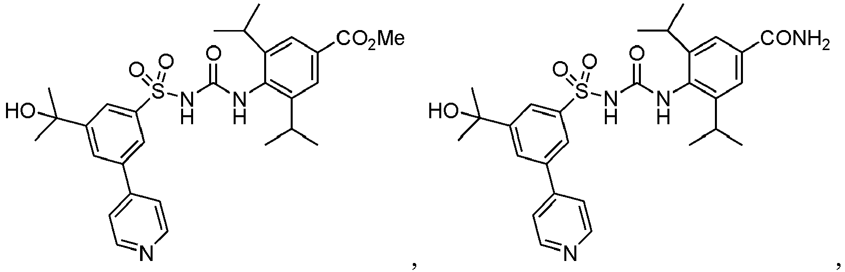 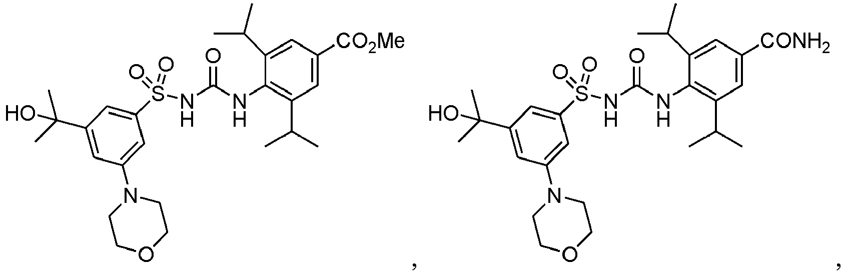 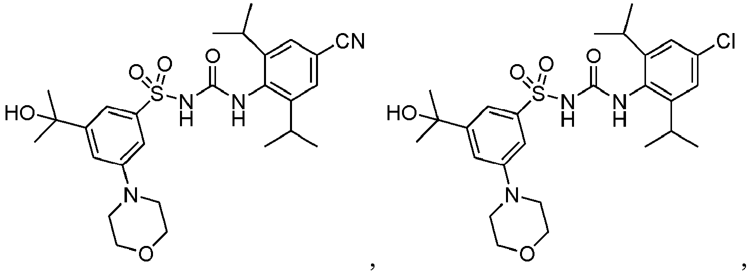 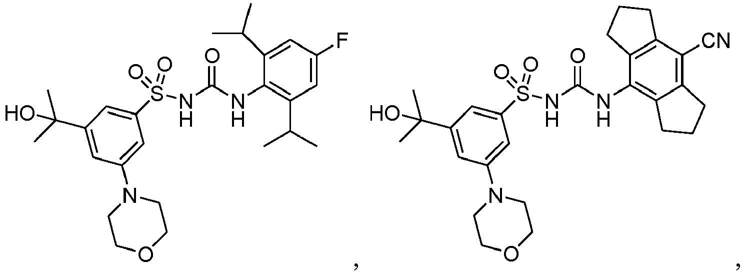 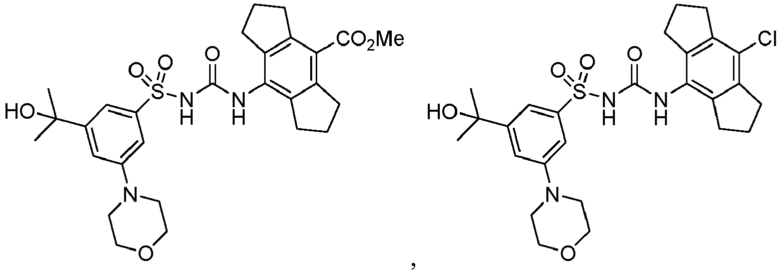 and

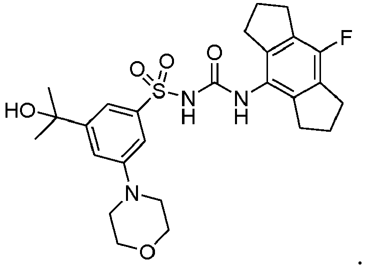

A third aspect of the invention provides a pharmaceutically acceptable salt, solvate or prodrug of any compound of the first or second aspect of the invention.

The compounds of the present invention can be used both in their free base form and their acid addition salt form. For the purposes of this invention, a "salt" of a compound of the present invention includes an acid addition salt. Acid addition salts are preferably pharmaceutically acceptable, non-toxic addition salts with suitable acids, including but not limited to inorganic acids such as hydrohalogenic acids (for example, hydrofluoric, hydrochloric, hydrobromic or hydroiodic acid) or other inorganic acids (for example, nitric, perchloric, sulfuric or phosphoric acid); or organic acids such as organic carboxylic acids (for example, propionic, butyric, glycolic, lactic, mandelic, citric, acetic, benzoic, salicylic, succinic, malic or hydroxysuccinic, tartaric, fumaric, maleic, hydroxymaleic, mucic or galactaric, gluconic, pantothenic or pamoic acid), organic sulfonic acids (for example, methanesulfonic, trifluoromethanesulfonic, ethanesulfonic, 2-hydroxyethanesulfonic, benzenesulfonic, toluene-p-sulfonic, naphthalene-2-sulfonic or camphorsulfonic acid) or amino acids (for example, ornithinic, glutamic or aspartic acid). The acid addition salt may be a mono-, di-, tri- or multi-acid addition salt. A preferred salt is a hydrohalogenic, sulfuric, phosphoric or organic acid addition salt. A preferred salt is a hydrochloric acid addition salt.

The compounds of the present invention can also be used both, in their free acid form and their salt form. For the purposes of this invention, a "salt" of a compound of the present invention includes one formed between a protic acid functionality (such as a carboxylic acid group) of a compound of the present invention and a suitable cation. Suitable cations include, but are not limited to lithium, sodium, potassium, magnesium, calcium and ammonium. The salt may be a mono-, di-, tri- or multi-salt. Preferably the salt is a mono- or di-lithium, sodium, potassium, magnesium, calcium or ammonium salt. More preferably the salt is a mono- or di-sodium salt or a mono- or dipotassium salt.

Preferably any salt is a pharmaceutically acceptable non-toxic salt. However, in addition to pharmaceutically acceptable salts, other salts are included in the present invention, since they have potential to serve as intermediates in the purification or preparation of other, for example, pharmaceutically acceptable salts, or are useful for identification, characterisation or purification of the free acid or base.

The compounds and/or salts of the present invention may be anhydrous or in the form of a hydrate (e.g. a hemihydrate, monohydrate, dihydrate or trihydrate) or other solvate. Such solvates may be formed with common organic solvents, including but not limited to, alcoholic solvents e.g. methanol, ethanol or isopropanol.

In some embodiments of the present invention, therapeutically inactive prodrugs are provided. Prodrugs are compounds which, when administered to a subject such as a human, are converted in whole or in part to a compound of the invention. In most embodiments, the prodrugs are pharmacologically inert chemical derivatives that can be converted *in vivo* to the active drug molecules to exert a therapeutic effect. Any of the compounds described herein can be administered as a prodrug to increase the activity, bioavailability, or stability of the compound or to otherwise alter the properties of the compound. Typical examples of prodrugs include compounds that have biologically labile protecting groups on a functional moiety of the active compound. Prodrugs include, but are not limited to, compounds that can be oxidized, reduced, aminated, deaminated, hydroxylated, dehydroxylated, hydrolyzed, dehydrolyzed, alkylated, dealkylated, acylated, deacylated, phosphorylated, and/or dephosphorylated to produce the active compound. The present invention also encompasses salts and solvates of such prodrugs as described above.

The compounds, salts, solvates and prodrugs of the present invention may contain at least one chiral centre. The compounds, salts, solvates and prodrugs may therefore exist in at least two isomeric forms. The present invention encompasses racemic mixtures of the compounds, salts, solvates and prodrugs of the present invention as well as enantiomerically enriched and substantially enantiomerically pure isomers. For the purposes of this invention, a "substantially enantiomerically pure" isomer of a compound comprises less than 5% of other isomers of the same compound, more typically less than 2%, and most typically less than 0.5% by weight.

The compounds, salts, solvates and prodrugs of the present invention may contain any stable isotope including, but not limited to ¹²C, ¹³C, ¹H, ²H (D), ¹⁴N, ¹⁵N, ¹⁶O, ¹⁷O, ¹⁸O, ¹⁹F and ¹²⁷I, and any radioisotope including, but not limited to ¹¹C, ¹⁴C, ³H (T), ¹³N, ¹⁵O, ¹⁸F, ¹²³I, ¹²⁴I, ¹²⁵I and ¹³¹I.

The compounds, salts, solvates and prodrugs of the present invention may be in any polymorphic or amorphous form.

A fourth aspect of the invention provides a pharmaceutical composition comprising a compound of the first or second aspect of the invention, or a pharmaceutically acceptable salt, solvate or prodrug of the third aspect of the invention, and a pharmaceutically acceptable excipient.

Conventional procedures for the selection and preparation of suitable pharmaceutical formulations are described in, for example, "Aulton's Pharmaceutics - The Design and Manufacture of Medicines", M. E. Aulton and K. M. G. Taylor, Churchill Livingstone Elsevier, 4th Ed., 2013.

Pharmaceutically acceptable excipients including adjuvants, diluents or carriers that may be used in the pharmaceutical compositions of the invention are those conventionally employed in the field of pharmaceutical formulation, and include, but are not limited to, sugars, sugar alcohols, starches, ion exchangers, alumina, aluminium stearate, lecithin, serum proteins such as human serum albumin, buffer substances such as phosphates, glycerine, sorbic acid, potassium sorbate, partial glyceride mixtures of saturated vegetable fatty acids, water, salts or electrolytes such as protamine sulfate, disodium hydrogen phosphate, potassium hydrogen phosphate, sodium chloride, zinc salts, colloidal silica, magnesium trisilicate, polyvinylpyrrolidone, cellulose-based substances, polyethylene glycol, sodium carboxymethylcellulose, polyacrylates, waxes, polyethylene-polyoxypropylene-block polymers, polyethylene glycol and wool fat.

A fifth aspect of the invention provides a compound of the first or second aspect of the invention, or a pharmaceutically acceptable salt, solvate or prodrug of the third aspect of the invention, or a pharmaceutical composition of the fourth aspect of the invention, for use in medicine, and/or for use in the treatment or prevention of a disease, disorder or condition. Typically, the use comprises the administration of the compound, salt, solvate, prodrug or pharmaceutical composition to a subject.

The term "treatment" as used herein refers equally to curative therapy, and ameliorating or palliative therapy. The term includes obtaining beneficial or desired physiological results, which may or may not be established clinically. Beneficial or desired clinical results include, but are not limited to, the alleviation of symptoms, the prevention of symptoms, the diminishment of extent of disease, the stabilisation (i.e., not worsening) of a condition, the delay or slowing of progression/worsening of a condition/symptoms, the amelioration or palliation of the condition/symptoms, and remission (whether partial or total), whether detectable or undetectable. The term "palliation", and variations thereof, as used herein, means that the extent and/or undesirable manifestations of a physiological condition or symptom are lessened and/or time course of the progression is slowed or lengthened, as compared to not administering a compound, salt, solvate, prodrug or pharmaceutical composition of the present invention. The term "prevention" as used herein in relation to a disease, disorder or condition, relates to prophylactic or preventative therapy, as well as therapy to reduce the risk of developing the disease, disorder or condition. The term "prevention" includes both the avoidance of occurrence of the disease, disorder or condition, and the delay in onset of the disease, disorder or condition. Any statistically significant (p ≤ 0.05) avoidance of occurrence, delay in onset or reduction in risk as measured by a controlled clinical trial may be deemed a prevention of the disease, disorder or condition. Subjects amenable to prevention include those at heightened risk of a disease, disorder or condition as identified by genetic or biochemical markers. Typically, the genetic or biochemical markers are appropriate to the disease, disorder or condition under consideration and may include for example, inflammatory biomarkers such as C-reactive protein (CRP) and monocyte chemoattractant protein 1 (MCP-1) in the case of inflammation; total cholesterol, triglycerides, insulin resistance and C-peptide in the case of NAFLD and NASH; and more generally IL1β and IL18 in the case of a disease, disorder or condition responsive to NLRP3 inhibition.

A sixth aspect of the invention provides the use of a compound of the first or second aspect, or a pharmaceutically effective salt, solvate or prodrug of the third aspect, in the manufacture of a medicament for the treatment or prevention of a disease, disorder or condition. Typically, the treatment or prevention comprises the administration of the compound, salt, solvate, prodrug or pharmaceutical composition to a subject.

A seventh aspect of the invention provides a method of treatment or prevention of a disease, disorder or condition, the method comprising the step of administering an effective amount of a compound of the first or second aspect, or a pharmaceutically acceptable salt, solvate or prodrug of the third aspect, or a pharmaceutical composition of the fourth aspect, to thereby treat or prevent the disease, disorder or condition. Typically, the administration is to a subject in need thereof.

An eighth aspect of the invention provides a compound of the first or second aspect of the invention, or a pharmaceutically acceptable salt, solvate or prodrug of the third aspect of the invention, or a pharmaceutical composition of the fourth aspect of the invention, for use in the treatment or prevention of a disease, disorder or condition in an individual, wherein the individual has a germline or somatic non-silent mutation in NLRP3. The mutation may be, for example, a gain-of-function or other mutation resulting in increased NLRP3 activity. Typically, the use comprises the administration of the compound, salt, solvate, prodrug or pharmaceutical composition to the individual. The use may also comprise the diagnosis of an individual having a germline or somatic non-silent mutation in NLRP3, wherein the compound, salt, solvate, prodrug or pharmaceutical composition is administered to an individual on the basis of a positive diagnosis for the mutation. Typically, identification of the mutation in NLRP3 in the individual may be by any suitable genetic or biochemical means.

A ninth aspect of the invention provides a method of treatment or prevention of a disease, disorder or condition, the method comprising the steps of diagnosing of an individual having a germline or somatic non-silent mutation in NLRP3, and administering an effective amount of a compound of the first or second aspect, or a pharmaceutically acceptable salt, solvate or prodrug of the third aspect, or a pharmaceutical composition of the fourth aspect, to the positively diagnosed individual, to thereby treat or prevent the disease, disorder or condition. Typically, the administration is to a subject in need thereof.

In general embodiments, the disease, disorder or condition may be a disease, disorder or condition of the immune system, the cardiovascular system, the endocrine system, the gastrointestinal tract, the renal system, the hepatic system, the metabolic system, the respiratory system, the central nervous system, may be a cancer or other malignancy, and/or may be caused by or associated with a pathogen.

It will be appreciated that these general embodiments defined according to broad categories of diseases, disorders and conditions are not mutually exclusive. In this regard any particular disease, disorder or condition may be categorized according to more than one of the above general embodiments. A non-limiting example is type I diabetes which is an autoimmune disease and a disease of the endocrine system.

In one embodiment of the fifth, sixth, seventh, eighth or ninth aspect of the invention, the disease, disorder or condition is responsive to NLRP3 inhibition. As used herein, the term "NLRP3 inhibition" refers to the complete or partial reduction in the level of activity of NLRP3 and includes, for example, the inhibition of active NLRP3 and/or the inhibition of activation of NLRP3.

There is evidence for a role of NLRP3-induced IL-1 and IL-18 in the inflammatory responses occurring in connection with, or as a result of, a multitude of different disorders (Menu et al., Clinical and Experimental Immunology, 166: 1-15, 2011; Strowig et al., Nature, 481:278-286, 2012).

NLRP3 has been implicated in a number of autoinflammatory diseases, including Familial Mediterranean fever (FMF), TNF receptor associated periodic syndrome (TRAPS), hyperimmunoglobulinemia D and periodic fever syndrome (HIDS), pyogenic arthritis, pyoderma gangrenosum and acne (PAPA), Sweet's syndrome, chronic nonbacterial osteomyelitis (CNO), and acne vulgaris (Cook et al., Eur. J. Immunol., 40: 595-653, 2010). In particular, NLRP3 mutations have been found to be responsible for a set of rare autoinflammatory diseases known as CAPS (Ozaki et al., J. Inflammation Research, 8:15-27, 2015; Schroder et al., Cell, 140: 821-832, 2010; and Menu et al., Clinical and Experimental Immunology, 166: 1-15, 2011). CAPS are heritable diseases characterized by recurrent fever and inflammation and are comprised of three autoinflammatory disorders that form a clinical continuum. These diseases, in order of increasing severity, are familial cold autoinflammatory syndrome (FCAS), Muckle-Wells syndrome (MWS), and chronic infantile cutaneous neurological articular syndrome (CINCA; also called neonatal-onset multisystem inflammatory disease, NOMID), and all have been shown to result from gain-of-function mutations in the NLRP3 gene, which leads to increased secretion of IL-1β.

A number of autoimmune diseases have been shown to involve NLRP3 including, in particular, multiple sclerosis, type-1 diabetes (T1D), psoriasis, rheumatoid arthritis (RA), Behcet's disease, Schnitzler syndrome, macrophage activation syndrome (Masters Clin. Immunol. 2013; Braddock et al., Nat. Rev. Drug Disc., 3: 1-10, 2004; and Inoue et al., Immunology, 139, 11-18), and systemic sclerosis (Artlett et al., Arthritis Rheum., 2011; 63(11): 3563-74). NLRP3 has also been shown to play a role in a number of lung diseases including chronic obstructive pulmonary disorder (COPD), asthma, asbestosis, and silicosis (De Nardo et al., Am. J. Pathol., 184: 42-54, 2014). NLRP3 has also been suggested to have a role in a number of central nervous system conditions, including Parkinson's disease (PD), Alzheimer's disease (AD), dementia, Huntington's disease, cerebral malaria, brain injury from pneumococcal meningitis (Walsh et al., Nature Reviews, 15: 84-97, 2014), intracranial aneurysms (Zhang et al., J. Stroke & Cerebrovascular Dis., 2015; 24; 5: 972-979), and traumatic brain injury (Ismael et al., J. Neurotrauma, 2018, Jan 2). NRLP3 activity has also been shown to be involved in various metabolic diseases including type 2 diabetes (T2D), atherosclerosis, obesity, gout, pseudo-gout, and metabolic syndrome (Wen et al., Nature Immunology, 13: 352-357, 2012; Duewell et al., Nature, 464: 1357-1361, 2010; Strowig et al., Nature, 481: 278-286, 2012). A role for NLRP3 via IL-1β has also been suggested in atherosclerosis and other cardiovascular events (Ridker et al., N Engl J Med., doi: 10.1056/NEJMoa1707914, 2017). Other diseases in which NLRP3 has been shown to be involved include: ocular diseases such as age-related macular degeneration (Doyle et al., Nature Medicine, 18: 791-798, 2012), diabetic retinopathy (Loukovaara et al., Acta Ophthalmol., 2017; 95(8): 803-808), and optic nerve damage (Puyang et al., Sci. Rep., 2016 Feb 19; 6: 20998); liver diseases including non-alcoholic steatohepatitis (NASH) (Henao-Meija et al., Nature, 482: 179-185, 2012); inflammatory reactions in the skin including contact hypersensitivity such as bullous pemphigoid (Fang et al., J. Dermatol. Sci., 2016; 83(2): 116-23), and UVB irradiation (Schroder et al., Science, 327: 296-300, 2010); inflammatory reactions in the joints (Braddock et al., Nat. Rev. Drug Disc., 3: 1-10, 2004), stroke (Walsh et al., Nature Reviews, 15: 84-97, 2014); and inflammatory bowel diseases including ulcerative colitis and Crohn's disease (Braddock et al., Nat. Rev. Drug Disc., 3: 1-10, 2004).

The inflammasome, and NLRP3 specifically, has also been proposed as a target for modulation by various pathogens including viruses such as DNA viruses (Amsler et al., Future Virol. (2013) 8(4), 357-370).

NLRP3 has also been implicated in the pathogenesis of many cancers (Menu et al., Clinical and Experimental Immunology 166: 1-15, 2011; and Masters Clin. Immunol. 2013). For example, several previous studies have suggested a role for IL-1β in cancer invasiveness, growth and metastasis, and inhibition of IL-1β with canakinumab has been shown to reduce the incidence of lung cancer and total cancer mortality in a randomised, double-blind, placebo-controlled trial (Ridker et al. Lancet, S0140-6736(17)32247-X, 2017). Inhibition of the NLRP3 inflammasome or IL-1β has also been shown to inhibit the proliferation and migration of lung cancer cells *in vitro* (Wang et al., Oncol Rep., 2016; 35(4): 2053-64). A role for the NLRP3 inflammasome has been suggested in myelodysplastic syndromes (Basiorka et al., Blood, 2016 Dec 22; 128(25): 2960-2975) and also in the carcinogenesis of various other cancers including glioma (Li et al., Am. J. Cancer Res., 2015; 5(1): 442-449) and squamous cell carcinoma of the head and neck (Huang et al., J. Exp. Clin. Cancer Res., 2017 2; 36(1): 116). Activation of the NLRP3 inflammasome has also been shown to mediate chemoresistance of tumour cells to 5-Fluorouracil (Feng et al., J. Exp. Clin. Cancer Res., 2017 21; 36(1): 81), and activation of NLRP3 inflammasome in peripheral nerve contributes to chemotherapy-induced neuropathic pain (Jia et al., Mol Pain., 2017; 13: 1-11).

NLRP3 has also been shown to be required for the efficient control of viral, bacterial, fungal, and helminth pathogen infections (Strowig et al., Nature, 481:278-286, 2012).

Accordingly, examples of diseases, disorders or conditions which may be responsive to NLRP3 inhibition and which may be treated or prevented in accordance with the fifth, sixth, seventh, eighth or ninth aspect of the present invention include:
(i) inflammation, including inflammation occurring as a result of an inflammatory disorder, e.g. an autoinflammatory disease, inflammation occurring as a symptom of a non-inflammatory disorder, inflammation occurring as a result of infection, or inflammation secondary to trauma, injury or autoimmunity;
(ii) auto-immune diseases such as acute disseminated encephalitis, Addison's disease, ankylosing spondylitis, antiphospholipid antibody syndrome (APS), anti-synthetase syndrome, aplastic anemia, autoimmune adrenalitis, autoimmune hepatitis, autoimmune oophoritis, autoimmune polyglandular failure, autoimmune thyroiditis, Coeliac disease, Crohn's disease, type 1 diabetes (T1D), Goodpasture's syndrome, Graves' disease, Guillain-Barre syndrome (GBS), Hashimoto's disease, idiopathic thrombocytopenic purpura, Kawasaki's disease, lupus erythematosus including systemic lupus erythematosus (SLE), multiple sclerosis (MS), myasthenia gravis, opsoclonus myoclonus syndrome (OMS), optic neuritis, Ord's thyroiditis, pemphigus, pernicious anaemia, polyarthritis, primary biliary cirrhosis, rheumatoid arthritis (RA), psoriatic arthritis, juvenile idiopathic arthritis, Reiter's syndrome, Sjögren's syndrome, systemic sclerosis, a systemic connective tissue disorder, Takayasu's arteritis, temporal arteritis, warm autoimmune hemolytic anemia, Wegener's granulomatosis, alopecia universalis, Behçet's disease, Chagas' disease, dysautonomia, endometriosis, hidradenitis suppurativa (HS), interstitial cystitis, neuromyotonia, psoriasis, sarcoidosis, scleroderma, ulcerative colitis, Schnitzler syndrome, macrophage activation syndrome, Blau syndrome, vitiligo or vulvodynia;
(iii) cancer including lung cancer, pancreatic cancer, gastric cancer, myelodisplastic syndrome, leukaemia including acute lymphocytic leukaemia (ALL) and acute myeloid leukaemia (AML), adrenal cancer, anal cancer, basal and squamous cell skin cancer, bile duct cancer, bladder cancer, bone cancer, brain and spinal cord tumours, breast cancer, cervical cancer, chronic lymphocytic leukaemia (CLL), chronic myeloid leukaemia (CML), chronic myelomonocytic leukaemia (CMML), colorectal cancer, endometrial cancer, oesophagus cancer, Ewing family of tumours, eye cancer, gallbladder cancer, gastrointestinal carcinoid tumours, gastrointestinal stromal tumour (GIST), gestational trophoblastic disease, glioma, Hodgkin lymphoma, Kaposi sarcoma, kidney cancer, laryngeal and hypopharyngeal cancer, liver cancer, lung carcinoid tumour, lymphoma, malignant mesothelioma, melanoma skin cancer, Merkel cell skin cancer, multiple myeloma, nasal cavity and paranasal sinuses cancer, nasopharyngeal cancer, neuroblastoma, non-Hodgkin lymphoma, non-small cell lung cancer, oral cavity and oropharyngeal cancer, osteosarcoma, ovarian cancer, penile cancer, pituitary tumours, prostate cancer, retinoblastoma, rhabdomyosarcoma, salivary gland cancer, skin cancer, small cell lung cancer, small intestine cancer, soft tissue sarcoma, stomach cancer, testicular cancer, thymus cancer, thyroid cancer, uterine sarcoma, vaginal cancer, vulvar cancer, Waldenstrom macroglobulinemia, and Wilms tumour;
(iv) infections including viral infections (e.g. from influenza virus, human immunodeficiency virus (HIV), alphavirus (such as Chikungunya and Ross River virus), flaviviruses (such as Dengue virus and Zika virus), herpes viruses (such as Epstein Barr Virus, cytomegalovirus, Varicella-zoster virus, and KSHV), poxviruses (such as vaccinia virus (Modified vaccinia virus Ankara) and Myxoma virus), adenoviruses (such as Adenovirus 5), or papillomavirus), bacterial infections (e.g. from *Staphylococcus aureus, Helicobacter pylori, Bacillus anthracis, Bordatella pertussis, Burkholderia pseudomallei, Corynebacterium diptheriae, Clostridium tetani, Clostridium botulinum, Streptococcus pneumoniae, Streptococcus pyogenes, Listeria monocytogenes, Hemophilus influenzae, Pasteurella multicida, Shigella dysenteriae, Mycobacterium tuberculosis, Mycobacterium leprae, Mycoplasma pneumoniae, Mycoplasma hominis, Neisseria meningitidis, Neisseria gonorrhoeae, Rickettsia rickettsii, Legionella pneumophila, Klebsiella pneumoniae, Pseudomonas aeruginosa, Propionibacterium acnes, Treponema pallidum, Chlamydia trachomatis, Vibrio cholerae, Salmonella typhimurium, Salmonella typhi, Borrelia burgdorferi* or *Yersinia pestis*), fungal infections (e.g. from Candida or Aspergillus species), protozoan infections (e.g. from Plasmodium, Babesia, Giardia, Entamoeba, Leishmania or Trypanosomes), helminth infections (e.g. from schistosoma, roundworms, tapeworms or flukes) and prion infections;
(v) central nervous system diseases such as Parkinson's disease, Alzheimer's disease, dementia, motor neuron disease, Huntington's disease, cerebral malaria, brain injury from pneumococcal meningitis, intracranial aneurysms, and traumatic brain injury;
(vi) metabolic diseases such as type 2 diabetes (T2D), atherosclerosis, obesity, gout, and pseudo-gout;
(vii) cardiovascular diseases such as hypertension, ischaemia, reperfusion injury, stroke including ischemic stroke, myocardial infarction including recurrent myocardial infarction, congestive heart failure, embolism, abdominal aortic aneurism, and pericarditis including Dressler's syndrome;
(viii) respiratory diseases including chronic obstructive pulmonary disorder (COPD), asthma such as allergic asthma and steroid-resistant asthma, asbestosis, silicosis, nanoparticle induced inflammation, cystic fibrosis and idiopathic pulmonary fibrosis;
(ix) liver diseases including non-alcoholic fatty liver disease (NAFLD), and non-alcoholic steatohepatitis (NASH); alcoholic fatty liver disease (AFLD), and alcoholic steatohepatitis (ASH);
(x) renal diseases including chronic kidney disease, oxalate nephropathy, nephrocalcinosis, glomerulonephritis, and diabetic nephropathy;
(xi) ocular diseases including those of the ocular epithelium, age-related macular degeneration (AMD), uveitis, corneal infection, diabetic retinopathy, optic nerve damage, and dry eye;
(xii) skin diseases including dermatitis such as contact dermatitis, contact hypersensitivity, sunburn, skin lesions, hidradenitis suppurativa (HS), and other cyst-causing skin diseases;
(xiii) lymphatic conditions such as lymphangitis and Castleman's disease;
(xiv) psychological disorders such as depression and psychological stress;
(xv) graft versus host disease; and
(xvi) any disease where an individual has been determined to carry a germline or somatic non-silent mutation in NLRP3.

In one embodiment, the disease, disorder or condition is selected from:
(i) cancer;
(ii) an infection;
(iii) a central nervous system disease;
(iv) a cardiovascular disease;
(v) a liver disease;
(vi) an ocular diseases; or
(vii) a skin disease.

More typically, the disease, disorder or condition is selected from:
(i) cancer;
(ii) an infection;
(iii) a central nervous system disease; or
(iv) a cardiovascular disease.

In a further typical embodiment of the invention, the disease, disorder or condition is inflammation. Examples of inflammation that may be treated or prevented in accordance with the fifth, sixth, seventh, eighth or ninth aspect of the present invention include inflammatory responses occurring in connection with, or as a result of:
(i) a skin condition such as contact hypersensitivity, bullous pemphigoid, sunburn, psoriasis, atopical dermatitis, contact dermatitis, allergic contact dermatitis, seborrhoetic dermatitis, lichen planus, scleroderma, pemphigus, epidermolysis bullosa, urticaria, erythemas, or alopecia;
(ii) a joint condition such as osteoarthritis, systemic juvenile idiopathic arthritis, adult-onset Still's disease, relapsing polychondritis, rheumatoid arthritis, juvenile chronic arthritis, gout, or a seronegative spondyloarthropathy (e.g. ankylosing spondylitis, psoriatic arthritis or Reiter's disease);
(iii) a muscular condition such as polymyositis or myasthenia gravis;
(iv) a gastrointestinal tract condition such as inflammatory bowel disease (including Crohn's disease and ulcerative colitis), gastric ulcer, coeliac disease, proctitis, pancreatitis, eosinopilic gastro-enteritis, mastocytosis, antiphospholipid syndrome, or a food-related allergy which may have effects remote from the gut (e.g., migraine, rhinitis or eczema);
(v) a respiratory system condition such as chronic obstructive pulmonary disease (COPD), asthma (including bronchial, allergic, intrinsic, extrinsic or dust asthma, and particularly chronic or inveterate asthma, such as late asthma and airways hyper-responsiveness), bronchitis, rhinitis (including acute rhinitis, allergic rhinitis, atrophic rhinitis, chronic rhinitis, rhinitis caseosa, hypertrophic rhinitis, rhinitis pumlenta, rhinitis sicca, rhinitis medicamentosa, membranous rhinitis, seasonal rhinitis e.g. hay fever, and vasomotor rhinitis), sinusitis, idiopathic pulmonary fibrosis (IPF), sarcoidosis, farmer's lung, silicosis, asbestosis, adult respiratory distress syndrome, hypersensitivity pneumonitis, or idiopathic interstitial pneumonia;
(vi) a vascular condition such as atherosclerosis, Behcet's disease, vasculitides, or wegener's granulomatosis;
(vii) an autoimmune condition such as systemic lupus erythematosus, Sjogren's syndrome, systemic sclerosis, Hashimoto's thyroiditis, type I diabetes, idiopathic thrombocytopenia purpura, or Graves disease;
(viii) an ocular condition such as uveitis, allergic conjunctivitis, or vernal conjunctivitis;
(ix) a nervous condition such as multiple sclerosis or encephalomyelitis;
(x) an infection or infection-related condition, such as Acquired Immunodeficiency Syndrome (AIDS), acute or chronic bacterial infection, acute or chronic parasitic infection, acute or chronic viral infection, acute or chronic fungal infection, meningitis, hepatitis (A, B or C, or other viral hepatitis), peritonitis, pneumonia, epiglottitis, malaria, dengue hemorrhagic fever, leishmaniasis, streptococcal myositis, mycobacterium tuberculosis, mycobacterium avium intracellulare, pneumocystis carinii pneumonia, orchitis/epidydimitis, legionella, Lyme disease, influenza A, epstein-barr virus, viral encephalitis/aseptic meningitis, or pelvic inflammatory disease;
(xi) a renal condition such as mesangial proliferative glomerulonephritis, nephrotic syndrome, nephritis, glomerular nephritis, acute renal failure, uremia, or nephritic syndrome;
(xii) a lymphatic condition such as Castleman's disease;
(xiii) a condition of, or involving, the immune system, such as hyper IgE syndrome, lepromatous leprosy, familial hemophagocytic lymphohistiocytosis, or graft versus host disease;
(xiv) a hepatic condition such as chronic active hepatitis, non-alcoholic steatohepatitis (NASH), alcohol-induced hepatitis, non-alcoholic fatty liver disease (NAFLD), alcoholic fatty liver disease (AFLD), alcoholic steatohepatitis (ASH) or primary biliary cirrhosis;
(xv) a cancer, including those cancers listed above;
(xvi) a burn, wound, trauma, haemorrhage or stroke;
(xvii) radiation exposure; and/or
(xviii) obesity; and/or
(xix) pain such as inflammatory hyperalgesia.

In one embodiment of the fifth, sixth, seventh, eighth or ninth aspect of the present invention, the disease, disorder or condition is an autoinflammatory disease such as cryopyrin-associated periodic syndromes (CAPS), Muckle-Wells syndrome (MWS), familial cold autoinflammatory syndrome (FCAS), familial Mediterranean fever (FMF), Neonatal onset multisystem inflammatory disease (NOMID), Tumour Necrosis Factor (TNF) Receptor-Associated Periodic Syndrome (TRAPS), hyperimmunoglobulinemia D and periodic fever syndrome (HIDS), deficiency of interleukin 1 receptor antagonist (DIRA), Majeed syndrome, pyogenic arthritis, pyoderma gangrenosum and acne syndrome (PAPA), adult-onset Still's disease (AOSD), haploinsufficiency of A20 (HA20), pediatric granulomatous arthritis (PGA), PLCG2-associated antibody deficiency and immune dysregulation (PLAID), PLCG2-associated autoinflammatory, antibody deficiency and immune dysregulation (APLAID), or sideroblastic anaemia with B-cell immunodeficiency, periodic fevers and developmental delay (SIFD).

Examples of diseases, disorders or conditions which may be responsive to NLRP3 inhibition and which may be treated or prevented in accordance with the fifth, sixth, seventh, eighth or ninth aspect of the present invention are listed above. Some of these diseases, disorders or conditions are substantially or entirely mediated by NLRP3 inflammasome activity, and NLRP3-induced IL-1β and/or IL-18. As a result, such diseases, disorders or conditions may be particularly responsive to NLRP3 inhibition and may be particularly suitable for treatment or prevention in accordance with the fifth, sixth, seventh, eighth or ninth aspect of the present invention. Examples of such diseases, disorders or conditions include cryopyrin-associated periodic syndromes (CAPS), Muckle-Wells syndrome (MWS), familial cold autoinflammatory syndrome (FCAS), Neonatal onset multisystem inflammatory disease (NOMID), familial Mediterranean fever (FMF), pyogenic arthritis, pyoderma gangrenosum and acne syndrome (PAPA), hyperimmunoglobulinemia D and periodic fever syndrome (HIDS), and Tumour Necrosis Factor (TNF) Receptor-Associated Periodic Syndrome (TRAPS), systemic juvenile idiopathic arthritis, adult-onset Still's disease (AOSD), relapsing polychondritis, Schnitzler's syndrome, Sweet's syndrome, Behcet's disease, anti-synthetase syndrome, deficiency of interleukin 1 receptor antagonist (DIRA), and haploinsufficiency of A20 (HA20).

Moreover, some of the diseases, disorders or conditions mentioned above arise due to mutations in NLRP3, in particular, resulting in increased NLRP3 activity. As a result, such diseases, disorders or conditions may be particularly responsive to NLRP3 inhibition and may be particularly suitable for treatment or prevention in accordance with the fifth, sixth, seventh, eighth or ninth aspect of the present invention. Examples of such diseases, disorders or conditions include cryopyrin-associated periodic syndromes (CAPS), Muckle-Wells syndrome (MWS), familial cold autoinflammatory syndrome (FCAS), and Neonatal onset multisystem inflammatory disease (NOMID).

A tenth aspect of the invention provides a method of inhibiting NLRP3, the method comprising the use of a compound of the first or second aspect of the invention, or a pharmaceutically acceptable salt, solvate or prodrug of the third aspect of the invention, or a pharmaceutical composition of the fourth aspect of the invention, to inhibit NLRP3.

In one embodiment of the tenth aspect of the present invention, the method is performed *ex vivo* or *in vitro,* for example in order to analyse the effect on cells of NLRP3 inhibition.

In another embodiment of the tenth aspect of the present invention, the method is performed *in vivo.* For example, the method may comprise the step of administering an effective amount of a compound of the first or second aspect, or a pharmaceutically acceptable salt, solvate or prodrug of the third aspect, or a pharmaceutical composition of the fourth aspect, to thereby inhibit NLRP3. Typically, the administration is to a subject in need thereof.

Alternately, the method of the tenth aspect of the invention may be a method of inhibiting NLRP3 in a non-human animal subject, the method comprising the steps of administering the compound, salt, solvate, prodrug or pharmaceutical composition to the non-human animal subject and optionally subsequently mutilating or sacrificing the non-human animal subject. Typically, such a method further comprises the step of analysing one or more tissue or fluid samples from the optionally mutilated or sacrificed non-human animal subject.

An eleventh aspect of the invention provides a compound of the first or second aspect of the invention, or a pharmaceutically acceptable salt, solvate or prodrug of the third aspect of the invention, or a pharmaceutical composition of the fourth aspect of the invention, for use in the inhibition of NLRP3. Typically, the use comprises the administration of the compound, salt, solvate, prodrug or pharmaceutical composition to a subject.

A twelfth aspect of the invention provides the use of a compound of the first or second aspect of the invention, or a pharmaceutically effective salt, solvate or prodrug of the third aspect of the invention, in the manufacture of a medicament for the inhibition of NLRP3. Typically, the inhibition comprises the administration of the compound, salt, solvate, prodrug or pharmaceutical composition to a subject.

Unless stated otherwise, in any of the fifth to twelfth aspects of the invention, the subject may be any human or other animal. Typically, the subject is a mammal, more typically a human or a domesticated mammal such as a cow, pig, lamb, sheet, goat, horse, cat, dog, rabbit, mouse etc. Most typically, the subject is a human.

Any of the medicaments employed in the present invention can be administered by oral, parenteral (including intravenous, subcutaneous, intramuscular, intradermal, intratracheal, intraperitoneal, intraarticular, intracranial and epidural), airway (aerosol), rectal, vaginal or topical (including transdermal, buccal, mucosal and sublingual) administration.

Typically, the mode of administration selected is that most appropriate to the disorder or disease to be treated or prevented.

For oral administration, the compounds, salts, solvates or prodrugs of the present invention will generally be provided in the form of tablets, capsules, hard or soft gelatine capsules, caplets, troches or lozenges, as a powder or granules, or as an aqueous solution, suspension or dispersion.

Tablets for oral use may include the active ingredient mixed with pharmaceutically acceptable excipients such as inert diluents, disintegrating agents, binding agents, lubricating agents, sweetening agents, flavouring agents, colouring agents and preservatives. Suitable inert diluents include sodium and calcium carbonate, sodium and calcium phosphate, and lactose. Corn starch and alginic acid are suitable disintegrating agents. Binding agents may include starch and gelatine. The lubricating agent, if present, may be magnesium stearate, stearic acid or talc. If desired, the tablets may be coated with a material, such as glyceryl monostearate or glyceryl distearate, to delay absorption in the gastrointestinal tract. Tablets may also be effervescent and/or dissolving tablets.

Capsules for oral use include hard gelatine capsules in which the active ingredient is mixed with a solid diluent, and soft gelatine capsules wherein the active ingredient is mixed with water or an oil such as peanut oil, liquid paraffin or olive oil.

Powders or granules for oral use may be provided in sachets or tubs. Aqueous solutions, suspensions or dispersions may be prepared by the addition of water to powders, granules or tablets.

Any form suitable for oral administration may optionally include sweetening agents such as sugar, flavouring agents, colouring agents and/or preservatives.

Formulations for rectal administration may be presented as a suppository with a suitable base comprising, for example, cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

For parenteral use, the compounds, salts, solvates or prodrugs of the present invention will generally be provided in a sterile aqueous solution or suspension, buffered to an appropriate pH and isotonicity. Suitable aqueous vehicles include Ringer's solution and isotonic sodium chloride or glucose. Aqueous suspensions according to the invention may include suspending agents such as cellulose derivatives, sodium alginate, polyvinylpyrrolidone and gum tragacanth, and a wetting agent such as lecithin. Suitable preservatives for aqueous suspensions include ethyl and n-propyl p-hydroxybenzoate. The compounds of the invention may also be presented as liposome formulations.

For transdermal and other topical administration, the compounds, salts, solvates or prodrugs of the invention will generally be provided in the form of ointments, cataplasms (poultices), pastes, powders, dressings, creams, plasters or patches.

Suitable suspensions and solutions can be used in inhalers for airway (aerosol) administration.

The dose of the compounds, salts, solvates or prodrugs of the present invention will, of course, vary with the disorder or disease to be treated or prevented. In general, a suitable dose will be in the range of 0.01 to 500 mg per kilogram body weight of the recipient per day. The desired dose may be presented at an appropriate interval such as once every other day, once a day, twice a day, three times a day or four times a day. The desired dose may be administered in unit dosage form, for example, containing 1 mg to 50 g of active ingredient per unit dosage form.

For the avoidance of doubt, insofar as is practicable any embodiment of a given aspect of the present invention may occur in combination with any other embodiment of the same aspect of the present invention. In addition, insofar as is practicable it is to be understood that any preferred, typical or optional embodiment of any aspect of the present invention should also be considered as a preferred, typical or optional embodiment of any other aspect of the present invention.

By way of example, combinations of aspects and embodiments that are typical of the present invention include the following.

In a first combination, a compound of the first aspect of the invention is provided wherein:
Q is O;
R¹ is R¹¹;
R¹¹ is an aryl or a heteroaryl group, wherein the aryl or the heteroaryl group may optionally be substituted; and
R² is a phenyl or a 6-membered heteroaryl group substituted at the 2-, 4- and 6-positions, wherein the phenyl or the 6-membered heteroaryl group may optionally be further substituted.

In a second combination, a compound of the first aspect of the invention is provided wherein:
Q is O;
R¹ is R¹³;
R¹³ is a heterocyclic group, wherein the heterocyclic group contains at least one nitrogen atom in its ring structure, and wherein the heterocyclic group may optionally be substituted; and
R² is a phenyl or a 6-membered heteroaryl group substituted at the 2-, 4- and 6-positions, wherein the phenyl or the 6-membered heteroaryl group may optionally be further substituted.

In a third combination, a compound of the first aspect of the invention is provided wherein the compound is a compound of formula (Ib), and wherein:
Q is O;
R¹ is R¹¹;
R¹¹ is a phenyl group or a 5- or 6-membered heteroaryl group, wherein the phenyl group or the 5- or 6-membered heteroaryl group may optionally be substituted with one or more monovalent substituents independently selected from halo, -OH, -NO₂, -CN, -R¹⁴ or -OR¹⁴, wherein each R¹⁴ is independently selected from a C₁-C₄ alkyl, C₁-C₄ haloalkyl, C₃-C₄ cycloalkyl or C₃-C₄ halocycloalkyl group;
R³ is a -COR³⁴, -COOR³⁴, -CH₂OH, -CH(R³⁴)OH or -C(R³⁴)₂OH group, wherein each R³⁴ is independently selected from a C₁-C₄ alkyl or a C₃-C₄ cycloalkyl group, wherein the C₁-C₄ alkyl or C₃-C₄ cycloalkyl group may optionally be substituted with one or more fluoro and/or chloro groups, or wherein any two R³⁴ attached to the same carbon atom may, together with the carbon atom to which they are attached, form a C₃-C₄ cycloalkyl group, wherein the C₃-C₄ cycloalkyl group may optionally be substituted with one or more fluoro and/or chloro groups;
R² has a formula selected from: 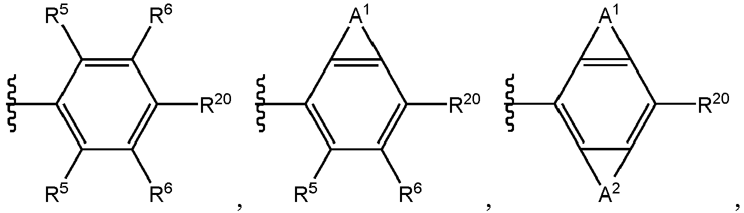 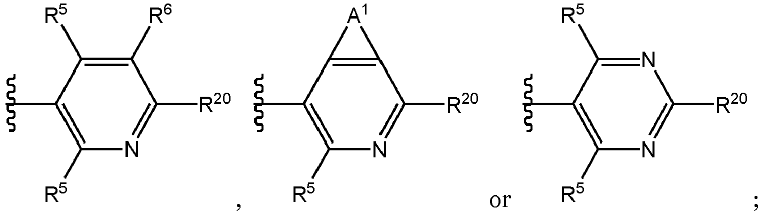
A¹ and A² are each independently selected from a straight chain alkylene group, wherein one or two carbon atoms in the backbone of the alkylene group may optionally be replaced by one or two heteroatoms independently selected from nitrogen and oxygen, wherein A¹ and A² are unsubstituted or substituted with one or more halo, -OH, -CN, -NO₂, -O(C₁-C₄ alkyl) or -O(C₁-C₄ haloalkyl) groups, and wherein any ring containing A¹ or A² is a 5- or 6-membered ring;
each R⁵ is independently selected from a C₁-C₆ alkyl, C₂-C₆ alkenyl, C₂-C₆ alkynyl or a 3- to 7-membered cyclic group, wherein the C₁-C₆ alkyl, C₂-C₆ alkenyl or C₂-C₆ alkynyl group may optionally be substituted with one or more halo, -OH, -CN, -NO₂, -O(C₁-C₄ alkyl) or -O(C₁-C₄ haloalkyl) groups, and wherein the 3- to 7-membered cyclic group may optionally be substituted with one or more halo, -OH, -NH₂, -CN, -NO₂, -R⁵², -OR⁵², -NHR⁵² or -N(R⁵²)₂ groups, wherein R⁵² is a C₁-C₄ alkyl, C₁-C₄ alkenyl or C₁-C₄ alkynyl group which may optionally be halo-substituted;
each R⁶ is independently selected from hydrogen or a halo group; and
each R²⁰ is a halo, -NO₂, -CN, -COOR²¹, -CONH₂, -CONHR²¹ or -CON(R²¹)₂ group, wherein each R²¹ is independently selected from a C₁-C₄ alkyl group, and wherein any R²¹ may optionally be substituted with one or more halo groups.

In a fourth combination, a compound of the first aspect of the invention is provided wherein the compound is a compound of formula (Ib), and wherein:
Q is O;
R¹ is R¹³;
R¹³ is a 4-, 5- or 6-membered fully saturated monocyclic heterocyclic group, wherein the 4-, 5- or 6-membered fully saturated monocyclic heterocyclic group contains at least one nitrogen atom in its ring structure, and wherein the 4-, 5- or 6-membered fully saturated monocyclic heterocyclic group may optionally be substituted with one or more monovalent substituents independently selected from halo, -CN, -OH, -NH₂, -R¹⁵, -OR¹⁵, -NHR¹⁵ and -N(R¹⁵)₂, wherein each R¹⁵ is independently selected from a C₁-C₄ alkyl, C₁-C₄ haloalkyl, C₃-C₄ cycloalkyl or C₃-C₄ halocycloalkyl group, or any two R¹⁵ directly attached to the same nitrogen atom may together form a C₂-C₅ alkylene or C₂-C₅ haloalkylene group;
R³ is a -COR³⁴, -COOR³⁴, -CH₂OH, -CH(R³⁴)OH or -C(R³⁴)₂OH group, wherein each R³⁴ is independently selected from a C₁-C₄ alkyl or a C₃-C₄ cycloalkyl group, wherein the C₁-C₄ alkyl or C₃-C₄ cycloalkyl group may optionally be substituted with one or more fluoro and/or chloro groups, or wherein any two R³⁴ attached to the same carbon atom may, together with the carbon atom to which they are attached, form a C₃-C₄ cycloalkyl group, wherein the C₃-C₄ cycloalkyl group may optionally be substituted with one or more fluoro and/or chloro groups;
R² has a formula selected from: 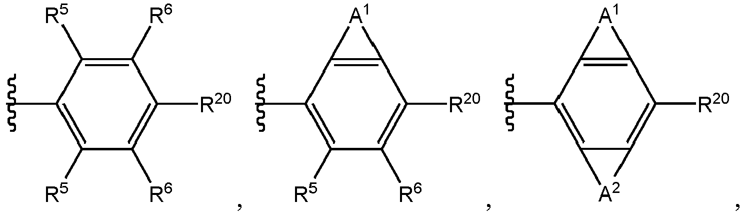 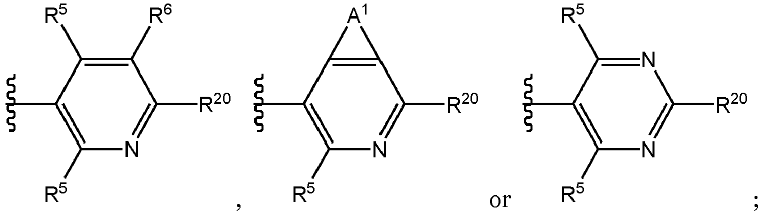
A¹ and A² are each independently selected from a straight chain alkylene group, wherein one or two carbon atoms in the backbone of the alkylene group may optionally be replaced by one or two heteroatoms independently selected from nitrogen and oxygen, wherein A¹ and A² are unsubstituted or substituted with one or more halo, -OH, -CN, -NO₂, -O(C₁-C₄ alkyl) or -O(C₁-C₄ haloalkyl) groups, and wherein any ring containing A¹ or A² is a 5- or 6-membered ring;
each R⁵ is independently selected from a C₁-C₆ alkyl, C₂-C₆ alkenyl, C₂-C₆ alkynyl or a 3- to 7-membered cyclic group, wherein the C₁-C₆ alkyl, C₂-C₆ alkenyl or C₂-C₆ alkynyl group may optionally be substituted with one or more halo, -OH, -CN, -NO₂, -O(C₁-C₄ alkyl) or -O(C₁-C₄ haloalkyl) groups, and wherein the 3- to 7-membered cyclic group may optionally be substituted with one or more halo, -OH, -NH₂, -CN, -NO₂, -R⁵², -OR⁵², -NHR⁵² or -N(R⁵²)₂ groups, wherein R⁵² is a C₁-C₄ alkyl, C₁-C₄ alkenyl or C₁-C₄ alkynyl group which may optionally be halo-substituted;
each R⁶ is independently selected from hydrogen or a halo group; and
each R²⁰ is a halo, -NO₂, -CN, -COOR²¹, -CONH₂, -CONHR²¹ or -CON(R²¹)₂ group, wherein each R²¹ is independently selected from a C₁-C₄ alkyl group, and wherein any R²¹ may optionally be substituted with one or more halo groups.

### Compound synthesis

The compounds of the invention may be synthesised, for example, by methods analogous to those outlined in WO 2016/131098 A1.

### Biological studies

The compounds of the invention may be evaluated using the following protocol.

### NLRP3 and Pyroptosis

It is well established that the activation of NLRP3 leads to cell pyroptosis and this feature plays an important part in the manifestation of clinical disease (Yan-gang Liu et al., Cell Death & Disease, 2017, 8(2), e2579; Alexander Wree et al., Hepatology, 2014, 59(3), 898-910; Alex Baldwin et al., Journal of Medicinal Chemistry, 2016, 59(5), 1691-1710; Ema Ozaki et al., Journal of Inflammation Research, 2015, 8, 15-27; Zhen Xie & Gang Zhao, Neuroimmunology Neuroinflammation, 2014, 1(2), 60-65; Mattia Cocco et al., Journal of Medicinal Chemistry, 2014, 57(24), 10366-10382; T. Satoh et al., Cell Death & Disease, 2013, 4, e644). Therefore, it is anticipated that inhibitors of NLRP3 will block pyroptosis, as well as the release of pro-inflammatory cytokines (e.g. IL-1β) from the cell.

### THP-1 Cells: Culture and Preparation

THP-1 cells (ATCC # TIB-202) are grown in RPMI containing L-glutamine (Gibco #11835) supplemented with 1mM sodium pyruvate (Sigma # S8636) and penicillin (100units/ml) / streptomycin (0.1mg/ml) (Sigma # P4333) in 10% Fetal Bovine Serum (FBS) (Sigma # F0804). The cells are routinely passaged and grown to confluency (~10⁶cells/ml). On the day of the experiment, THP-1 cells are harvested and resuspended into RPMI medium (without FBS). The cells are then counted and viability (>90%) checked by Trypan blue (Sigma # T8154). Appropriate dilutions are made to give a concentration of 625,000cells/ml. To this diluted cell solution is added LPS (Sigma # L4524) to give a 1µg/ml Final Assay Concentration (FAC). 40µl of the final preparation is aliquoted into each well of a 96-well plate. The plate thus prepared is used for compound screening.

### THP-1 Cells Pyroptosis Assay

The following method step-by-step assay may be followed for compound screening.
1. Seed THP-1 cells (25,000cells/well) containing 1.0µg/ml LPS in 40µl of RPMI medium (without FBS) in 96-well, black walled, clear bottom cell culture plates coated with poly-D-lysine (VWR # 734-0317)
2. Add 5µl compound (8 points half-log dilution, with 10µM top dose) or vehicle (DMSO 0.1% FAC) to the appropriate wells
3. Incubate for 3hrs at 37°C in 5% CO₂
4. Add 5µl nigericin (Sigma # N7143) (FAC 5µM) to all wells
5. Incubate for 1hr at 37°C and 5% CO₂
6. At the end of the incubation period, spin plates at 300×g for 3mins and remove supernatant
7. Then add 50µl of resazurin (Sigma # R7017) (FAC 100 µM resazurin in RPMI medium without FBS) and incubate plates for a further 1-1.5h at 37°C and 5% CO₂
8. Read plates in an Envision reader at Ex 560nm and Em 590nm
9. Fit IC₅₀ data to a non-linear regression equation (log inhibitor vs response-variable slope 4-parameters)

### 96-well Plate Map

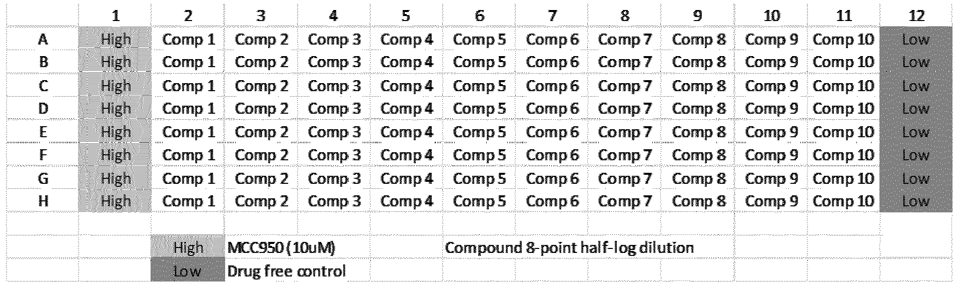

The results of the pyroptosis assay performed may be summarised as THP IC₅₀.

### Human Whole Blood IL1β Release Assay

For systemic delivery, the ability to inhibit NLRP3 when the compounds are present within the bloodstream is of great importance. For this reason, the NLRP3 inhibitory activity of the compounds of the invention in human whole blood may be investigated in accordance with the following protocol.

Human whole blood in Li-heparin tubes is obtained from healthy donors from a volunteer donor panel.
1. Plate out 80µl of whole blood containing 1µg/ml of LPS in 96-well, clear bottom cell culture plate (Corning # 3585)
2. Add 10µl compound (8 points half-log dilution with 10µM top dose) or vehicle (DMSO 0.1% FAC) to the appropriate wells
3. Incubate for 3hrs at 37°C, 5% CO₂
4. Add 10µl Nigericin (Sigma # N7143) (10µM FAC) to all wells
5. Incubate for 1hr at 37°C, 5% CO₂
6. At the end of the incubation period, spin plates at 300×g for 5mins to pellet cells and remove 20µl of supernatant and add to 96-well v-bottom plates for IL-1β analysis (note: these plates containing the supernatants can be stored at -80°C to be analysed at a later date)
7. Measure IL-1β according to the manufacturer protocol (Perkin Elmer-AlphaLisa IL-1 Kit AL220F-5000)
8. Fit IC₅₀ data to a non-linear regression equation (log inhibitor vs response-variable slope 4-parameters)

The results of the human whole blood assay may be summarised as HWB IC₅₀.

It will be understood that the present invention has been described above by way of example only. The examples are not intended to limit the scope of the invention. Various modifications and embodiments can be made without departing from the scope and spirit of the invention, which is defined by the following claims only.

### Embodiments

Embodiment 1. A compound of formula (I): 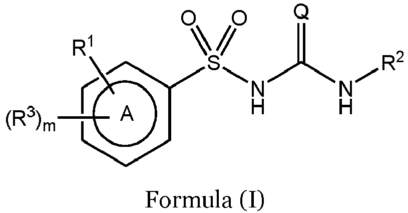 wherein:
Q is selected from O or S;
R¹ is selected from (i) a monovalent group comprising an aryl or a heteroaryl group, or (ii) a monovalent group comprising a heterocyclic group, wherein the heterocyclic group contains at least one nitrogen atom in its ring structure;
R² is a 6-membered cyclic group substituted at the 2- and 4-positions, wherein the 6-membered cyclic group may optionally be further substituted;
m is 0, 1, 2, 3 or 4;
each R³ is independently a halo, -OH, -NO₂, -NH₂, -N₃, -SH, -SO₂H, -SO₂NH, or a saturated or unsaturated hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton; and
wherein optionally any R³, and any two adjacent carbon atoms of ring A, may together form a 4- to 12-membered saturated or unsaturated cyclic group fused to ring A, wherein the cyclic group fused to ring A may optionally be substituted.

Embodiment 2. A compound as claimed in embodiment Embodiment 1, wherein ring A is monocyclic.

Embodiment 3. A compound as claimed in embodiment Embodiment 1 or embodiment Embodiment 2, wherein R¹ is a monovalent group comprising an aryl or a heteroaryl group.

Embodiment 4. A compound as claimed in embodiment Embodiment 3, wherein the aryl or the heteroaryl group is monocyclic.

Embodiment 5. A compound as claimed in embodiment Embodiment 3 or embodiment Embodiment 4, wherein a ring atom of the aryl group or a ring atom of the heteroaryl group is directly attached to a ring atom of ring A.

Embodiment 6. A compound as claimed in embodiment Embodiment 1 or embodiment Embodiment 2, wherein R¹ is a monovalent group comprising a heterocyclic group, wherein the heterocyclic group contains at least one nitrogen atom in its ring structure.

Embodiment 7. A compound as claimed in embodiment Embodiment 6, wherein the heterocyclic group is a non-aromatic heterocyclic group.

Embodiment 8. A compound as claimed in embodiment Embodiment 6 or embodiment Embodiment 7, wherein the heterocyclic group is monocyclic.

Embodiment 9. A compound as claimed in any one of embodiments Embodiment 6 to Embodiment 8, wherein a ring atom of the heterocyclic group is directly attached to a ring atom of ring A.

Embodiment 10. A compound as claimed in any one of embodiments Embodiment 1 to Embodiment 9, wherein the compound is a compound of formula (Ia): 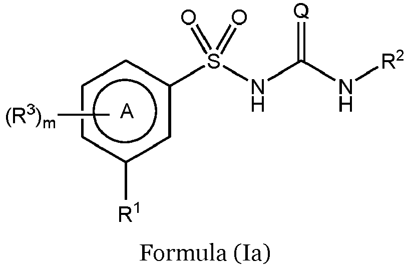 wherein R¹, R², R³, m and Q are defined in any one of embodiments Embodiment 1 to Embodiment 9.

Embodiment 11. A compound as claimed in any one of embodiments Embodiment 1 to Embodiment 10, wherein each R³ is independently selected from halo; -CN; -NO₂; -N₃; -R^{β}; -OH; -OR^{β}; -R^{α}-halo; -R^{α}-CN; -R^{α}-NO₂; -R^{α}-N₃; -R^{α}-R^{β}; -R^{α}-OH; -R^{α}-OR^{β}; -SH; -SR^{β}; -SOR^{β}; -SO₂H; -SO₂R^{β}; -SO₂NH₂; -SO₂NHR^{β}; -SO₂N(R^{β})₂; -R^{α}-SH; -R^{α}-SR^{β}; -R^{α}-SOR^{β}; -R^{α}-SO₂H; -R^{α}-SO₂R^{β}; -R^{α}-SO₂NH₂; -R^{α}-SO₂NHR^{β}; -R^{α}-SO₂N(R^{β})₂; -NH₂; -NHR^{β}; -N(R^{β})₂; -R^{α}-NH₂; -R^{α}-NHR^{β}; -R^{α}-N(R^{β})₂; -CHO; -COR^{β}; -COOH; -COOR^{β}; -OCOR^{β}; -R^{α}-CHO; -R^{α}-COR^{β}; -R^{α}-COOH; -R^{α}-COOR^{β}; or -R^{α}-OCOR^{β};
wherein each -R^{α}- is independently selected from an alkylene, alkenylene or alkynylene group, wherein the alkylene, alkenylene or alkynylene group contains from 1 to 6 atoms in its backbone, wherein one or more carbon atoms in the backbone of the alkylene, alkenylene or alkynylene group may optionally be replaced by one or more heteroatoms N, O or S, and wherein the alkylene, alkenylene or alkynylene group may optionally be substituted with one or more halo and/or -R^{β} groups; and
wherein each -R^{β} is independently selected from a C₁-C₆ alkyl, C₂-C₆ alkenyl, C₂-C₆ alkynyl or C₂-C₆ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -C=CH, oxo (=O), or 4- to 6-membered heterocyclic group.

Embodiment 12. A compound as claimed in any one of embodiments Embodiment 1 to Embodiment 11, wherein the compound is a compound of formula (Ib): 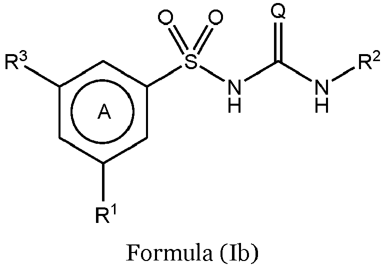 wherein R¹, R² and Q are as defined in any one of embodiments Embodiment 1 to Embodiment 11, and wherein R³ is a saturated or unsaturated hydrocarbyl group, wherein the hydrocarbyl group may be straight-chained or branched, or be or include cyclic groups, wherein the hydrocarbyl group may optionally be substituted, wherein the hydrocarbyl group includes at least one oxygen atom in its carbon skeleton, and wherein the hydrocarbyl group may optionally include one or more additional heteroatoms N or O in its carbon skeleton.

Embodiment 13. A compound as claimed in any one of embodiments Embodiment 1 to Embodiment 12, wherein the substituent at the 4-position of the 6-membered cyclic group of R² is a halo, -OH, -NO₂, -NH₂, -N₃, -SH, -SO₂H, -SO₂NH, or a saturated or unsaturated hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton.

Embodiment 14. A compound as claimed in embodiment Embodiment 13, wherein the substituent at the 4-position of the 6-membered cyclic group of R² is a halo, -NO₂, -CN, -COOR²¹, -CONH₂, -CONHR²¹ or -CON(R²¹)₂ group, wherein each -R²¹ is independently selected from a C₁-C₄ alkyl group, and wherein any -R²¹ may optionally be substituted with one or more halo groups.

Embodiment 15. A compound as claimed in any one of embodiments Embodiment 1 to Embodiment 14, wherein R² is a phenyl or a 6-membered heteroaryl group substituted at the 2- and 4-positions, wherein the phenyl or the 6-membered heteroaryl group may optionally be further substituted.

Embodiment 16. A compound as claimed in embodiment Embodiment 15, wherein R² is a phenyl or a 6-membered heteroaryl group substituted at the 2-, 4- and 6-positions, wherein the phenyl or the 6-membered heteroaryl group may optionally be further substituted.

Embodiment 17. A compound as claimed in embodiment Embodiment 16, wherein R² is a fused phenyl group, wherein a first cycloalkyl, cycloalkenyl, non-aromatic heterocyclic, aryl or heteroaryl ring is fused to the phenyl group across the 2,3-positions and a second cycloalkyl, cycloalkenyl, non-aromatic heterocyclic, aryl or heteroaryl ring is fused to the phenyl group across the 5,6-positions, wherein the phenyl group is further substituted at the 4-position, and wherein R² may optionally be further substituted.

Embodiment 18. A compound as claimed in any one of embodiments Embodiment 1 to Embodiment 16, wherein the substituent at the 2-position of the 6-membered cyclic group of R² is a monovalent heterocyclic group or a monovalent aromatic group, wherein a ring atom of the monovalent heterocyclic or monovalent aromatic group is directly attached to the ring atom at the 2-position of the 6-membered cyclic group, wherein the monovalent heterocyclic or monovalent aromatic group may optionally be substituted.

Embodiment 19. A compound as claimed in any one of embodiments Embodiment 1 to Embodiment 14, wherein R² is a 6-membered cyclic group substituted at the 2-, 4- and 6-positions, wherein the 6-membered cyclic group may optionally be further substituted.

Embodiment 20. A compound as claimed in any one of embodiments Embodiment 1 to Embodiment 19, wherein Q is O.

Embodiment 21. A compound selected from the group consisting of: 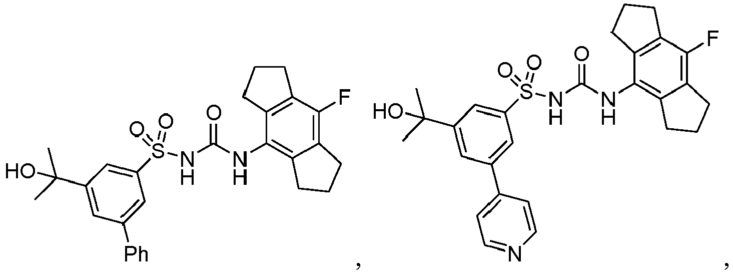 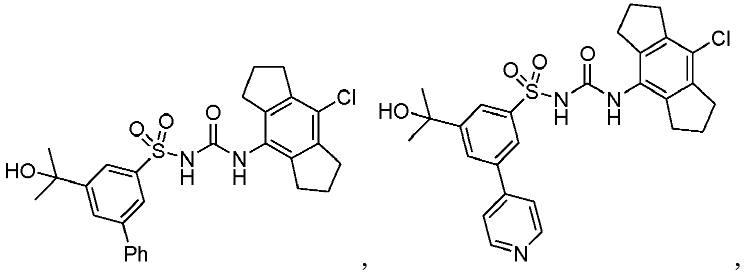 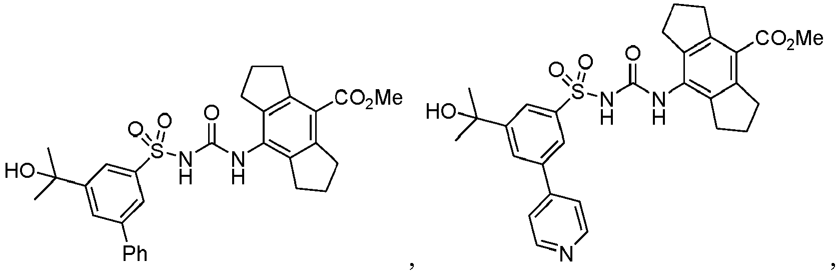 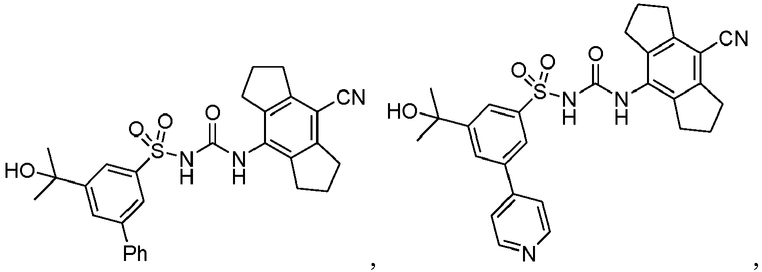 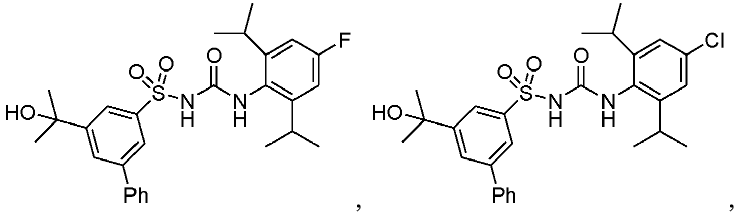 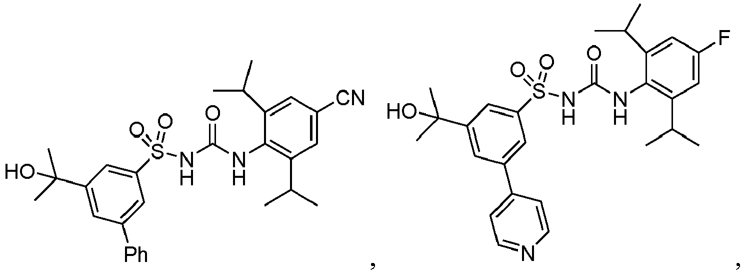 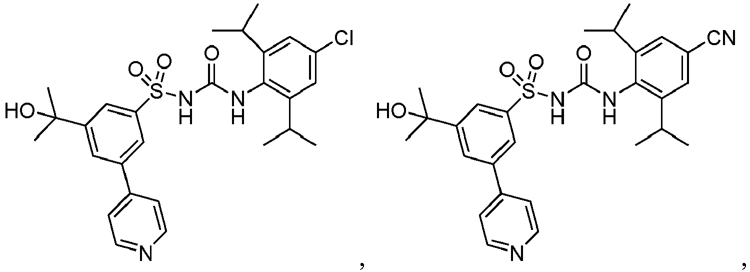 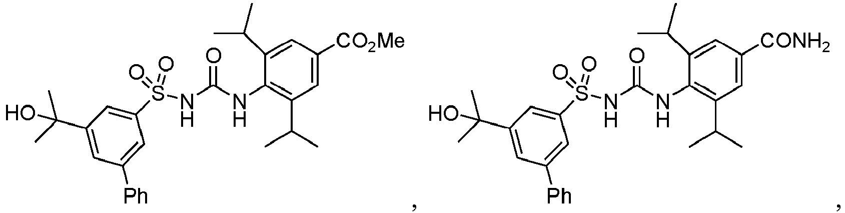 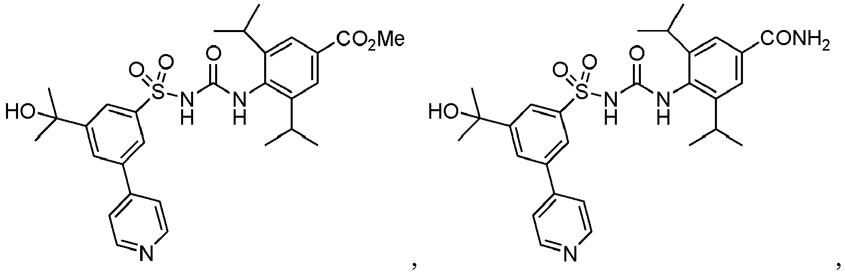 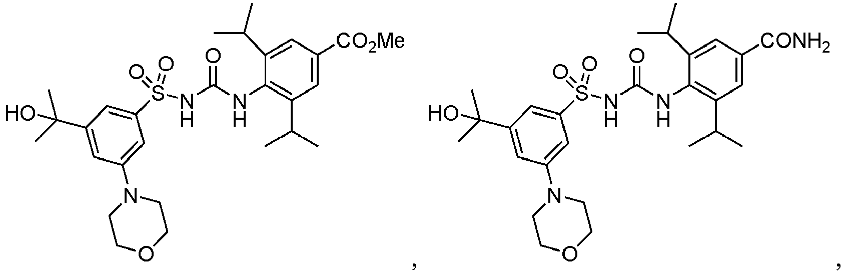 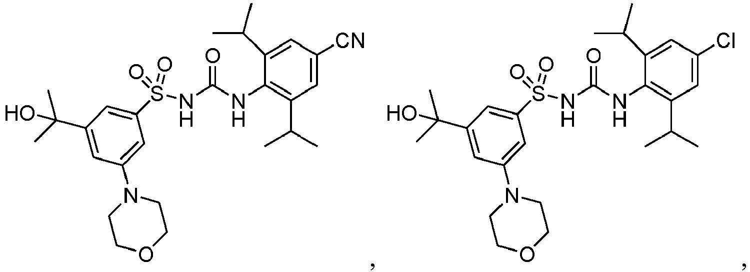 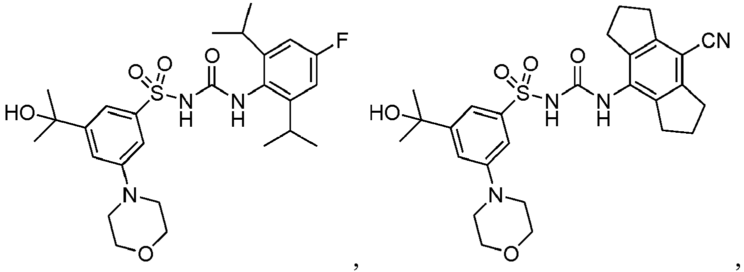 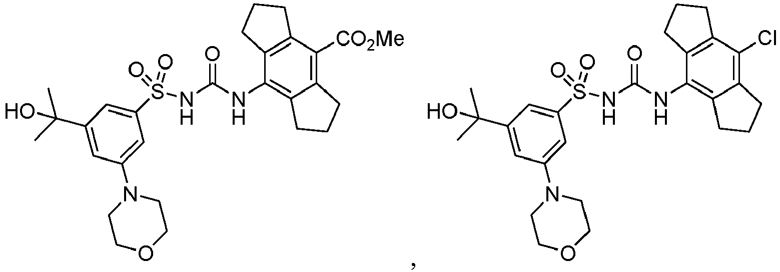 and

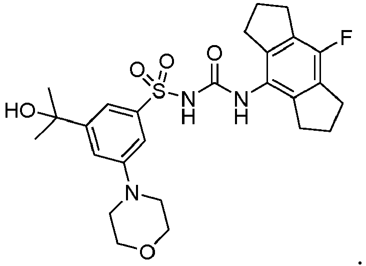

Embodiment 22. A pharmaceutically acceptable salt, solvate or prodrug of a compound as claimed in any one of embodiments Embodiment 1 to Embodiment 21.

Embodiment 23. A pharmaceutical composition comprising a compound as claimed in any one of embodiments Embodiment 1 to Embodiment 21, or a pharmaceutically acceptable salt, solvate or prodrug as claimed in embodiment Embodiment 22, and a pharmaceutically acceptable excipient.

Embodiment 24. A compound as claimed in any one of embodiments Embodiment 1 to Embodiment 21, or a pharmaceutically acceptable salt, solvate or prodrug as claimed in embodiment Embodiment 22, or a pharmaceutical composition as claimed in embodiment Embodiment 23, for use in medicine.

Embodiment 25. A compound, pharmaceutically acceptable salt, solvate, prodrug or pharmaceutical composition as claimed in embodiment Embodiment 24, for use in the treatment or prevention of a disease, disorder or condition, wherein the disease, disorder or condition is responsive to NLRP3 inhibition.

Embodiment 26. A compound, pharmaceutically acceptable salt, solvate, prodrug or pharmaceutical composition as claimed in embodiment Embodiment 24 or embodiment Embodiment 25, for use in the treatment or prevention of a disease, disorder or condition selected from:
(i) inflammation;
(ii) an auto-immune disease;
(iii) cancer;
(iv) an infection;
(v) a central nervous system disease;
(vi) a metabolic disease;
(vii) a cardiovascular disease;
(viii) a respiratory disease;
(ix) a liver disease;
(x) a renal disease;
(xi) an ocular disease;
(xii) a skin disease;
(xiii) a lymphatic condition;
(xiv) a psychological disorder;
(xv) graft versus host disease; and
(xvi) any disease where an individual has been determined to carry a germline or somatic non-silent mutation in NLRP3.

Embodiment 27. A compound, pharmaceutically acceptable salt, solvate, prodrug or pharmaceutical composition as claimed in embodiment Embodiment 24 or embodiment Embodiment 25, for use in the treatment or prevention of a disease, disorder or condition selected from:
(i) cryopyrin-associated periodic syndromes (CAPS);
(ii) Muckle-Wells syndrome (MWS);
(iii) familial cold autoinflammatory syndrome (FCAS);
(iv) neonatal onset multisystem inflammatory disease (NOMID);
(v) familial Mediterranean fever (FMF);
(vi) pyogenic arthritis, pyoderma gangrenosum and acne syndrome (PAPA);
(vii) hyperimmunoglobulinemia D and periodic fever syndrome (HIDS);
(viii) Tumour Necrosis Factor (TNF) Receptor-Associated Periodic Syndrome (TRAPS);
(ix) systemic juvenile idiopathic arthritis;
(x) adult-onset Still's disease (AOSD);
(xi) relapsing polychondritis;
(xii) Schnitzler's syndrome;
(xiii) Sweet's syndrome;
(xiv) Behcet's disease;
(xv) anti-synthetase syndrome;
(xvi) deficiency of interleukin 1 receptor antagonist (DIRA); and
(xvii) haploinsufficiency of A20 (HA20).

Embodiment 28. A method of inhibiting NLRP3, the method comprising the use of a compound as claimed in any one of embodiments Embodiment 1 to Embodiment 21, or a pharmaceutically acceptable salt, solvate or prodrug as claimed in embodiment Embodiment 22, or a pharmaceutical composition as claimed in embodiment Embodiment 23, to inhibit NLRP3.

## Claims

1. A compound of formula (I): 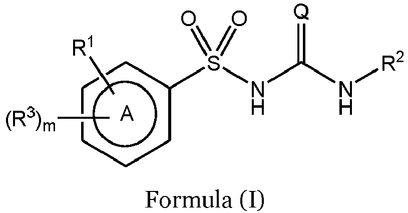 wherein:
Q is selected from O or S;
R¹ is selected from (i) a monovalent group comprising an aryl or a heteroaryl group, or (ii) a monovalent group comprising a heterocyclic group, wherein the heterocyclic group contains at least one nitrogen atom in its ring structure;
R² is a 6-membered cyclic group substituted at the 2- and 4-positions, wherein the 6-membered cyclic group may optionally be further substituted;
m is 0, 1, 2, 3 or 4;
each R³ is independently a halo, -OH, -NO₂, -NH₂, -N₃, -SH, -SO₂H, -SO₂NH, or a saturated or unsaturated hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton; and
wherein optionally any R³, and any two adjacent carbon atoms of ring A, may together form a 4- to 12-membered saturated or unsaturated cyclic group fused to ring A, wherein the cyclic group fused to ring A may optionally be substituted.

2. A compound as claimed in claim 1, wherein ring A is monocyclic.

3. A compound as claimed in claim 1 or claim 2, wherein R¹ is a monovalent group comprising an aryl or a heteroaryl group.

4. A compound as claimed in claim 3, wherein the aryl or the heteroaryl group is monocyclic.

5. A compound as claimed in claim 3 or claim 4, wherein a ring atom of the aryl group or a ring atom of the heteroaryl group is directly attached to a ring atom of ring A.

6. A compound as claimed in claim 1 or claim 2, wherein R¹ is a monovalent group comprising a heterocyclic group, wherein the heterocyclic group contains at least one nitrogen atom in its ring structure.

7. A compound as claimed in claim 6, wherein the heterocyclic group is a non-aromatic heterocyclic group.

8. A compound as claimed in claim 6 or claim 7, wherein the heterocyclic group is monocyclic.

9. A compound as claimed in any one of claims 6 to 8, wherein a ring atom of the heterocyclic group is directly attached to a ring atom of ring A.

10. A compound as claimed in any one of claims 1 to 9, wherein the compound is a compound of formula (Ia): 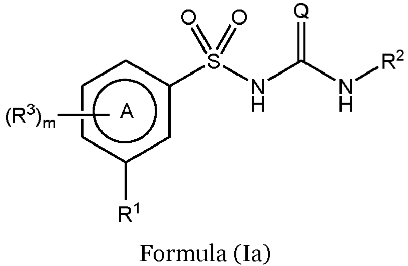 wherein R¹, R², R³, m and Q are defined in any one of claims 1 to 9.

11. A compound as claimed in any one of claims 1 to 10, wherein each R³ is independently selected from halo; -CN; -NO₂; -N₃; -R^{β}; -OH; -OR^{β}; -R^{α}-halo; -R^{α}-CN; -R^{α}-NO₂; -R^{α}-N₃; -R^{α}-R^{β}; -R^{α}-OH; -R^{α}-OR^{β}; -SH; -SR^{β}; -SOR^{β}; -SO₂H; -SO₂R^{β}; -SO₂NH₂; -SO₂NHR^{β}; -SO₂N(R^{β})₂; -R^{α}-SH; -R^{α}-SR^{β}; -R^{α}-SOR^{β}; -R^{α}-SO₂H; -R^{α}-SO₂R^{β}; -R^{α}-SO₂NH₂; -R^{α}-SO₂NHR^{β}; -R^{α}-SO₂N(R^{β})₂; -NH₂; -NHR^{β}; -N(R^{β})₂; -R^{α}-NH₂; -R^{α}-NHR^{β}; -R^{α}-N(R^{β})₂; -CHO; -COR^{β}; -COOH; -COOR^{β}; -OCOR^{β}; -R^{α}-CHO; -R^{α}-COR^{β}; -R^{α}-COOH; -R^{α}-COOR^{β}; or -R^{α}-OCOR^{β};
wherein each -R^{α}- is independently selected from an alkylene, alkenylene or alkynylene group, wherein the alkylene, alkenylene or alkynylene group contains from 1 to 6 atoms in its backbone, wherein one or more carbon atoms in the backbone of the alkylene, alkenylene or alkynylene group may optionally be replaced by one or more heteroatoms N, O or S, and wherein the alkylene, alkenylene or alkynylene group may optionally be substituted with one or more halo and/or -R^{β} groups; and
wherein each -R^{β} is independently selected from a C₁-C₆ alkyl, C₂-C₆ alkenyl, C₂-C₆ alkynyl or C₂-C₆ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -C=CH, oxo (=O), or 4- to 6-membered heterocyclic group.

12. A compound as claimed in any one of claims 1 to 11, wherein the compound is a compound of formula (Ib): 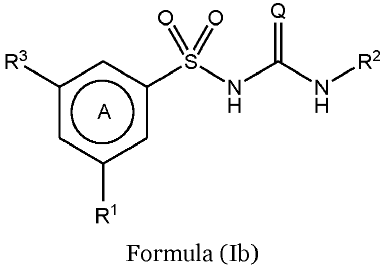 wherein R¹, R² and Q are as defined in any one of claims 1 to 11, and wherein R³ is a saturated or unsaturated hydrocarbyl group, wherein the hydrocarbyl group may be straight-chained or branched, or be or include cyclic groups, wherein the hydrocarbyl group may optionally be substituted, wherein the hydrocarbyl group includes at least one oxygen atom in its carbon skeleton, and wherein the hydrocarbyl group may optionally include one or more additional heteroatoms N or O in its carbon skeleton.

13. A compound as claimed in any one of claims 1 to 12, wherein the substituent at the 4-position of the 6-membered cyclic group of R² is a halo, -OH, -NO₂, -NH₂, -N₃, -SH, -SO₂H, -SO₂NH, or a saturated or unsaturated hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton.

14. A compound as claimed in claim 13, wherein the substituent at the 4-position of the 6-membered cyclic group of R² is a halo, -NO₂, -CN, -COOR²¹, -CONH₂, -CONHR²¹ or -CON(R²¹)₂ group, wherein each -R²¹ is independently selected from a C₁-C₄ alkyl group, and wherein any -R²¹ may optionally be substituted with one or more halo groups.

15. A compound as claimed in any one of claims 1 to 14, wherein R² is a phenyl or a 6-membered heteroaryl group substituted at the 2- and 4-positions, wherein the phenyl or the 6-membered heteroaryl group may optionally be further substituted.

16. A compound as claimed in claim 15, wherein R² is a phenyl or a 6-membered heteroaryl group substituted at the 2-, 4- and 6-positions, wherein the phenyl or the 6-membered heteroaryl group may optionally be further substituted.

17. A compound as claimed in claim 16, wherein R² is a fused phenyl group, wherein a first cycloalkyl, cycloalkenyl, non-aromatic heterocyclic, aryl or heteroaryl ring is fused to the phenyl group across the 2,3-positions and a second cycloalkyl, cycloalkenyl, non-aromatic heterocyclic, aryl or heteroaryl ring is fused to the phenyl group across the 5,6-positions, wherein the phenyl group is further substituted at the 4-position, and wherein R² may optionally be further substituted.

18. A compound as claimed in any one of claims 1 to 16, wherein the substituent at the 2-position of the 6-membered cyclic group of R² is a monovalent heterocyclic group or a monovalent aromatic group, wherein a ring atom of the monovalent heterocyclic or monovalent aromatic group is directly attached to the ring atom at the 2-position of the 6-membered cyclic group, wherein the monovalent heterocyclic or monovalent aromatic group may optionally be substituted.

19. A compound as claimed in any one of claims 1 to 14, wherein R² is a 6-membered cyclic group substituted at the 2-, 4- and 6-positions, wherein the 6-membered cyclic group may optionally be further substituted.

20. A compound as claimed in any one of claims 1 to 19, wherein Q is O.

21. A compound selected from the group consisting of: 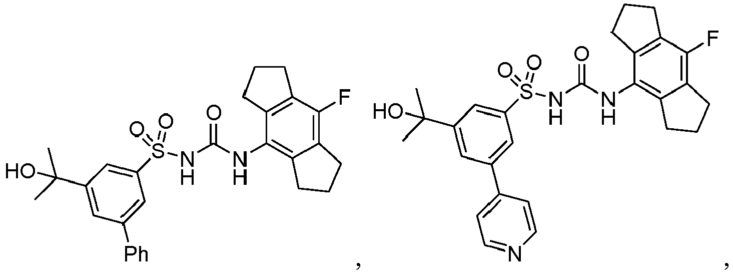 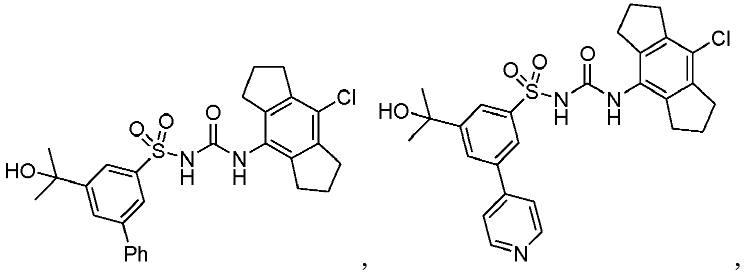 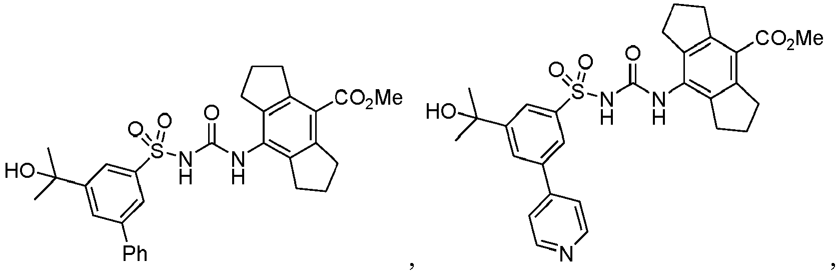 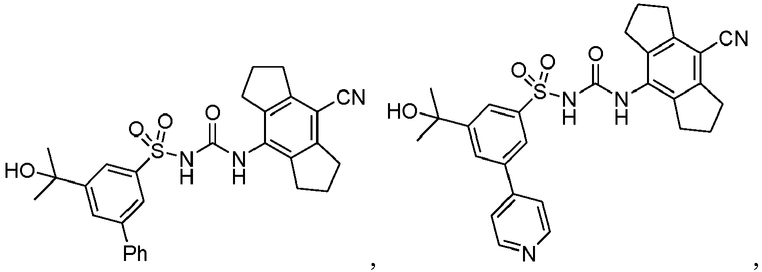 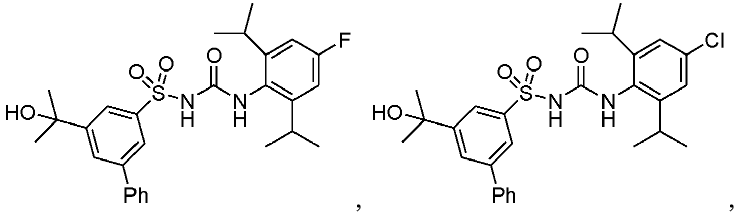 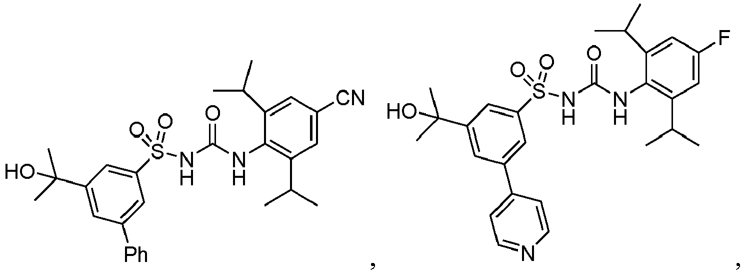 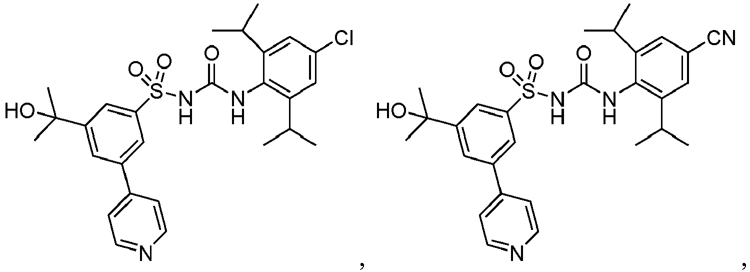 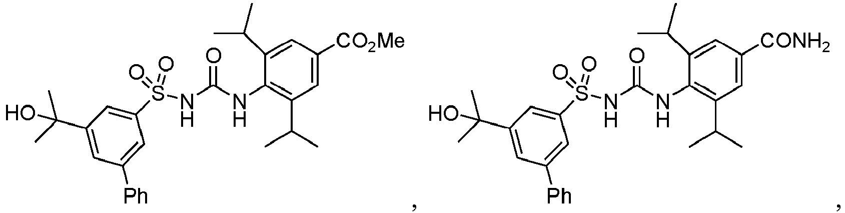 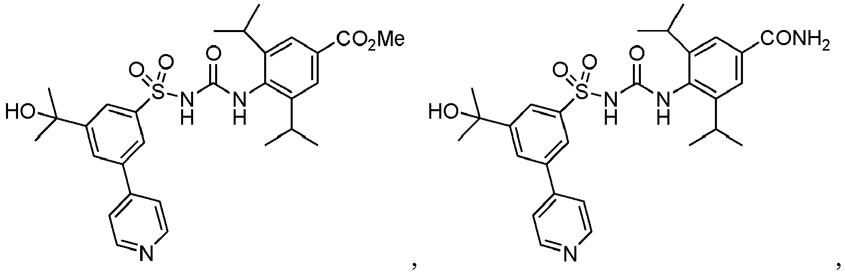 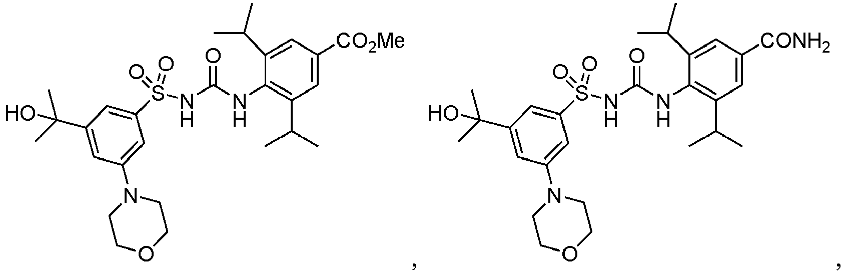 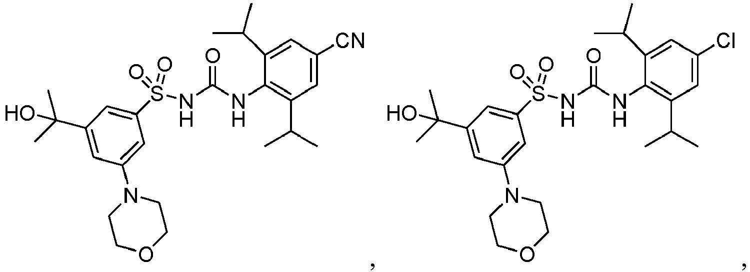 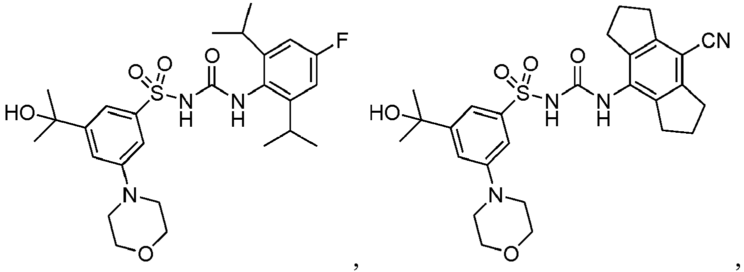 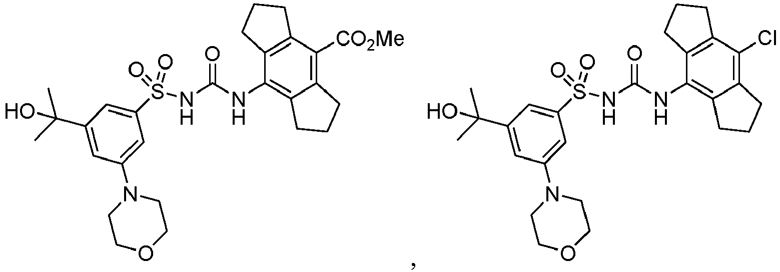 and

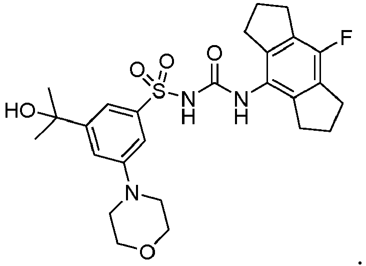

22. A pharmaceutically acceptable salt, solvate or prodrug of a compound as claimed in any one of claims 1 to 21.

23. A pharmaceutical composition comprising a compound as claimed in any one of claims 1 to 21, or a pharmaceutically acceptable salt, solvate or prodrug as claimed in claim 22, and a pharmaceutically acceptable excipient.

24. A compound as claimed in any one of claims 1 to 21, or a pharmaceutically acceptable salt, solvate or prodrug as claimed in claim 22, or a pharmaceutical composition as claimed in claim 23, for use in medicine.

25. A compound, pharmaceutically acceptable salt, solvate, prodrug or pharmaceutical composition as claimed in claim 24, for use in the treatment or prevention of a disease, disorder or condition, wherein the disease, disorder or condition is responsive to NLRP3 inhibition.

26. A compound, pharmaceutically acceptable salt, solvate, prodrug or pharmaceutical composition as claimed in claim 24 or claim 25, for use in the treatment or prevention of a disease, disorder or condition selected from:
(i) inflammation;
(ii) an auto-immune disease;
(iii) cancer;
(iv) an infection;
(v) a central nervous system disease;
(vi) a metabolic disease;
(vii) a cardiovascular disease;
(viii) a respiratory disease;
(ix) a liver disease;
(x) a renal disease;
(xi) an ocular disease;
(xii) a skin disease;
(xiii) a lymphatic condition;
(xiv) a psychological disorder;
(xv) graft versus host disease; and
(xvi) any disease where an individual has been determined to carry a germline or somatic non-silent mutation in NLRP3.

27. A compound, pharmaceutically acceptable salt, solvate, prodrug or pharmaceutical composition as claimed in claim 24 or claim 25, for use in the treatment or prevention of a disease, disorder or condition selected from:
(i) cryopyrin-associated periodic syndromes (CAPS);
(ii) Muckle-Wells syndrome (MWS);
(iii) familial cold autoinflammatory syndrome (FCAS);
(iv) neonatal onset multisystem inflammatory disease (NOMID);
(v) familial Mediterranean fever (FMF);
(vi) pyogenic arthritis, pyoderma gangrenosum and acne syndrome (PAPA);
(vii) hyperimmunoglobulinemia D and periodic fever syndrome (HIDS);
(viii) Tumour Necrosis Factor (TNF) Receptor-Associated Periodic Syndrome (TRAPS);
(ix) systemic juvenile idiopathic arthritis;
(x) adult-onset Still's disease (AOSD);
(xi) relapsing polychondritis;
(xii) Schnitzler's syndrome;
(xiii) Sweet's syndrome;
(xiv) Behcet's disease;
(xv) anti-synthetase syndrome;
(xvi) deficiency of interleukin 1 receptor antagonist (DIRA); and
(xvii) haploinsufficiency of A20 (HA20).

28. A method of inhibiting NLRP3, the method comprising the use of a compound as claimed in any one of claims 1 to 21, or a pharmaceutically acceptable salt, solvate or prodrug as claimed in claim 22, or a pharmaceutical composition as claimed in claim 23, to inhibit NLRP3.
